(19) Europäisches Patentamt
European Patent Office
Office européen des brevets

(11) Veröffentlichungsnummer: **0 064 740**
**B1**

(12) # EUROPÄISCHE PATENTSCHRIFT

(45) Veröffentlichungstag der Patentschrift: **09.04.86**

(51) Int. Cl.⁴: **C 07 D 501/46, A 61 K 31/545**

(21) Anmeldenummer: **82103989.8**

(22) Anmeldetag: **07.05.82**

(54) **Cephalosporinderivate und Verfahren zu ihrer Herstellung.**

(30) Priorität: **12.05.81 DE 3118732**

(43) Veröffentlichungstag der Anmeldung:
**17.11.82 Patentblatt 82/46**

(45) Bekanntmachung des Hinweises auf die Patenterteilung:
**09.04.86 Patentblatt 86/15**

(84) Benannte Vertragsstaaten:
**AT BE CH DE FR GB IT LI LU NL SE**

(56) Entgegenhaltungen:
**EP-A-0 034 760**
**EP-A-0 055 466**
**EP-A-0 060 144**
**FR-A-2 348 218**
**FR-A-2 385 722**
**FR-A-2 387 234**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Lattrell, Rudolf, Dr., Heuhohlweg 6H, D-6240 Königstein/Taunus (DE)**
Erfinder: **Wieduwilt, Manfred, Dr., Rauenthaler Weg 20, D-6000 Frankfurt am Main 71 (DE)**
Erfinder: **Dürckheimer, Walter, Dr., Im Lerchenfeld 45, D-6234 Hattersheim am Main (DE)**
Erfinder: **Blumbach, Jürgen, Dr., Merziger Weg 1A, D-6000 Frankfurt am Main 71 (DE)**
Erfinder: **Seeger, Karl, Dr., Schwalbenweg 9, D-6238 Hofheim am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 064 740 B1

LIBER, STOCKHOLM 1986

**Beschreibung**

Die Erfindung betrifft neue Cephalosporinderivate und Verfahren zu ihrer Herstellung, insbesondere polare Cephemderivate, die in 3-Stellung des Cephemrings durch bestimmte Pyridiniummethyl-Reste substituiert sind und die eine sehr gute antimikrobielle Wirkung gegen gram-positive und gram-negative Bakterien besitzen und deshalb als Arzneimittel zur Behandlung von mikrobiellen Infektionen geeignet sind.

Gegenstand der Erfindung sind daher Cephemderivate der allgemeinen Formel I

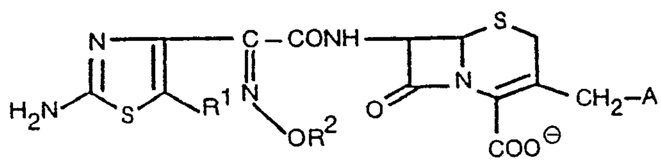

(I)

worin bedeuten

$R^1$ Wasserstoff oder Halogen,

$R^2$ Wasserstoff oder $C_1$—$C_6$-Alkyl,

A einen Pyridiniumrest

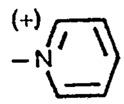

der ein- oder mehrfach, gleich oder verschieden substituiert sein kann

durch $C_1$—$C_6$-Alkyl, das ein- oder mehrfach substituiert sein kann durch Hydroxy, Carboxy, $C_1$—$C_6$-Alkoxycarbonyl, Formyl oder $C_1$—$C_6$-Alkylcarbonyl, Carbamoyl, N-Hydroxycarbamoyl, Sulfo, $C_1$—$C_6$-Alkoxy, Hydroxy-$C_1$—$C_6$-alkoxy, $C_1$—$C_6$-Alkylthio, $C_1$—$C_6$-Alkylsulfinyl, $C_1$—$C_6$-Alkylsulfonyl, $C_2$—$C_6$-Alkenyloxy, $C_2$—$C_6$-Alkenylthio, $C_2$—$C_6$-Alkenylsulfinyl oder $C_2$—$C_6$-Alkenylsulfonyl und wobei 2 Alkylgruppen auch zu einem gegebenenfalls durch $C_1$—$C_6$-Alkyl, $C_1$—$C_4$-Alkoxy, Hydroxymethyl, Halogen, Hydroxy, Oxo, Hydroxyimino, Exomethylen, Carboxy, $C_1$—$C_6$-Alkoxycarbonyl, Cyano oder Carbamoyl substituierten Di- bis Decamethylen-Ring geschlossen sein können, in dem ein C-Atom durch ein Sauerstoff- oder Schwefelatom ersetzt sein kann und weiterhin auch noch eine oder zwei Doppelbindungen enthalten sein können, durch Cyano-$C_1$—$C_3$-alkyl, Epoxy-$C_2$—$C_6$-alkyl, Trifluormethyl, Hydroxyiminomethyl oder $C_1$—$C_4$-Alkoxyiminomethyl, Pentafluoräthyl,

durch $C_2$—$C_6$-Alkenyl, das durch Hydroxy substituiert sein kann,

durch $C_2$—$C_6$-Alkinyl,

durch $C_3$—$C_7$-Cycloalkyl oder $C_3$—$C_7$-Cycloalkyl-methyl, wobei in beiden Substituenten der Ring auch substituiert sein kann durch Hydroxy, Halogen, Carboxy, $C_1$—$C_6$-Alkoxycarbonyl oder Cyano,

durch $C_4$—$C_7$-Cycloalkenyl,

durch $C_1$—$C_6$-Alkoxy, das durch Hydroxy, Carboxy oder $C_1$—$C_6$-Alkoxycarbonyl substituiert sein kann,

durch Epoxy-$C_2$—$C_6$-Alkoxy,

durch $C_2$—$C_6$-Alkenyloxy oder $C_2$—$C_6$-Alkinyloxy,

durch Halogen, Cyano, Hydroxy oder Mercapto,

durch $C_1$—$C_6$-Alkylthio, $C_1$—$C_6$-Alkylsulfinyl oder $C_1$—$C_6$-Alkylsulfonyl, die im Alkylteil durch Hydroxy substituiert sein können,

durch Methylthio, Methylsulfinyl oder Methylsulfonyl, die im Methylteil substituiert sind durch Carboxy oder $C_1$—$C_6$-Alkyloxycarbonyl,

durch $C_2$—$C_6$-Alkenylthio, $C_2$—$C_6$-Alkenylsulfinyl oder $C_2$—$C_6$-Alkenylsulfonyl,

durch Phenyl oder Benzyl, die auch durch Halogen substituiert sein können,

durch 2′-Thienyl oder 3′-Thienyl,

durch Formyl,

durch $C_1$—$C_6$-Alkylcarbonyl, das auch durch Hydroxy substituiert sein kann,

durch Benzoyl oder $C_1$—$C_6$-Alkylcarbonylamino,

durch Carboxy oder $C_1$—$C_6$-Alkoxycarbonyl,

durch Carbamoyl, das am Stickstoff einmal substituiert sein kann durch $C_1$—$C_6$-Alkyl, Hydroxy-$C_1$—$C_6$-Alkyl, $C_1$—$C_6$-Alkoxycarbonyl, $C_1$—$C_6$-Alkylcarbonyl, Carboxymethyl, $C_1$—$C_6$-Alkoxycarbonylmethyl, Aminocarbonylmethyl, $C_1$—$C_6$-Alkylaminocarbonyl, Carbamoyl, Hydroxy oder Pyridyl, oder das am Stickstoff zweimal substituiert sein kann durch $C_1$—$C_6$-Alkyl,

durch Carbazoyl, das durch $C_1$—$C_4$-Alkyl substituiert sein kann, oder N-Carbamoylcarbazoyl,

durch Sulfamoyl, das am Stickstoff einmal substituiert sein kann durch $C_1$—$C_6$-Alkylaminocarbonyl,

durch Pyridyl oder 4-Pyridon-1-yl,

wobei bei diesen unter die allgemeine Formel I fallenden Verbindungen die $R^2$O-Gruppe in syn-Position steht und wobei für den Fall, daß

A. $R^1$ = Wasserstoff und

$R^2$ = Methyl ist,

A nicht sein kann Pyridinium, Chinolinium oder Pyridinium, das einfach substituiert ist durch 3-Methyl, 4-Methyl, 4-Hydroxymethyl, 4-Carboxymethyl, 4-Trifluormethyl, 3-Chlor, 3-Brom, 3-Jod, 4-Carbamoyl, 4-N-Hydroxymethyl-carbamoyl, 4-Methoxycarbonyl-carbamoyl, Hydroxycarbamoyl, N-Hydroxy-N-methyl-carbamoyl, N-Hydroxycarbamoylmethyl.

B. $R^1$ = Chlor und
$R^2$ = Methyl oder Äthyl ist
A nicht Pyridinium sein kann.

Diese Substituenten können an den genannten, an den Pyridiniumrest ankondensierten Ringen auftreten, unabhängig davon, ob der jeweilige Ring gesättigt, ungesättigt oder auch noch durch ein Heteroatom unterbrochen ist. Bevorzugt treten sie jedoch erfindungsgemäß an ankondensierten gesättigten Ringen auf, die kein Heteroatom enthalten.

Der an den Pyridiniumrest ankondensierte Ring kann 2 bis 10 Ringglieder (Di- bis Decamethylen), vorzugsweise jedoch 3 bis 5 Ringglieder enthalten und somit beispielsweise einen Cyclopenteno-, Cyclohexeno- oder Cyclohepteno-Ring darstellen. Enthält ein solcher ankondensierter Ring eine Doppelbindung, so seien als Beispiele ein Dehydrocyclopenteno-, Dehydrocyclohexeno- oder Dehydrocyclohepteno-Ring genannt. In derartigen Ringen kann ein C-Atom durch Sauerstoff oder Schwefel ersetzt sein. Als ein Sauerstoffatom enthaltende, ankondensierte Ringe, die zwei oder eine Doppelbindung enthalten, seien beispielsweise erwähnt Furo, Pyrano, Dihydrofuro und Dihydropyrano, als ankondensierte Ringe mit einem Schwefelatom, die zwei oder eine Doppelbindung enthalten Thieno, Thiopyrano, Dihydrothieno und Dihydrothiopyrano. Von den ein Sauerstoff- oder Schwefelatom enthaltenden, ankondensierten Ringen kommen für eine Substitution, insbesondere durch die vorstehend angegebenen Substituenten, insbesondere diejenigen Ringe in Betracht, die nur eine Doppelbindung enthalten.

Als besonders bevorzugt kommen beispielsweise die folgenden Substituenten in Betracht:

$R^1$: Wasserstoff,
Chlor und Fluor, insbesondere Chlor,

$R^2$: Wasserstoff,
$C_1$—$C_4$-Alkyl, wie z.B. Methyl, Äthyl, Propyl, Isopropyl, vorzugsweise Methyl, Äthyl, insbesondere Methyl,

A: ein Pyridiniumrest, der ein- oder mehrfach, vorzugsweise 1- bis 3-fach, insbesondere 1- bis 2-fach substituiert sein kann, beispielsweise durch
$C_1$—$C_4$-Alkyl, wie insbesondere Methyl, Äthyl, Propyl, Isopropyl, n-Butyl, sec.-Butyl, tert.-Butyl, Dimethyl, Trimethyl, Methyl und Äthyl, Methyl und Propyl, Methyl und Isopropyl, Äthyl und Äthyl,
Hydroxy-$C_1$—$C_4$-alkyl, wie insbesondere Hydroxymethyl, Hydroxyäthyl, Hydroxypropyl, Hydroxyisopropyl, Hydroxybutyl, Hydroxy-sec.-butyl oder Hydroxy-tert.-butyl, und wobei z.B. auch zwei oder drei Hydroxylgruppen am Alkylrest stehen können, Carboxy-$C_1$—$C_4$-alkyl, wie insbesondere Carboxymethyl und Carboxyäthyl,
$C_1$—$C_4$-Alkoxycarbonyl-$C_1$—$C_4$-alkyl, wie insbesondere Methoxycarbonylmethyl, Äthoxycarbonylmethyl, Methoxycarbonyl-Äthyl, Formyl-$C_1$—$C_4$-alkyl, wie insbes. Formylmethyl,
$C_1$—$C_4$-Alkylcarbonyl-$C_1$—$C_4$-alkyl, wie insbesondere Methylcarbonylmethyl, Äthylcarbonylmethyl, Methylcarbonyläthyl und Äthylcarbonyläthyl, deren beide Alkylgruppen auch noch durch Hydroxy substituiert sein können,
Carbamoyl-$C_1$—$C_4$-alkyl, wie insbesondere Carbamoylmethyl und Carbamoyläthyl, die am Stickstoff auch noch durch Hydroxy substituiert sein können, wie insbesondere N-Hydroxy-carbamoylmethyl,
Sulfo-$C_1$—$C_4$-alkyl, wie insbesondere Sulfoäthyl oder 1-Hydroxy-1-sulfomethyl,
$C_1$—$C_4$-Alkoxy-$C_1$—$C_4$-alkyl, wie insbesondere Methoxymethyl, Äthoxymethyl, Propoxymethyl, Isopropoxymethyl, Methoxyäthyl, Äthoxyäthyl, Methoxypropyl und Methoxyisopropyl, die auch durch Hydroxy substituiert sein können, wie insbesondere Hydroxyäthoxymethyl und Hydroxyäthoxyäthyl,
$C_1$—$C_4$-Alkylthio-$C_1$—$C_4$-alkyl, wie insbesondere Methylthiomethyl, Äthylthiomethyl, Methylthioäthyl und Äthylthioäthyl,
$C_1$—$C_4$-Alkylsulfinyl-$C_1$—$C_4$-alkyl, wie insbesondere Methylsulfinylmethyl, Äthylsulfinylmethyl, Methylsulfinyläthyl und Äthylsulfinyläthyl,
$C_1$—$C_4$-Alkylsulfonyl-$C_1$—$C_4$-alkyl, wie insbesondere Methylsulfonylmethyl, Äthylsulfonylmethyl, Methylsulfonyläthyl und Äthylsulfonyläthyl,
$C_3$-Alkenylloxy-$C_1$—$C_4$-alkyl, wie insbesondere Allyloxymethyl und Allyloxyäthyl,
$C_3$-Alkenylthio-$C_1$—$C_4$-alkyl, wie insbesondere Allylthiomethyl,
$C_3$-Alkenylsulfinyl-$C_1$—$C_4$-alkyl, wie insbesondere Allylsulfinylmethyl,
$C_3$-Alkenylsulfonyl-$C_1$—$C_4$-alkyl, wie insbesondere Allylsulfonylmethyl,
Cyano-$C_1$—$C_3$-alkyl, wie insbesondere Cyanomethyl und Cyanoäthyl,
Epoxy-$C_2$—$C_3$-alkyl, wie insbesondere Epoxyäthyl und Epoxypropyl,
Trifluormethyl, Hydroxyiminomethyl und $C_1$—$C_3$-Alkoxyiminomethyl, wie insbesondere Methoxyiminomethyl,
$C_3$—$C_4$-Alkenyl, wie insbesondere Allyl, 2-Methylallyl und Buten-3-yl, die auch noch durch Hydroxy substituiert sein können, wie insbesondere Hydroxyallyl und Hydroxybutenyl,
$C_3$-Alkinyl, wie insbesondere Propargyl,
$C_3$—$C_6$-Cycloalkyl und $C_3$—$C_6$-Cycloalkyl-methyl, wobei sich die Kohlenstoffzahl auf den Cycloalkylteil

bezieht, wie insbesondere Cyclopropyl, Cyclobutyl, Cyclopentyl, Cyclohexyl und Cyclopentylmethyl, wobei die Ringe auch substituiert sein können, z.B. durch Hydroxy, wie insbesondere 1-Hydroxy-1-cyclopentyl und 1-Hydroxy-1-cyclohexyl, oder durch Halogen, vorzugsweise Chlor, durch Carboxy, $C_1$—$C_4$-Alkoxycarbonyl oder Cyano,

$C_5$—$C_6$-Cycloalkenyl, wie insbesondere Cyclopenten-1-yl und Cyclohexen-1-yl,

$C_1$—$C_4$-Alkoxy, wie insbesondere Methoxy, Äthoxy, Propoxy, Isopropoxy, Butoxy, Isobutoxy und tert.-Butoxy, vorzugsweise Methoxy, wobei diese Alkoxygruppen auch noch substituiert sein können, beispielsweise durch Hydroxy, Carboxy oder $C_1$—$C_4$-Alkoxycarbonyl, insbesondere Carboxymethoxy und Methoxycarbonylmethoxy,

Epoxy-$C_2$—$C_3$-alkoxy, wie insbesondere Epoxyäthoxy oder Epoxypropoxy,

$C_3$-Alkenyloxy, wie insbesondere Allyloxy,

$C_3$-Alkinyloxy, wie insbesondere Propargyloxy, Halogen, wie insbesondere Fluor, Chlor, Brom, Jod, Cyano, Hydroxy, insbesondere 3-Hydroxy,

$C_1$—$C_4$-Alkylthio, wie insbesondere Methylthio, Äthylthio, Propylthio und Isopropylthio, die auch substituiert sein können durch Hydroxy, insbesondere Hydroxyäthylthio,

$C_1$—$C_4$-Alkylsulfinyl, wie insbesondere Methylsulfinyl, Äthylsulfinyl, Propylsulfinyl und Isopropylsulfinyl, die auch substituiert sein können durch Hydroxy, insbesondere Hydroxyäthylsulfinyl,

$C_1$—$C_4$-Alkylsulfonyl, wie Methyl- oder Äthyl- oder Propyl- oder Isopropylsulfonyl, die auch substituiert sein können durch Hydroxy, insbesondere Hydroxyäthylsulfonyl,

Carboxymethylthio und $C_1$—$C_4$-Alkoxycarbonylmethylthio, insbesondere Methoxycarbonylmethylthio, Carboxymethyl-sulfinyl und -sulfonyl, sowie $C_1$—$C_4$-Alkoxycarbonylmethyl-sulfinyl und -sulfonyl, insbesondere Methoxycarbonylmethyl-sulfinyl und -sulfonyl,

$C_3$-Alkenylthio, wie Allylthio und Propen-1-ylthio,

$C_3$-Alkenylsulfinyl, wie Allylsulfinyl und Propen-1-ylsulfinyl,

$C_3$-Alkenylsulfonyl, wie Allylsulfonyl und Propen-1-ylsulfonyl,

Phenyl und Benzyl, die auch substituiert sein können, beispielsweise durch Halogen, insbesondere Chlor, wie z.B. 4-Chlorbenzyl,

2'-Thienyl und 3'-Thienyl,

Formyl,

$C_1$—$C_4$-Alkylcarbonyl, insbesondere Acetyl und Propionyl, vorzugsweise Acetyl, die auch durch Hydroxy substituiert sein können,

Benzoyl,

$C_1$—$C_4$-Alkylcarbonylamino, insbesondere Acetylamino und Propionylamino,

Formylamino,

Carboxy, beispielsweise auch 2,3,4-Carboxy,

$C_1$—$C_4$-Alkoxycarbonyl, insbesondere Methoxycarbonyl und Äthoxycarbonyl, wie z.B. auch 2,3,4-Methoxy- oder -Äthoxycarbonyl,

Carbamoyl(beispielsweise auch 2,3,4-Carbamoyl), das am Stickstoffatom einmal substituiert sein kann durch $C_1$—$C_4$-Alkyl, wie insbesondere N-Methyl- und N-Äthyl-carbamoyl, durch Hydroxy-$C_1$—$C_4$-alkyl, wie insbesondere N-Hydroxymethyl-carbamoyl und N-Hydroxyäthyl-carbamoyl, durch $C_1$—$C_4$-Alkoxycarbonyl, wie insbesonder N-Methoxycarbonylcarbamoyl und N-Äthoxycarbonylcarbamoyl, durch $C_1$—$C_4$-Alkylcarbonyl, wie insbesondere N-Acetyl-carbamoyl, durch Carboxymethyl, durch $C_1$—$C_4$-Alkoxycarbonylmethyl, wie insbesondere N-Meth- und N-Äthoxycarbonylmethylcarbamoyl, durch Aminocarbonylmethyl, durch N-$C_1$—$C_4$-Alkylaminocarbonyl, wie insbesondere N-Methyl- und N-Äthylaminocarbonylcarbamoyl, durch Carbamoyl (=Ureidocarbonyl), durch Hydroxy oder Pyridyl, wie insbesondere N-3'-Pyridylcarbamoyl und N-4'-Pyridylcarbamoyl,

N-$C_1$—$C_4$-Dialkyl-carbamoyl, wie insbesondere N,N-Dimethylcarbamoyl und N,N-Diäthylcarbamoyl,

Carbazoyl, das substituiert sein kann durch $C_1$—$C_4$-Alkyl, insbesondere Methyl oder Äthyl, durch Carbamoyl, wie N-Carbamoyl-carbazoyl,

Sulfamoyl, das am Stickstoffatom substituiert sein kann durch $C_1$—$C_4$-Alkylaminocarbonyl, wie insbesondere Äthylaminocarbonylsulfamoyl,

Pyridyl, wie insbesondere 2'-, 3'- und 4'-Pyridyl und 4-Pyridon-1-yl.

Stellt A einen Pyridiniumrest dar, der durch zwei zu einem Di- bis Decamethylen-Ring geschlossene Alkylgruppen substituiert ist, der wiederum ein- oder mehrfach, vorzugsweise einfach substituiert sein und eine oder zwei Doppelbindungen enthalten kann, so kommen hiefür ganz besonders die folgenden ankondensierten Ringsysteme in Betracht:

Cyclopenteno, Hydroxycyclopenteno, Chlorcyclopenteno, Bromcyclopenteno, Oxocyclopenteno, Hydroxymethylcyclopenteno, Exomethylen-cyclopenteno, Carboxycyclopenteno, $C_1$—$C_4$-Alkoxycarbonyl-cyclopenteno, insbesondere Methoxycarbonylcyclopenteno und Carbamoyl-cyclopenteno,

Cyclohexeno, Hydroxycyclohexeno, Chlor- oder Bromcyclohexeno, Oxocyclohexeno, Hydroxymethyl-cyclohexeno, Exomethylen-cyclohexeno, Carboxycyclohexeno, $C_1$—$C_4$-Alkoxycarbonyl-cyclohexeno, insbesondere Methoxycarbonylcyclohexeno und Carbamoylcyclohexeno,

Cyclohepteno, Hydroxy-, Chlor-, Brom-, Oxo-, Hydroxymethyl-, Exomethylen- oder Carboxy-cyclohepteno, $C_1$—$C_4$-Alkoxycarbonylcyclohepteno, insbesondere Methoxycarbonylcyclohepteno und Carbamoyl-cyclohepteno, Dehydro-cyclopenteno, Dehydro-cyclohexeno und Dehydro-cyclohepteno.

Ist in den vorstehend genannten ankondensierten Ringsystemen ein C-Atom durch Sauerstoff oder Schwefel, ersetzt, so kommen insbesondere in Betracht: 2,3- und 3,4-Furo, 2,3- und 3,4-Pyrano, 2,3- und 3,4-Dihydrofuro, 2,3- und 3,4-Dihydropyrano, Methyl-dihydrofuro, Methoxydihydropyrano und Hydroxydihydropyrano.

Gegenstand der Erfindung ist weiterhin ein Verfahren zur Herstellung von Verbindungen der Formel I und ihrer physiologisch verträglichen Säureadditionssalze, das dadurch gekennzeichnet ist, daß man

a) eine Verbindung der allgemeinen Formel II

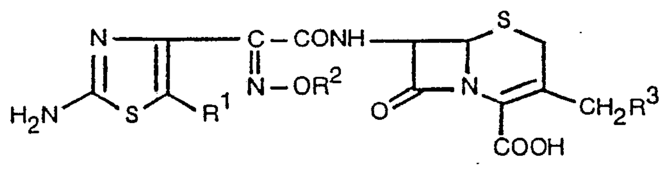

(II)

oder deren Salze, worin $R^1$ und $R^2$ die oben genannte Bedeutung haben und $R^3$ eine durch Pyridin oder substituierte Pyridine, die den Pyridiniumresten A der Formel I entsprechen, austauschbare Gruppe bedeutet, mit Pyridin oder einem solchen Pyridinderivat umsetzt oder

b) eine 7-Amino-cephemverbindung der allgemeinen Formel III

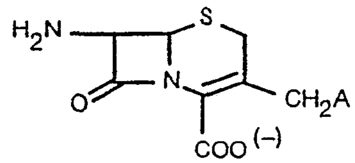

(III)

oder deren Säureadditionssalze, wodei die Aminogruppe auch in Form eines reaktionsfähigen Derivats vorliegen kann, mit einer 2-(2-Aminothiazol-4-yl)-2-syn-oximino-essigsäure der allgemeinen Formel IV

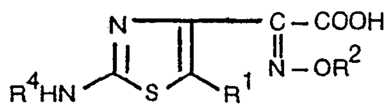

(IV)

worin $R^1$ und $R^2$ die obige Bedeutung besitzen und $R^4$ für Wasserstoff oder eine Aminoschutzgruppe steht oder mit einem aktivierten Derivat dieser Verbindung umsetzt und

α) eine gegebenenfalls vorhandene Schutzgruppe abspaltet und

β) falls erforderlich, das erhaltene Produkt in ein physiologisch verträgliches Säureadditionssalz überführt.

Soll die Herstellung der Verbindungen der allgemeinen Formel I durch nucleophilen Austausch von $R^3$ in den Verbindungen der allgemeinen Formel II durch Pyridin oder eines der angegebenen Pyridinderivate erfolgen, so kommen als Reste $R^3$ insbesondere in Betracht Acyloxyreste von niederen aliphatischen Carbonsäuren, vorzugsweise mit 1 bis 4 C-Atomen, wie z.B. Acetoxy oder Propionyloxy, insbesondere Acetyoxy, die gegebenenfalls substituiert sein können, wie z.B. Chloracetoxy oder Acetylacetoxy. Für $R^3$ kommen auch andere Gruppen in Betracht, wie beispielsweise Halogen, insbesondere Chlor oder Brom, oder Carbamoyloxy.

Erfindungsgemäß werden bei der nucleophilen Austausch-reaktion Ausgangsverbindungen der allgemeinen Formel II, in denen $R^3$ für Acetoxy steht, eingesetzt, oder deren Salze, wie z.B. ein Natrium- oder Kaliumsalz. Die Reaktion wird in einem Lösungsmittel, vorzugsweise in Wasser, oder in einem Gemisch aus Wasser und einem mit Wasser leicht mischbaren organischen Lösungsmittel, wie z.B. Aceton, Dioxan, Acetonitril, Dimethylformamid, Dimethylsulfoxid oder Äthanol durchgeführt. Die Reaktionstemperatur liegt im allgemeinen im Bereich von etwa 10 bis etwa 100°C, vorzugsweise zwischen 20 und 80°C. Die Pyridinkomponente wird in Mengen zugegeben, die zwischen etwa Äquimolaren Mengen und einem bis zu etwa 5-fachen Überschuß liegt. Der Austausch des Restes $R^3$ wird durch Anwesenheit von Neutralsalzionen, vorzugsweise von Jodid- oder Thiocyanationen, im Reaktionsmedium erleichtert. Insbesondere werden etwa 10 bis etwa 30 Äquivalente Kaliumjodid, Natriumjodid, Kaliumthiocyanat oder Natriumthiocyanat zugegeben. Die Reaktion wird vorteilhaft in der Nähe des Neutralpunktes, vorzugsweise bei einem pH-Wert im Bereich von etwa 5 bis etwa 8 durchgeführt.

Für den Fall, daß $R^3$ für eine Carbamoyloxygruppe steht, wird die Austauschreaktion analog durchgeführt. Steht $R^3$ für Halogen, insbesondere Brom, so erfolgt der Austausch in literaturbekannter Weise.

Die Acylierung der Verbindungen der allgemeinen Formel III oder von deren Additionssalzen, beispielsweise mit Chlorwasserstoffsäure, Bromwasserstoffsäure, Salpetersäure, Phosphorsäure, oder einer organischen Säure, wie z.B. Methansulfonsäure oder p-Toluolsulfonsäure, kann mit Carbonsäuren der allgemeinen Formel IV oder mit einem reaktionsfahigen Derivat einer solchen Säure durchgeführt werden. In manchen Fällen ist es dabei von Vorteil, die 2-Aminogruppe in den Verbindungen der

allgemeinen Formel IV vor der Umsetzung zu schützen. Als Aminoschutzgruppen $R^4$ eignen sich z.B. gegebenenfalls substituiertes Alkyl, wie beispielsweise tert.-Butyl, tert.-Amyl, Benzyl, p-Methoxybenzyl, Trityl, Benzyhydryl, vorzugsweise Trityl, Trialkylsilyl, wie beispielsweise Trimethyllsilyl, gegebenenfalls substituiertes aliphatisches Acyl, wie z.B. Formyl, Chloracetyl, Bromacetyl, Trichloracetyl und Trifluoracetyl, vorzugsweise Chloracetyl oder gegebenenfalls substituiertes Alkoxycarbonyl wie beispielsweise Trichloräthoxycarbonyl, Benzyloxycarbonyl oder tert.-Butyloxycarbonyl, vorzugsweise tert.-Butyloxycarbonyl und Benzyloxycarbonyl.

Die Schutzgruppe kann nach der Acylierung in an sich bekannter Weise abgespalten werden, z.B. die Tritylgruppe mittels einer Carbonsäure, wie z.B. Essigsäure oder Ameisensäure, oder die Chloracetylgruppe mittels Thioharnstoff.

Werden die Carbonsäuren der allgemeinen Formel IV sowie ihre an der Aminogruppe geschützten Derivate selbst als Acylierungsmittel eingesetzt, so wird zweckmäßig in Gegenwart eines Kondensationsmittels, beispielsweise eines Carbodiimids, wie beispielsweise N,N'-Dicyclohexylcarbodiimid gearbeitet.

Die Aktivierung der Carbonsäuren der allgemeinen Formel IV kann in besonders günstiger Weise durch Behandlung mit bestimmten Carbonsäureamiden und beispielsweise Phosgen, Phosphorpentachlorid, Tosylchlorid, Thionylchlorid oder Oxalylchlorid erfolgen, wie sie in der deutschen Patentschrift 28 04 040 beschrieben wird.

Als aktivierte Derivate der Carbonsäuren der allgemeinen Formel IV eignen sich insbesondere auch Halogenide, vorzugsweise Chloride, die in an sich bekannter Weise durch Behandlung mit Halogenierungsmitteln, wie z.B. Phosphorpentachlorid, Phosgen oder Thionylchlorid unter für die Cephalosporinchemie literaturbekannten, schonenden Reaktionsbedingungen erhalten werden.

Als aktivierte Derivate der Carbonsäuren der allgemeinen Formel IV eigenen sich ferner die Anhydride und gemischten Anhydride, Azide und aktivierten Ester, vorzugsweise mit p-Nitrophenol, 2,4-Dinitrophenol, Methylencyanhydrin, N-Hydroxysuccinimid und N-Hydroxyphthalimid, insbesondere diejenigen mit 1-Hydroxybenzotriazol und 6-Chlor-1-hydroxy-benzotriazol. Als gemischte Anhydride sind besonders geeignet solche mit niederen Alkansäuren, wie z.B. Essigsäure, und besonders bevorzugt solche mit substituierten Essigsäuren, wie z.B. Trichloressigsäure, Pivalinsäure oder Cyanessigsäure. Besonders geeignet sind aber auch die gemischten Anhydride mit Kohlensäurehalbestern, die man beispielsweise durch Umsetzung der Carbonsäuren der Formel IV, in denen die Aminogruppe geschützt ist, mit Chlorameisensäure-benzylester, -p-nitrobenzylester, -iso-butylester, -ethylester oder -allylester gewinnt. Die aktivierten Derivate können als isolierte Substanzen, aber auch in situ umgesetzt werden.

Im allgemeinen erfolgt die Umsetzung der Cephemderivate der allgemeinen Formel III mit einer Carbonsäure der allgemeinen Formel IV oder einem aktivierten Derivat derselben in Gegenwart eines inerten Lösungsmittels. Insbesondere eignen sich chlorierte Kohlenwasserstoffe, wie vorzugsweise Methylenchlorid und Chloroform; Äther, wie z.B. Diäthyläther, vorzugsweise Tetrahydrofuran und Dioxan; Ketone, wie vorzugsweise Aceton und Butanon; Amide, wie vorzugsweise Dimethylformamid und Dimethylacetamid oder Wasser. Es kann sich auch als vorteilhaft erweisen, Gemische der genannten Lösungsmittel zu verwenden. Dies ist oftmals dann der Fall, wenn die Cephemverbindung der allgemeinen Formel III mit einem in situ erzeugten aktivierten Derivat einer Carbonsäure der Formel IV umgesetzt wird.

Die Umsetzung von Cephemverbindungen der Formel III mit Carbonsäuren der Formel IV bzw. deren aktivierten Derivaten kann in einer Temperaturbereich von etwa −80 bis etwa +80°C vorzugsweise zwischen −30 und +50°C, insbesondere jedoch zwischen etwa −20°C und Raumtemperatur erfolgen.

Die Reaktionsdauer hängt von den Reaktanten, der Temperatur und dem Lösungsmittel bzw. dem Lösungsmittelgemisch ab und liegt normalerweise zwischen etwa 1/4 und etwa 72 Stunden.

Die Reaktion mit Säurehalogeniden kann gegebenenfalls in Gegenwart eines säurebindenden Mittels zur Bindung des freigesetzten Halogenwasserstoffs durchgeführt werden. Als solche eignen sich insbesondere tertiäre Amine, wie z.B. Triäthylamin oder Dimethylanilin, anorganische Basen; wie z.B. Kaliumcarbonat oder Natriumcarbonat, Alkylenoxide, wie z.B. Propylenoxid. Auch die Anwesenheit eines Katalysators, wie z.B. von Dimethylaminopyridin kann gegebenenfalls von Vorteil sein.

Liegt in den Verbindungen der allgemeinen Formel III die Aminogruppe in Form eines reaktionsfähigen Derivats vor, so kann es sich um ein solches handeln, wie es aus der Literatur für Amidierungen bekannt ist. So kommen beispielsweise Silylderivate in Betracht, die bei der Umsetzung von Verbindungen der allgemeinen Formel III mit einer Silylverbindung gebildet werden, wie z.B. Trimethylsilylchlorsilan oder Bis-(trimethylsilyl)-acetamid. Wird die Umsetzung mit einer solchen, an der Aminogruppe aktivierten Verbindung durchgeführt, so ist est zweckmäßig, die Reaktion in einem inerten Lösungsmittel, wie z.B. Methylenchlorid, Tetrahydrofuran oder Dimethylformamid durchzuführen.

Als physiologisch verträgliche Säureadditionssalze der Verbindungen der allgemeinen Formel I seien beispielsweise erwähnt solche mit Chlorwasserstoffsäure, Bromwasserstoffsäure, Salpetersäure, Phosphorsäure oder organischen Säuren, wie z.B. Methansulfonsäure oder p-Toluolsulfonsäure.

Die Verbindungen der allgemeinen Formel III können in an sich bekannter Weise (vgl. z.B. DOS 30 19 838), beispielsweise aus der an der Aminogruppe geschützten 7-Aminocephalosporansäure auf dieselbe Weise erhalten werden, wie sie vorstehend für den nucleophilen Austausch von $R^3$ beschrieben wurde.

Die Verbindungen der allgemeinen Formel IV sowie die den Pyridiniumresten A entsprechenden Pyridinderivate sind literaturbekannt oder nach literaturbekannten Verfahren herstellbar.

Die erfindungsgemäß erhaltenen Verbindungen der allgemeinen Formel I und ihre physiologisch verträglichen Säureadditionssalze zeigen bemerkenswert gute antibakterielle Wirksamkeiten, sowohl gegen grampositive als such gramnegative bakterielle Keime.

Auch gegen penicillinase- und cephalosporinase-bildene Bakterien sind die Verbindungen der Formel I unerwartet gut wirksam. Da sie zudem günstige toxikologische und pharmakologische Eigenschaften zeigen, stellen sie wertvolle Chemotherapeutika dar.

Die Erfindung betrifft somit auch Arzneipräparate zur Behandlung von mikrobiellen Infektionen, die durch einen Gehalt an einer oder mehreren der erfindungsgemäßen Verbindungen charakterisiert sind.

Die erfindungsgemäßen Produkte können auch in Kombination mit anderen Wirkstoffen, beispielsweise aus der Reihe der Penicilline, Cephalosporine oder Aminoglykoside zur Anwendung kommen.

Die Verbindungen der allgemeinen Formel I und ihre physiologisch verträglichen Säureadditionssalze können oral, intramuskulär oder intravenös verabfolgt werden. Arzneipräparate, die eine oder mehrere Verbindungen der allgemeinen Formel I als Wirkstoff enthalten, können hergestellt werden, indem man die Verbindungen der Formel I mit einem oder mehreren pharmakologisch verträglichen Trägerstoffen oder Verdünnungsmitteln, wie z.B. Füllstoffen, Emulgatoren, Gleitstoffen, Geschmackskorrigentien, Farbstoffen oder Puffersubstanzen vermischt und in eine geeignete galenische Zubereitungsform bringt, wie beispielsweise Tabletten, Dragees, Kapseln, oder eine zur parenteralen Applikation geeignete Suspension oder Lösung.

Als Träger- oder Verdünnungsmittel seien beispielsweise erwähnt Traganth, Milchzucker, Talkum, Agar-Agar, Polyglykole, Äthanol und Wasser. Für die parenterale Applikation kommen vorzugsweise Suspensionen oder Lösungen in Wasser in Betracht. Es ist auch möglich, die Wirkstoffe als solche ohne Träger- oder Verdünnungsmittel in geeigneter Form, beispielsweise in Kapseln, zu applizieren.

Geeignete Dosen der Verbindungen der allgemeinen Formel I oder ihrer physiologisch verträglichen Säureadditionssalze liegen bei etwa 0,4 bis 20 g/Tag, vorzugsweise bei 0,5 bis 4 g/Tag für einen Erwachsenen von etwa 60 kg Körpergewicht.

Es können Einzel- oder im allgemeinen Mehrfachdosen verabreicht werden, wobei die Einzeldosis den Wirkstoff in einer Menge von etwa 50 bis 1000 mg vorzugsweise von etwa 100 bis 500 mg enthalten kann.

Cephem-Verbindungen, die in 3-Stellung des Cephemrings einen substituierten Pyridiniummethylrest tragen, sind aus den deutschen Offenlegungsschriften 29 21 316 und 27 16 707 bekannt.

In der FR—A—23 48 218 werden antibiotisch wirksame Verbindungen beschrieben, in denen $R^1$ für Wasserstoff, $R^2$ für Methyl und A für Pyridinium oder 4-Carbamoylpyridinium steht.

Erfindungsgemäß lassen sich außer den in den Ausführungsbeispielen beschriebenen Produkten beispielsweise auch Verbindungen herstellen, die der allgemeinen Formel I' entsprechen.

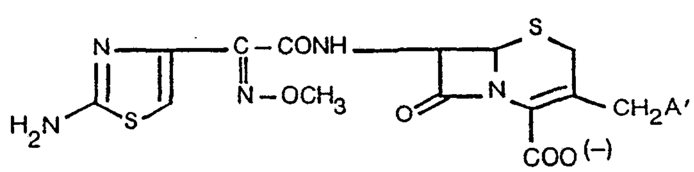

I'

in der —$OCH_3$ in syn-Position steht und A'

a) einen Pyridiniumrest darstellt, der durch die in Tabelle 1 angegebenen Reste substituiert ist oder

b) einen der in Tabelle 2 wiedergegebenen Rest bedeutet.

In Tabelle I geben die Zahlen die Stellung des (oder der) Substituenten am Pyridiniumrest wieder.

0 064 740

TABELLE 1

| | | |
|---|---|---|
| 2,3-di-$CH_3$ | 4-$CH_2C{\equiv}CH$ | 2-$CH_2CH_2OH$ |
| 2,5-di-$CH_3$ | | 3-$CH_2CH_2OH$ |
| 2-Propyl | | 4-$CH_2CH_2OH$ |
| 3-Propyl | | 2-$CH_2CH_2OH$-4-$CH_3$ |
| 2-Isopropyl | 2 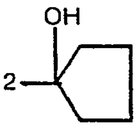 | 2-$CH_2CH_2OH$-3-$CH_3$ |
| 2-n-Butyl | | 2-$CH_2CH_2OH$-5-$CH_3$ |
| 3-n-Butyl | | 3-$CH_2CH_2OH$-2-$CH_3$ |
| 4-n-Butyl | | 3-$CH_2CH_2OH$-4-$CH_3$ |
| 2-sec-Butyl | | 3-$CH_2CH_2OH$-5-$CH_3$ |
| 3-sec-Butyl | 3 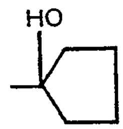 | 3-$CH_2CH_2OH$-6-$CH_3$ |
| 4-sec-Butyl | | 4-$CH_2CH_2OH$-3-$CH_3$ |
| 2-tert-Butyl | | 4-$CH_2CH_2OH$-2-$CH_3$ |
| 3-tert-Butyl | | 2-$CH(CH_3)OH$-3-$CH_3$ |
| 2-$C_2H_5$-3-$CH_3$ | | 2-$CH(CH_3)OH$-4-$CH_3$ |
| 2-$C_2H_5$-4-$CH_3$ | 4 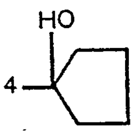 | 2-$CH(CH_3)OH$-5-$CH_3$ |
| 2-$C_2H_5$-5-$CH_3$ | | 3-$CH(CH_3)OH$-2-$CH_3$ |
| 3-$C_2H_5$-2-$CH_3$ | | 3-$CH(CH_3)OH$-4-$CH_3$ |
| 3-$C_2H_5$-5-$CH_3$ | | 3-$CH(CH_3)OH$-5-$CH_3$ |
| 4-$C_2H_5$-2-$CH_3$ | | 3-$CH(CH_3)OH$-6-$CH_3$ |
| 4-$C_2H_5$-3-$CH_3$ | | 4-$CH(CH_3)OH$-2-$CH_3$ |
| 2,3,4-tri$CH_3$ | 2 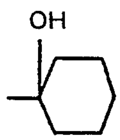 | 4-$CH(CH_3)OH$-3-$CH_3$ |
| 2,3,5-tri$CH_3$ | | 2-$CH(C_2H_5)OH$ |
| 2,4,5-tri$CH_3$ | | 3-$CH(C_2H_5)OH$ |
| 3,4,5-tri$CH_3$ | | 4-$CH(C_2H_5)OH$ |
| 2-$CH_2CH{=}CH_2$ | | 2-$CH(CH_3)CH_2OH$ |
| 3-$CH_2CH{=}CH_2$ | 3 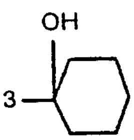 | 3-$CH(CH_3)CH_2OH$ |
| 4-$CH_2CH{=}CH_2$ | | 4-$CH(CH_3)CH_2OH$ |
| 2-$CH_2CH_2CH{=}CH_2$ | | 4-$CH(CH_3)CH_2OH$-5-$CH_3$ |
| 3-$CH_2CH_2CH{=}CH_2$ | | 2-$(CH_2)_3OH$ |
| 4-$CH_2CH_2CH{=}CH_2$ | | 3-$(CH_2)_3OH$ |
| 2-$CH_2C(CH_3){=}CH_2$ | 4 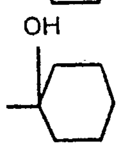 | 4-$(CH_2)_3OH$ |
| 3-$CH_2C(CH_3){=}CH_2$ | | 2-$CH_2CH(OH)CH_3$ |
| 4-$CH_2C(CH_3){=}CH_2$ | | 3-$CH_2CH(OH)CH_3$ |
| 2-Cyclopropyl | 2-$CH_2OH$-3-$CH_3$ | 4-$CH_2CH(OH)CH_3$ |
| 3-Cyclopropyl | 2-$CH_2OH$-4-$CH_3$ | 2-$CH_2CH(OH)CH_3$-4-$CH_3$ |
| 4-Cyclopropyl | 2-$CH_2OH$-5-$CH_3$ | 2-$C(CH_3)_2OH$-4-$CH_3$ |
| 2-Cyclobutyl | 3-$CH_2OH$-2-$CH_3$ | 3-$C(CH_3)_2OH$-6-$CH_3$ |
| 3-Cyclobutyl | 3-$CH_2OH$-4-$CH_3$ | 4-$C(CH_3)_2OH$-3-$CH_3$ |
| 4-Cyclobutyl | 3-$CH_2OH$-5-$CH_3$ | 2-$CH(CH_3)OH$-4-$C_2H_5$ |
| 2-Cyclopentyl | 3-$CH_2OH$-6-$CH_3$ | 2-$CH_2CH_2OH$-5-$C_2H_5$ |
| 3-Cyclopentyl | 4-$CH_2OH$-2-$CH_3$ | 3-$CH(CH_3)OH$-2,5-di$CH_3$ |
| 4-Cyclopentyl | 4-$CH_2OH$-3-$CH_3$ | 4-$CH_2CH_2OH$-3,5-di$CH_3$ |
| 2-Cyclopentylmethyl | 2-$CH_2OH$-3-$C_2H_5$ | 2-$CH(C_3H_7)OH$ |
| 3-Cyclopentylmethyl | 2-$CH_2OH$-4-$C_2H_5$ | 3-$CH(C_3H_7)OH$ |
| 4-Cyclopentylmethyl | 2-$CH_2OH$-5-$C_2H_5$ | 4-$CH(C_3H_7)OH$ |
| 2-Cyclohexyl | 3-$CH_2OH$-2-$C_2H_5$ | 2-$CH(C_2H_5)CH_2OH$ |
| 3-Cyclohexyl | 3-$CH_2OH$-4-$C_2H_5$ | 3-$CH(C_2H_5)CH_2OH$ |
| 4-Cyclohexyl | 3-$CH_2OH$-5-$C_2H_5$ | 4-$CH(C_2H_5)CH_2OH$ |
| 2-Cyclopentyl-3-$CH_3$ | 3-$CH_2OH$-6-$C_2H_5$ | 2-$CH_2(CH_2)_3OH$ |
| 2-Cyclopentyl-4-$CH_3$ | 4-$CB_2OH$-2-$C_2H_5$ | 3-$CH_2(CH_2)_3OH$ |
| 2-Cyclopentyl-5-$CH_3$ | 4-$CH_2OH$-3-$C_2H_5$ | 4-$CH_2(CH_2)_3OH$ |
| 3-Cyclopentyl-4-$CH_3$ | 2-$CH_2OH$-3,4-di$CH_3$ | 2-$CH(CH_3)CH_2CH_2OH$ |
| 3-Cyclopentyl-5-$CH_3$ | 2-$CH_2OH$-3,5-di$CH_3$ | 3-$CH(CH_3)CH_2CH_2OH$ |
| 4-Cyclopentyl-2-$CH_3$ | 2-$CH_2OH$-4,5-di$CH_3$ | 4-$CH(CH_3)CH_2CH_2OH$ |
| 4-Cyclopentyl-3-$CH_3$ | 3-$CH_2OH$-2,4-di$CH_3$ | |
| 2-(1-Cyclopenten-1-yl) | 3-$CH_2OH$-2,5-di$CH_3$ | $CH_3$ |
| 3-(1-Cyclopenten-1-yl) | 3-$CH_2OH$-4,5-di$CH_3$ | |
| 4-(1-Cyclopenten-1-yl) | 3-$CH_2OH$-4,6-di$CH_3$ | 2-$C(C_2H_5)OH$ |
| 2-(1-Cyclohexen-1-yl) | 3-$CH_2OH$-5,6-di$CH_3$ | |
| 3-(1-Cyclohexen-1-yl) | 4-$CH_2OH$-2,3-di$CH_3$ | |
| 4-(1-Cyclohexen-1-yl) | 4-$CH_2OH$-2,5-di$CH_3$ | $CH_3$ |
| 2-$CH_2C{\equiv}CH$ | 4-$CH_2OH$-3,6-di$CH_3$ | |
| 3-$CH_2C{\equiv}CH$ | 3-$CH_2OH$-4,5,6-tri$CH_3$ | 3-$C(C_2H_5)OH$ |

TABELLE 1 — cont.

$CH_3$
|
$4-C(C_2H_5)OH$

$2-CH_2C(CH_3)_2OH$
$3-CH_2C(CH_3)_2OH$
$4-CH_2C(CH_3)_2OH$
$4-C(=CH_2)CH_2OH$
$3-C(=CH_2)CH_2OH$
$2-C(=CH_2)CH_2OH$
$4-CH(OH)CH=CH_2$
$2-CH(OH)CH=CH_2$
$3-CH(OH)CH=CH_2$
$2-CH(OH)CH_2CH=CH_2$

$3-CHCH_2CH=CH_2$
|
OH

$4-CHCH_2CH=CH_2$
|
OH

$2,3-di-CH_2OH$
$2,5-di-CH_2OH$
$2,4-di-CH_2OH$
$3,4-di-CH_2OH$
$3,5-di-CH_2OH$
$2-CH_2OH-3-OH$
$2-CH_2OH-3-OH-6-CH_3$
$2-CH-(CH_2OH)_2$
$3-CH(CH_2OH)_2$
$4-CH(CH_2OH)_2$
$3-C(CH_2OH)_3$
$4-C(CH_2OH)_3$
$2-CHOHCH_2OH$
$3-CHOHCH_2OH$
$4-CHOHCH_2OH$
$2-COCH_2OH$
$3-COCH_2OH$
$4-COCH_2OH$
$3-CH_2COCH_3$
$2-CH_2COCH_3$
$4-CH_2COCH_3$

$3-CHCH_2COCH_3$
|
OH

$4-CHCH_2COCH_3$
|
OH

$4-CH—COCH_3$
|
OH

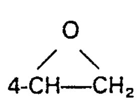

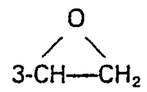

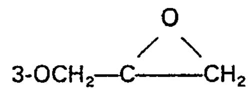

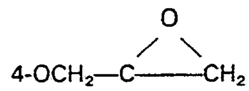

$4-CH_2CHO$
$3-CH_2CHO$
$2-CH_2CHO$
$2-OCH_3$
$3-OCH_3$
$2-OCH_3-3-CH_3$
$2-OCH_3-4-CH_3$
$2-OCH_3-5-CH_3$
$3-OCH_3-2-CH_3$
$3-OCH_3-4-CH_3$
$3-OCH_3-5-CH_3$
$3-OCH_3-6-CH_3$
$4-OCH_3-2-CH_3$
$4-OCH_3-3-CH_3$
$2-OC_2H_5$
$3-OC_2H_5$
$4-OC_2H_5$
$2-OC_2H_5-3-CH_3$
$2-OC_2H_5-4-CH_3$
$2-OC_2H_5-5-CH_3$
$3-OC_2H_5-2-CH_3$
$3-OC_2H_5-4-CH_3$
$3-OC_2H_5-5-CH_3$
$3-OC_2H_5-6-CH_3$
$4-OC_2H_5-2-CH_3$
$4-OC_2H_5-3-CH_3$
$2-OCH_3-4-C_2H_5$
$4-OCH_3-2,5-diCH_3$
$2-OCH(CH_3)_2$
$3-OCH(CH_3)_2$
$4-OCH(CH_3)_2$
$4-OCH(CH_3)_2-2-CH_3$
2-butoxy
3-butoxy
4-butoxy
$4$-butoxy-$2-CH_3$
2-Isobutoxy
3-Isobutoxy
4-Isobutoxy
2-tert butoxy
3-tert butoxy
4-tert butoxy
$2-OCH_2—CH=CH_2$
$3-OCH_2—CH=CH_2$
$4-O—CH_2—CH=CH_2$
$2-OCH_2CH_2OH$
$3-OCH_2CH_2OH$
$4-OCH_2CH_2OH$
$2-CH_2—OCH_3$
$3-CH_2—OCH_3$
$3-CH_2OCH_3-2CH_3$

$2-CH_2OC_2H_5$
$3-CH_2OC_2H_5$
$4-CH_2OC_2H_5$
$2-CH_2OC_3H_7$
$3-CH_2OC_3H_7$
$4-CH_2OC_3H_7$
$2-CH_2OCH(CH_3)_2$
$3-CH_2OCH(CH_3)_2$
$4-CH_2OCH(CH_3)_2$
$2-CH_2OCH_2CH=CH_2$
$3-CH_2OCH_2CH=CH_2$
$4-CH_2OCH_2CH=CH_2$
$2-CH_2CH_2OCH=CH_2$
$3-CH_2CH_2OCH=CH_2$
$4-CH_2CH_2OCH=CH_2$
$2-CH_2CH_2OCH_3$
$3-CH_2CH_2OCH_3$
$4-CH_2CH_2OCH_3$
$3-CH_2CH_2OCH_3-4-CH_3$
$2-CH_2CH_2OC_2H_5$
$3-CH_2CH_2OC_2H_5$
$4-CH_2CH_2OC_2H_5$
$2-CH(OCH_3)CH_3$
$3-CH(OCH_3)CH_3$
$4-CH(OCH_3)CH_3$
$2-CH(OC_2H_5)CH_3$
$3-CH(OC_2H_5)CH_3$
$4-CH(OC_2H_5)CH_3$
$2-(CH_2)_3OCH_3$
$3-(CH_2)_3OCH_3$
$4-(CH_2)_3OCH_3$

$2-C(OCH_3)CH_3$
|
$CH_3$

$3-C(OCH_3)CH_3$
|
$CH_3$

$4-C(OCH_3)CH_3$
|
$CH_3$

$3-OH-2-C_2H_5$
$3-OH-4-C_2H_5$
$3-OH-5-C_2H_5$
$3-OH-6-C_2H_5$
$3-OH-2,4-di-CH_3$
$3-OH-2,5-diCH_3$
$3-OH-4,5-diCH_3$
$3-OH-4,6-diCH_3$
$3-OH-2-CH_3$
$3-OH-4-CH_3$
$3-OH-5-CH_3$
$3-OH-6-CH_3$
$3-OH-2-C_2H_5-5-CH_3$
$3-OH-5-C_2H_5-2-CH_3$
$3-OH-2-HC(CH_3)_2$
$3-OH-2-CH_2CH_2CH_3$
3-OH-2-butyl
3-OH-2-sec.butyl
3-OH-2-tert.butyl

TABELLE 1 — cont.

| | | |
|---|---|---|
| 3-OH-4-butyl | 2-$CH_2SOCH_3$ | 2-$OCH_3$-3-Br |
| 3-OH-5-sec.butyl | 4-$CH_2SOCH_3$ | 4-$OCH_3$-3-Br |
| 3-OH-2,4,5-tri-$CH_3$ | 2-$CH_2SO_2CH_3$ | 5-$OCH_3$-3-Br |
| 3-OH-4,5,6-tri-$CH_3$ | 4-$CH_2SO_2CH_3$ | 6-$OCH_3$-3-Br |
| 3-OH-6-$CH=CH(CH_3)$ | 2-$CH_2SC_2H_5$ | 2-$OCH_3$-3-Cl |
| 3-OH-2-$CH=CH(CH_3)$ | 3-$CH_2SC_2H_5$ | 4-$OCH_3$-3-Cl |
| 3-OH-4-$CH_2CH=CH_2$ | 4-$CH_2SC_2H_5$ | 5-$OCH_3$-3-Cl |
| 3-OH-2-Cl | 2-$CH_2SOC_2H_5$ | 6-$OCH_3$-3-Cl |
| 3-OH-5-Cl | 3-$CH_2SOC_2H_5$ | 2-$OCH_3$-3-F |
| 3-OH-6-Cl | 4-$CH_2SOC_2H_5$ | 4-$OCH_3$-3-F |
| 3-OH-2-Br | 2-$CH_2SO_2C_2H_5$ | 5-$OCH_3$-3-F |
| 2-$CH_2OCH_2CH_2OH$ | 3-$CH_2SO_2C_2H_5$ | 6-$OCH_3$-3-F |
| 3-$CH_2O$—$CH_2CH_2OH$ | 4-$CH_2SO_2C_2H_5$ | 3-F-5-OH |
| 4-$CH_2OCH_2CH_2OH$ | 4-$SCH_2CH_2CH_3$ | 3-Cl-5-OH |
| 2-$(CH_2)_2OCH_2CH_2OH$ | 3-$SOCH_2CH_2CH_3$ | 3-Br-5-OH |
| 3-$(CH_2)_2OCH_2CH_2OH$ | 2-$SO_2CH_2CH_2CH_3$ | 3-J-5-OH |
| 4-$(CH_2)_2OCH_2CH_2OH$ | 2-$SCH(CH_3)_2$ | 2-$CH_2OH$-3-Br |
| 2-OH | 3-$SOCH(CH_3)_2$ | 4-$CH_2OH$-3-Br |
| 2-OH-3-$CH_3$ | 4-$SO_2CH(CH_3)_2$ | 5-$CH_2OH$-3-Br |
| 2-OH-4-$CH_3$ | 2-$CH_2CH_2SCH_3$ | 6-$CH_2OH$-3-Br |
| 2-OH-5-$CH_3$ | 3-$CH_2CH_2SOCH_3$ | 2-$CH_2OH$-3-Cl |
| 4-OH | 4-$CH_2CH_2SO_2CH_3$ | 4-$CH_2OH$-3-Cl |
| 4-OH-2-$CH_3$ | 2-$CH_2CH_2SOC_2H_5$ | 5-$CH_2OH$-3-Cl |
| 4-OH-3-$CH_3$ | 3-$CH_2CH_2SO_2C_2H_5$ | 6-$CH_2OH$-3-Cl |
| 3-$SCH_3$ | 4-$CH_2CH_2SC_2H_5$ | 2-$CH_2OH$-3-F |
| 4-$SCH_3$ | 2-$SCH_2CH_2OH$ | 4-$CH_2OH$-3-F |
| 2-$SOCH_3$ | 3-$SCH_2CH_2OH$ | 5-$CH_2OH$-3-F |
| 3-$SOCH_3$ | 4-$SCH_2CH_2OH$ | 6-$CH_2OH$-3-F |
| 4-$SOCH_3$ | 2-$SOCH_2CH_2OH$ | 5-$OC_2H_5$-3-Br |
| 2-$SO_2CH_3$ | 3-$SOCH_2CH_2OH$ | 5-$OC_2H_5$-3-Cl |
| 3-$SO_2CH_3$ | 4-$SOCH_2CH_2OH$ | 5-$OC_2H_5$-3-F |
| 4-$SO_2CH_3$ | 2-$SO_2CH_2CH_2OH$ | 2-$CH_2OCH_3$-3-Br |
| 2-$SC_2H_5$ | 3-$SO_2CH_2CH_2OH$ | 4-$CH_2OCH_3$-3-Br |
| 3-$SC_2H_5$ | 4-$SO_2CH_2CH_2OH$ | 5-$CH_2OCH_3$-3-Br |
| 4-$SC_2H_5$ | 2-$SCH_2$—$CH=CH_2$ | 6-$CH_2OCH_3$-3-Br |
| 2-$SOC_2H_5$ | 3-$SOCH_2CH=CH_2$ | 2-$CH_2OCH_3$-3-Cl |
| 3-$SOC_2H_5$ | 4-$SO_2CH_2CH=CH_2$ | 4-$CH_2OCH_3$-3-Cl |
| 4-$SOC_2H_5$ | 2-S—CH=CH—$CH_3$ | 5-$CH_2OCH_3$-3-Cl |
| 2-$SO_2C_2H_5$ | 3-S—CH=CH—$CH_3$ | 6-$CH_2OCH_3$-3-Cl |
| 3-$SO_2C_2H_5$ | 4-S—CH=CH—$CH_3$ | 2-$CH_2OCH_3$-3-F |
| 4-$SO_2C_2H_5$ | 4-$CH_2S$—$CH_2CH=CH_2$ | 4-$CH_2OCH_3$-3-F |
| 2-$CH_2SCH_3$ | 2-$CF_3$ | 5-$CH_2OCH_3$-3-F |
| 3-$CH_2SCH_3$ | 3-$CF_3$ | 6-$CH_2OCH_3$-3-F |

TABELLE 2

| A′ | A′ | A′ |
|---|---|---|
| 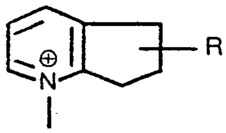<br>R=H<br>=7-OH<br>=7-$OCH_3$<br>=7-$CH_2OH$<br>=7,7-di$CH_2OH$<br>=7-Cl<br>=7-exo-methylen<br>=7-$CONH_2$<br>=3-OH<br>=3-$CH_2OH$<br>=4-$CH_3$<br>=4-$CH_2OH$<br>=5-OH, 7-$CH_3$<br>=6-OH, 7-$CH_3$<br>=5-$CH_3$<br>=6-$CH_3$<br>=7-$CH_3$<br>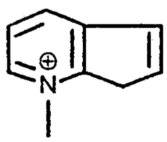<br>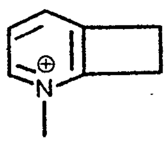<br>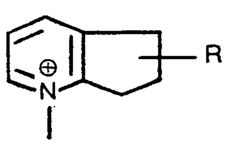<br>R=H<br>=8-$CH_3$<br>=8-OH<br>=8-OH, 3-$CH_3$<br>=8-$CH_2OH$<br>=8-$CH_2OH$, 3-$CH_3$<br>=8-$OCH_3$<br>=8,8-di-$CH_2OH$<br>=8-Cl<br>=8-Br<br>=8-exo-methylen<br>=8-$CONH_2$<br>=8-Oxo<br>=6-Cl<br>=5-OH<br>=5-oxo<br>=5-Cl<br>=3-$CH_2OH$<br>=3-OH<br>=4-$OCH_3$ | 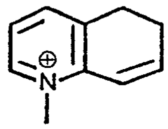<br>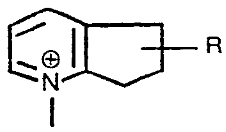<br>R=H<br>=4-OH<br>=4-$OCH_3$<br>=1-Cl<br>=5-Cl<br>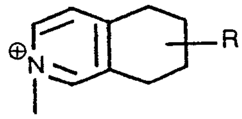<br>R=H<br>=4-OH<br>=4-$OCH_3$<br>=1-Cl<br>=5-Cl<br>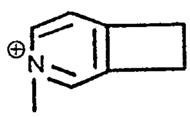<br>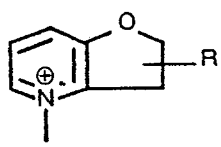 R=H<br>=2–$CH_3$<br>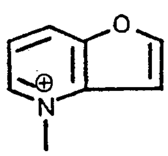<br>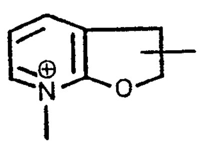 R=H<br>=3–oxo<br>=3–OH<br>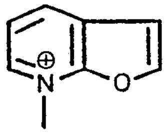<br>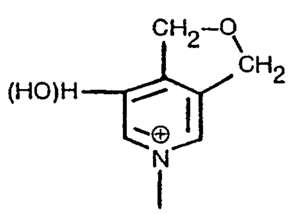 | 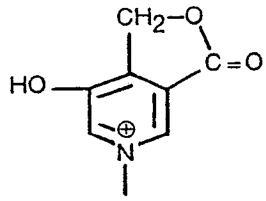<br>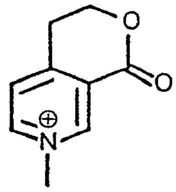<br>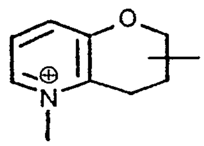 R=H<br>=2–$OCH_3$<br>=3–OH<br>=4–OH<br>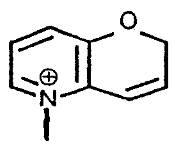<br>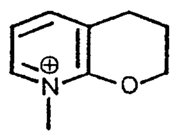<br>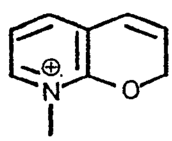<br>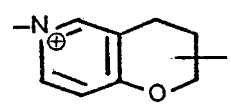 R=H<br>=–4–oxo<br>=–4–OH<br>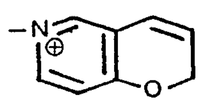<br>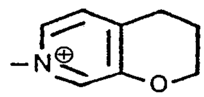<br>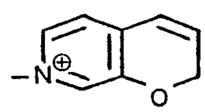 |

TABELLE 2 — cont.

| A' | A' | A' |
|---|---|---|
| 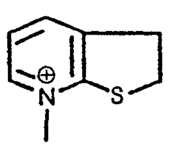 | 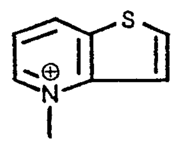 | 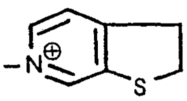 |
| 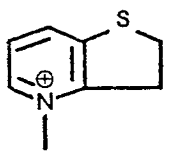 | 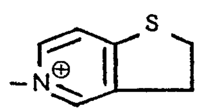 | |

Dieselben Verbindungen wie in Tab. 1 und 2 werden erhalten, wenn in der allg. Formel I anstelle von $R^2=CH_3$ der Rest $R^2=C_2H_5$ steht.

Die folgenden Ausführungsbeispiele für erfindungsgemäß herstellbare syn-Verbindungen dienen zur weiteren Erläuterung der Erfindung, schränken sie jedoch nicht darauf ein.

Beispiel 1

7-[2-(2-Aminothiazol-4-yl)-2-syn-methoxyimino-acetamido]-3-[(4-äthyl-1-pyridinium)-methyl]-ceph-3-em-carboxylat (Verfahren a)

*Variante a:*

Ein Gemisch aus 6,83 g (15 mmol) 7-[2-(2-Aminothiazol-4-yl)-2-syn-methoximino-acetamido]-cephalosporansäure, 1,38 g (16,5 mmol) Natriumhydrogencarbonat, 74,7 g (450 mmol) Kaliumjodid, 16,07 g (150 mmol) 4-Äthylpyridin und 70 ml Wasser wird 5 Stunden bei 50—55°C gerührt. Dabei hält man den pH-Wert durch gelegentliche Zugabe von Natriumhydrogencarbonat zwischen 6,8 und 7,2. Die Mischung wird mit 500 ml Aceton verdünnt und an Kieselgel (Merck 0,063—0,20 mm, 25 × 3 cm-Säule) chromatographiert. Mit Aceton/Wasser (7:1) wird Kaliumjodid, mit Aceton/Wasser (3:1) dann die Titelverbindung eluiert. Das gefriergetrocknete Rohprodukt wird an Kieselgel rechromatographiert (Merck "Lobar"-Säule C, ca. 1 bar, Aceton/Wasser 3:1). Durch Gefriertrocknen der Produktfraktionen erhält man 3,7 g der Titelverbindung in Form eines farblosen Feststoffs.

$^1$H—NMR ($CF_3CO_2D$): δ = 1,46 (t, J=7Hz, 3H, $CH_2$—$\underline{CH_3}$); 3,07 (q, J=7Hz, 2H, $\underline{CH_2}$—$CH_3$); 3,50 und 3,77 (AB, J=19Hz, 2H, S—$CH_2$); 4,23 (s, 3H, $OCH_3$); 5,19—6,22 (m, 4H, $CH_2$Py und 2 Lactam-H); 7,37 (s, 1H, Thiazol); 7,91 und 8,80 ppm (AA'BB', J=6Hz, 4H, Py)

IR (KBr): 1775 $cm^{-1}$ (Lactam-CO)

*Variante b:*

Die Titelverbindung wird in gleicher Reinheit wie vorstehend und in einer Ausbeute von 4,9 g erhalten, wenn 6,83 g (15 mMol) 7-[2-(2-Aminothiazol-4-yl)-2-syn-methoximino-acetamido]-cephalosporansäure, 1,38 g (16,5 mMol) Natriumhydrogencarbonat, 4,8 g (45 mMol) 4-Äthylpyridin, 31,5 g (210 mmol) Natriumjodid und 21 ml Wasser 110 Stunden bei 25°C gerührt werden.

*Variante c:*

Mit den Mengen der Komponenten wie vorstehend unter Variante b beschrieben und einer Reaktionszeit von 1 Stunde bei 80°C werden 4,5 g der Titelverbindung in Form eines farblosen Feststoffs erhalten.

Analog Beispiel 1 werden die nachfolgend aufgeführten Verbindungen erhalten, die der allgemeinen Formel I mit $R^1$ = Wasserstoff und $R^2$ = Methyl entsprechen und die im Pyridiniumrest (A in Formel I) die in der zweiten Spalte der Tabelle 3 angegebenen Substituenten tragen.

Die Zahl von den Substituenten gibt jeweils die genaue Stellung des oder der Substituenten im Pyridiniumrest an.

TABELLE 3

| Beispiel | Substituent | Ausbeute % d.Th. | Bemerkung | $^1H$—NMR: δ (ppm) |
|---|---|---|---|---|
| 2 | 3-$C_2H_5$ | 61 | Rechromatographie mit Aceton/Wasser 2:1 | ($F_3CCO_2D$): 1,57 (t, J=7Hz, 3H, $CH_2$—$CH_3$); 3,00 (q, J=7Hz, 2H, $CH_2$—$CH_3$); 3,49 und 3,77 (AB, J=20Hz, 2H, S—$CH_2$); 4,22 (2, 3H, $OCH_3$); 5,24—6,28 (m, 4H, $CH_2$-Py und 2 Lactam-H); 7,35 (s, 1H, Thiazol); 7,86—8,77 (m, 4H, Py) |
| 3 | 2-$C_2H_5$ | 28 | Rechromatographie mit Aceton/Wasser 2:1 | ($F_3CCO_2D$): 1,57 (t, J=7Hz, 3H, $CH_2$—$CH_3$); 3,12—3,99 (m, 4H, $CH_2$—$CH_3$ und S—$CH_2$); 4,23 (s, 3H, $OCH_3$); 5,35—6,12 (m, 4H, $CH_2$-Py und 2 Lactam-H); 7,36 (s, 1H, Thiazol), 7,80—8,76 (m, 4H, Py) |
| 4 | 4-$CH_2CH_2CH_3$ | 45 | Rechromatographie mit Aceton/Wasser 4:1 | ($F_3CCO_2D$): 1,09 (t, J=7Hz, 3H, $CH_2$—$CH_3$); 1,85 (sx, J=7Hz, 2H, $CH_2$—$CH_2$—$CH_3$); 2,87—4,06 (m, 4H, $CH_2$—$CH_2$ und S—$CH_2$); 4,22 (s, 3H, $OCH_3$); 5,17—6,21 (m, 4H, $CH_2$Py und 2 Lactam H); 7,35 (s, 1H, Thiazol); 7,86 und 8,78 (AA'BB', J=6Hz, 4H, Py) |
| 5 | 4-iso-$C_3H_7$ | 47 | Rechromatographie mit Aceton/Wasser 4:1 | ($F_3CCO_2D$): 1,46 (d, J=7Hz, 6H, iso-$C_3H_7$); 2,97—4,07 (m, 3H, iso-$C_3H_7$ und S—$CH_2$); 4,23 (s, 3H, $OCH_3$); 5,17—6,23 (m, 4H, $CH_2$-Py und 2 Lactam-H); 7,37 (s, 1H, Thiazol); 7,93 und 8,82 (AA'BB', J=6Hz, 4H, Py) |
| 6 | 2-$CH_3$ | 37 | Als Nebenprodukt werden bei der Rechromatographie mit Aceton/Wasser (2:1) geringe Mengen Δ2-Isomeres erhalten. | ($F_3CCO_2D$): 2,98 (s, 3H, $PyCH_3$); 3,52 und 3,71 (AB, J=19Hz, 2H, S-$CH_2$); 4,24 (s, 3H, $OCH_3$); 5,35—6,12 (m, 4H, $CH_2$Py und 2 Lactam-H); 7,37 (s, 1H, Thiazol); 7,79—8,76 (m, 4H, Py) |
| 7 | 4-t-$C_4H_9$ | 41 | Rechromatographie mit Aceton/Wasser 2:1 | ($F_3CCO_2D$): 1,51 (s, 9H, $C_4H_9$); 3,48 und 3,79 (AB, J=19Hz, 2H, S—$CH_2$); 4,23 (s, 3H, $OCH_3$), 5,16—6,21 (m, 4H, $CH_2$-Py und 2-Lactam-H); 7,36 (s, 1H, Thiazol); 8,07 und 8,82 (AA'BB', J=7Hz, 4H, Py) |
| 8 | 3-$CH_3$-4-$CH_3$ | 47 | Rechromatographie mit Aceton/Wasser 2:1 | ($F_3CCO_2D$): 2,55 (s, 3H, $CH_3Py$); 2,66 (s, 3H, $CH_3Py$); 3,47 und 3,74 (AB, J=19Hz, 2H, S—$CH_2$); 4,22 (s, 3H, $OCH_3$); 5,11—6,21 (m, 4H, $CH_2$-Py und 2 Lactam-H); 7,35 (s, 1H, Thiazol); 7,76—8,61 (m, 3H, Py) |

TABELLE 3 — cont.

| Beispiel | Substituenten | Ausbeute % d.Th. | Bemerkung | $^1$H—NMR: δ (ppm) |
|---|---|---|---|---|
| 9 | 3-$CH_3$-5-$CH_3$ | 51 | | ($F_3CCO_2D$): 2,62 (s, 6H, $CH_3$Py); 3,47 und 3,74 (AB, J=19Hz, 2H, S—$CH_2$); 4,22 (s, 3H, $OCH_3$); 5,14—6,27 (m, 4H, $CH_2$-Py und 2 Lactam-H); 7,36 (s, 1H, Thiazol); 8,18—8,57 (m, 3H, Py) |
| 10 | 3-$C_2H_5$-4-$CH_3$ | 38 | Rechromatographie mit Aceton/Wasser 4:1 | ($F_3CCO_2D$): 1,40 (t, J=7Hz, 3H, $CH_2$—$CH_3$); 2,69 (s, 3H, Py$CH_3$); 2,94 (q, J=7Hz, 2H, $CH_2$—$CH_3$); 3,47 und 3,73 (AB, J=19Hz, 2H, S—$CH_2$); 4,20 (s, 3H, $OCH_3$); 5,14—6,21 (m, 4H, $CH_2$-Py und 2 Lactam-H); 7,34 (s, 1H, Thiazol); 7,74—7,86 (m, 1H, Py); 8,49—8,70 (m, 2H, Py) |
| 11 | 3-$C_2H_5$-6-$CH_3$ | 22 | Rechromatographie mit Aceton/Wasser 4:1 | ($F_3CCO_2D$): 1,39 (t, J=7Hz, 3H, $CH_2$—$CH_3$); 2,73—3,11 (m, 5H, $CH_2$—$CH_3$ und Py$CH_3$); 3,44 und 3,61 (AB, J=19Hz, 2H, S—$CH_2$); 4,23 (s, 3H, $OCH_3$); 5,33—6,09 (m, 4H, $CH_2$-Py und 2 Lactam-H); 7,35 (s, 1H, Thiazol); 7,77—8,51 (m, 3H, Py) |
| 12 | 2-$CH_2C_6H_5$ | 11 | Rechromatographie mit Aceton/Wasser 4:1 | ($F_3CCO_2D$): 3,17 und 3,33 (AB, J=19Hz, 2H, S—$CH_2$); 4,23 (s, 3H, $OCH_3$); 4,61 (s, 2H, $CH_2C_6H_5$); 5,22—6,07 (m, 4H, $CH_2$-Py und 2 Lactam-H); 7,03—7,77 (m, 6H, Thiazol und $C_6H_5$); 7,77—8,83 (m, 4H, Py) |
| 13 | 4-$C_6H_5$ | 12 | Rechromatographie mit Aceton/Wasser 4:1 | ($F_3CCO_2D$): 3,55 und 3,82 (AB, J=19Hz, 2H, S—$CH_2$); 4,20 (s, 3H, $OCH_3$); 5,20—6,26 (m, 4H, $CH_2$-Py und 2 Lactam-H); 7,35 (s, 1H, Thiazol); 7,51—7,89 (m, 5H, $C_6H_5$); 8,26 und 8,91 (AA'BB', J=7Hz, 4H, Py) |
| 14 | 2-$CH_2OH$ | 38 | | ($CF_3CCO_2D$): 3,55 und 3,76 (AB, J=18Hz, 2H, S—$CH_2$); 4,23 (s, 3H, $OCH_3$); 5,32—6,20 (m, 6H, $CH_2$-Py, $CH_2OH$, 2 Lactam-H); 7,38 (s, 1H, Thiazol); 7,90—8,89 (m, 4H, Py) |
| 15 | 3-$CH_2OH$ | 58 | Reaktionszeit 8 Tage 25°C | ($CF_3COOD$): 3,54 und 3,79 (AB, J=18Hz, 2H, S—$CH_2$); 4,22 (s, 3H, $OCH_3$), 5,17 (s, 2H, $CH_2OH$), 5,14—6,32 (m, 4H, $CH_2$Py + 2 Lactam-H); 7,38 (s, 1H, Thiazol); 7,95—9,10 (m, 4H, Py) |

TABELLE 3 — cont.

| Beispiel | Substituent | Ausbeute % d.Th. | Bemerkung | $^1H$—NMR: δ (ppm) |
|---|---|---|---|---|
| 16 | 3-OH | 47 | Rechromatographie an XAD—2 mit Wasser | ($D_2O$): 3,21 und 3,45 (AB, J=17Hz, 2H, $CH_2S$); 3,93 (s, 3H, $OCH_3$); 5,35—5,85 (m, 4H, $CH_2$-Py und 2 Lactam-H); 6,93 (s, 1H, Thiazol); 7,43—7,95 (m, 4H, Py) |
| 17 | 2-$CHCH_3$<br>\|<br>OH | 35 | Reaktionszeit 110 Std. 23°C + 2 Std. 50°C | ($F_3CCO_2D$): 1,76 (d, J=7Hz, CH—$CH_3$); 3,20—4,36 (m, 3H, $SCH_2$ und CH—$CH_3$) 4,22 (s, 3H, $OCH_3$); 5,23—6,30 (m, 4H, $CH_2$-Py und 2 Lactam-H) 7,36 (s, 1H, Thiazol); 7,98—9,31 (m, 4H, Py) |
| 18 | 3-CH—$CH_3$<br>\|<br>OH | 67 | Reaktionszeit 110 Std. 23°C + 2 Std. 50°C Nach 1. Chromatographie rein erhalten. | ($F_3CCO_2D$): 1,88 (d, J=6Hz, CH—$CH_3$); 3,23—4,43 (m, 3H, $SCH_2$ und CH—$CH_3$); 4,22 (s, 3H, $OCH_3$); 5,38—6,39 (m, 3H, $CH_2$-Py und 2 Lactam-H); 7,37 (s, 1H, Thiazol); 7,90—8,88 (m, 4H, Py) |
| 19 | 4-$CH(CH_3)OH$ | 64 | Rechromatographie mit Aceton/Wasser 4:1 | ($F_3CCO_2D$): 1,75 (d, J=7Hz, CH—$CH_3$); 3,23—4,38 (m, 6H, S—$CH_2$, $OCH_3$ und CH—$CH_3$); 5,24—6,30 (m, 4H, $CH_2$-Py und 2 Lactam-H); 7,37 (s, 1H, Thiazol); 8,16 und 8,94 (AA'BB', J=6Hz, 4H, Py) |
| 20 | 3-$C(CH_3)_2OH$ | 58 | Rechromatographie mit Aceton/Wasser 4:1 | ($F_3CCO_2D$): 1,83 (s, 6H, $CH_3$); 3,54 und 3,78 (AB, J=20Hz, 2H, S—$CH_2$); 4,21 (s, 3H, $OCH_3$); 5,33—6,28 (m, 4H, $CH_2$-Py und 2 Lactam-H); 7,36 (s, 1H, Thiazol); 7,96—9,26 (m, 4H, Py) |
| 21 | 4-$C(CH_3)_2OH$ | 56 | Rechromatographie mit Aceton/Wasser 4:1 | ($F_3CCO_2D$): 1,83 (s, 6H, $CH_3$); 3,51 und 3,81 (AB, J=19Hz, 2H, S—$CH_2$); 4,23 (s, 3H, $OCH_3$); 5,23—6,24 (m, 4H, $CH_2$-Py und 2 Lactam-H); 7,36 (s, 1H, Thiazol); 8,21 und 8,92 (AA'BB', J=7Hz, 4H, Py) |
| 22 | 3-$CH_2CH_2CH_2OH$ | 38 | Rechromatographie mit Aceton/$H_2O$ 4:1, Reaktionszeit 3 Tage 23°C + 2 Std. 50°C | ($F_3CCO_2D$): 2,17 (m, 2H, propyl), 2,90—4,25 (m, 6H, S—$CH_2$ und 4 propyl-H); 4,22 (s, 3H, $OCH_3$); 5,20—6,88 (m, 4H, $CH_2$Py und 2 Lactam-H); 7,36 (s, 1H, Thiazol); 7,90—8,86 (m, 4H, Py) |

TABELLE 3 — cont.

| Beispiel | Substituent | Ausbeute % d.Th. | Bemerkung | $^1$H—NMR: δ (ppm) |
|---|---|---|---|---|
| 23 | 3-$COCH_3$ | 47 | Rechromatographie an XAD—2 (Säule 10 × 2 cm) mit Methanol/Wasser 4:1 | ($F_3CCO_2D$): 2,91 (s, 3H, Ac); 3,61 und 3,86 (AB, J=19Hz, 2H, S—$CH_2$); 4,24 (s, 3H, $OCH_3$); 5,34—6,39 (m, 4H, $CH_2$Py und 2 Lactam-H), 7,36 (s, 1H, Thiazol) 8,17—9,60 (m, 4H, Py) |
| 24 | 3-$COC_6H_5$ | 18 | | ($F_3CCO_2D$): 3,64 und 3,87 (AB, J=19Hz, 2H, S—$CH_2$); 4,23 (s, 3H, $OCH_3$); 5,35—6,42 (m, 4H, $CH_2$Py und 2 Lactam-H); 7,37—9,46 (m, 10H, Thiazol, $C_6H_5$ und Py) |
| 25 | 4-$COC_6H_5$ | 21 | Rechromatographie an XAD—2 mit Methanol/Wasser 4:1 | ($F_3CCO_2D$): 3,62 und 3,87 (AB, J=19Hz, 2H, S—$CH_2$); 4,23 (s, 3H, $OCH_3$); 5,35—6,41 (m, 4H, $CH_2$-Py und 2 Lactam-H); 7,37—9,26 (m, 10H, Thiazol, $C_6H_5$ und Py) |
| 26 | 4-$OCH_3$ | 45 | | ($CF_3COOD$): 3,48 und 3,72 (AB, J=18Hz, 2H, S—$CH_2$); 4,20 (s, 3H, $OCH_3$); 4,23 (s, 3H, $OCH_3$); 5,03—6,10 (m, 4H, $CH_2$-Py und 2 Lactam-H); 7,36—7,48 (3H, Thiazol-H, 2 Py-H); 8,62—8,75 (AA'BB', J=7Hz, 2H, Py) |
| 27 | 4-$CH_2OCH_3$ | 57 | Rechromatographie an XAD—2 mit Wasser | ($CF_3COOD$): 3,25—4,05 (AB, J=18Hz, 2H, $SCH_2$); 3,71 (s, 3H, $CH_2O\underline{CH_3}$); 4,22 (s, 3H, $OCH_3$); 4,95 (s, 2H, $\underline{CH_2}OCH_3$); 5,23—6,28 (m, 4H, $CH_2$-Py und 2 Lactam-H); 7,35 (s, 1H, Thiazol); 8,10 und 8,95 (AA'BB', J=6Hz, 4H, Py) |
| 28 | 2-$SCH_3$ | 14 | | ($CF_3COOD$): 2,91 (s, 3H, $SCH_3$); 3,52 und 3,74 (AB, J=18Hz, 2H, S—$CH_2$); 4,24 (s, 3H, $OCH_3$); 5,25—6,55 (m, 4H, $CH_2$Py + 2 Lactam-H); 7,36 (s, 1H, Thiazol); 7,55—8,80 (m, 4H, Py) |
| 29 | 4-$CH_2S$—$CH_3$ | 25 | | ($CF_3COOD$): 2,14 (s, 3H, $CH_3$); 3,47 und 3,80 (AB, J=18Hz, 2H, S—$CH_2$); 3,94 (s, 2H, $CH_2S$); 4,20 (s, 3H, $OCH_3$); 5,18—6,20 (m, 4H, $CH_2$-Py und 2 Lactam-H); 7,35 (s, 1H, Thiazol); 8,15 und 8,85 (AA'BB', J=6Hz, 4H, Py) |

TABELLE 3 — cont.

| Beispiel | Substituent | Ausbeute % d.Th. | Bemerkung | $^1$H—NMR: δ (ppm) |
|---|---|---|---|---|
| 30 | 3-$CH_2SOCH_3$ | 12 | Rechromatographie mit Aceton/Wasser 4:1 | ($CF_3COOD$): 3,01 (s, 3H, $SOCH_3$); 3,60 und 3,81 (AB, 2H, $SCH_2$); 4,24 (s, 3H, $OCH_3$); 4,57 (s, 2H, $CH_2SO$); 5,25—6,58 (m, 4H, $CH_2$-Py und 2 Lactam-H); 7,34 (s, 1H, Thiazol); 8,05—9,10 (m, 4H, Py) |
| 31 | 3-$CH_2SO_2CH_3$ | 23 | | ($CF_3COOD$): 3,34 (s, 3H, $SO_2CH_3$); 3,62 und 3,82 (AB, 2H, S—$CH_2$); 4,23 (s, 3H, $OCH_3$); 4,88 (s, 2H, $CH_2SO_2$); 5,30—6,35 (m, 4H, $CH_2$-Py und 2 Lactam-H); 7,35 (s, 1H, Thiazol); 8,05—9,18 (m, 4H, Py) |
| 32 | 3-F | 45 | Rechhromatographie mit Methanol/Wasser 4:1 | ($D_6$-DMSO): 3,76 (s, 3H, $OCH_3$); 4,52—5,96 (m, 4H, $CH_2$-Py und 2 Lactam-H); 6,63 (s, 1H, Thiazol); 7,10 (bs, 2H, $NH_2$); 7,80—9,86 (m, 5H, Py und NH) |
| 33 | 4-$NHCOCH_3$ | 22 | | ($CF_3COOD$): 2,46 (s, 3H, $COCH_3$); 3,47 und 3,80 (AB, J=18Hz, 2H, S—$CH_2$); 4,21 (s, 3H, $OCH_3$); 5,12—6,35 (m, 4H, $CH_2$-Py und 2 Lactam-H); 7,35 (s, 1H, Thiazol); 8,26 und 8,73 (AA'BB', J=6Hz, 4H, Py) |
| 34 | 4— 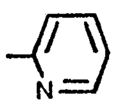 | 28 | Variante b, Zusatz von 20% Aceton zum Reaktionsmedium | ($CF_3COOD$): 3,60 und 3,87 (AB, J=18Hz, 2H, S—$CH_2$); 4,20 (s, 3H, $OCH_3$); 5,28—6,38 (AB, 3H, $CH_2$-Py); 5,37 (d, J=5Hz, $C_6$-Lactam-H); 6,08 (d, J=5Hz, $C_7$-Lactam-H); 7,38 (s, 1H, Thiazol); 8,15—9,41 (m, 8H, Py) |
| 35 | 4— 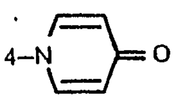 | 57 | Rechromatographie mit Aceton/Wasser 2:1 | ($D_2O$): 3,33 und 3,63 (AB, J=18Hz, 2H, S—$CH_2$); 3,86 (s, 3H, $OCH_3$); 5,05—5,76 (m, 4H, $CH_2$-Py und 2 Lactam-H); 6,76 (s, 1H, Thiazol); 6,53—6,80; 8,08—8,38; 9,00—9,15 (8H, Py) |
| 36 | 3-$CO_2C_2H_5$ | 27 | Rechromatographie an XAD—2 mit Wasser | ($F_3CCO_2D$): 1,54 (t, J=7Hz, 3H, $CH_2$—$CH_3$);, 3,33—4,14 (AB, 2H, S—$CH_2$); 4,23 (s, 3H, $OCH_3$); 4,66 (q, J=7Hz, 2H, $CH_2CH_3$); 5,35—6,36 (m, 4H, $CH_2$-Py und 2 Lactam-H); 7,37 (s, 1H, Thiazol); 8,13—9,67 (m, 4H, Py) |

TABELLE 3 — cont.

| Beispiel | Substituent | Ausbeute % d.Th. | Bemerkung | $^1$H—NMR: δ (ppm) |
|---|---|---|---|---|
| 37 | 4-$CO_2C_2H_5$ | 48 | | ($D_6$-DMSO): 1,35 (t, J=7Hz, 3H, $CH_2CH_3$); 3,77 (s, 3H, $OCH_3$); 4,22—5,62 (m, 6H, $CH_2CH_3$, $CH_2$Py und 2 Lactam-H); 6,62 (s, 1H, Thiazol); 7,08 (bs, 2H, $H_2$N-Thiazol); 8,44 (AA'BB', J=6Hz, 2H, Py); 9,33—9,63 (m, 5H, Py und Amid) |
| 38 | 3-$CH_2$—$CO_2H$ | 19 | Rechromatographie mit Aceton/Wasser 4:1 | ($F_3CCO_2D$): 3,55 und 3,81 (AB, J=19Hz, 2H, S—$CH_2$); 4,17 (s, 2H, $CH_2CO_2H$); 4,22 (s, 3H, $OCH_3$); 5,28—6,36 (m, 4H, $CH_2$-Py und 2-Lactam-H); 7,36 (s, 1H, Thiazol); 7,97—9,04 (m, 4H, Py) |
| 39 | 3-$CH_2CO_2C_2H_5$ | 47 | Rechromatographie mit Aceton/Wasser 4:1 | ($F_3CCO_2D$): 1,39 (t, J=7Hz, $CH_2CH_3$); 3,29—4,55 (m, 9H, S—$CH_2$, Py-$CH_2$, $CH_2CH_3$ und $OCH_3$); 5,29—6,33 (m, 4H, $CH_2$-Py und 2 Lactam-H); 7,36 (s, 1H, Thiazol); 7,95—9,02 (m, 4H, Py) |
| 40 | 4-$CONHCONHC_2H_5$ | 28 | Rechromatographie mit Aceton/Wasser 4:1 | ($CF_3COOD$): 1,43 (t, 3H, $CH_2CH_3$); 3,25—4,23 (m, 4H, $SCH_2$ und $CH_2CH_3$); 4,22 (s, 3H, $OCH_3$); 5,12—6,35 (m, 4H, $CH_2$Py + 2 Lactam-H); 7,37 (s, 1H, Thiazol); 8,62 und 9,24 (AA'BB', J=6Hz, 4H, Py) |
| 41 | 4$CH_2CONH_2$ | 43 | | ($D_2O$): 3,23 und 3,53 (AB, J=18Hz, 2H, S—$CH_2$); 3,93 (s, 3H, $OCH_3$); 5,22 (d, 1H, J=5Hz, $C_6$-Lactam-H); 5,31 und 5,44 (AB, 2H, $CH_2$-Py); 5,81 (d, 1H, J=5Hz, $C_7$-Lactam-H); 6,92 (s, 1H, Thiazol); 7,93 und 8,80 (AA'BB', J=6Hz, 4H, Py) |
| 42 | 3-$CONH_2$ | 47 | | ($CF_3COOD$): 3,60 und 3,83 (AB, J=18Hz, 2H, $SCH_2$); 4,22 (s, 3H, $OCH_3$); 5,33—6,38 (m, 4H, $CH_2$-Py und 2 Lactam-H); 7,36 (s, 1H, Thiazol); 8,12—9,60 (m, 4H, Py) |
| 43 | 3-$CONHCO_3C_2H_5$ | 17 | | ($CF_3COOD$): 1,40 (t, J=7Hz, 3H, $CH_2CH_3$); 3,25—4,26 (m, 4H, S—$CH_2$, $CH_2$—$CH_3$); 4,25 (s, 3H, $OCH_3$); 5,28—6,40 (4H, $CH_2$-Py und 2 Lactam-H); 7,38 (s, 1H, Thiazol); 8,08—9,62 (m, 4H, Py) |

TABELLE 3 — cont.

| Beispiel | Substituent | Ausbeute % d.Th. | Bemerkung | $^1$H—NMR: δ (ppm) |
|---|---|---|---|---|
| 44 | 4-$CONHCO_2C_2H_5$ | 19 | | ($CF_3COOD$): 1,45 (t, J=7Hz, 3H, $CH_2CH_3$); 3,25—4,61 (m, 4H, $SCH_2$ und $CH_2CH_3$); 4,23 (s, 3H, $OCH_3$); 5,25—6,40 (m, 4H, 2 Lactam-H und $CH_2$-Py) 7,36 (s, 1H, Thiazol); 8,42—9,30 (m, 4H, Py) |
| 45 | 4-$CONHC_2H_5$ | 43 | | ($CF_3COOD$): 1,38 (t, J=7Hz, 3H, $CH_2CH_3$); 3,15—4,25 (m, 4H, S—$CH_2$, $CH_2$—$CH_3$); 4,23 (s, 3H, $OCH_3$); 5,28—6,33 (4H, $CH_2$-Py und 2-Lactam-H); 7,36 (s, 1H, Thiazol); 8,41—9,20 (AA'BB', J=7Hz, 4H, Py) |
| 46 | 3-$CON(C_2H_5)_2$ | 36 | | ($CF_3COOD$): 1,22—1,53 (m, 6H, $C_2H_5$); 3,15—4,25 (m, 6H, S—$CH_2$ und $C_2H_5$); 4,25 (s, 3H, $OCH_3$); 5,35—6,40 (m, 4H, $CH_2$-Py und 2-Lactam-H); 7,41 (s, 1H, Thiazol); 8,10—9,22 (m, 4H, Py) |
| 47 | 4-CN | 31 | | ($D_2O$): 3,22 und 3,42 (AB, J=17Hz, 2H, $SCH_2$); 3,93 (S, 3H, $OCH_3$); 5,15—5,92 (m, 4H, $CH_2$-Py und 2 Lactam-H); 6,93 (s, 1H, Thiazol); 8,25—9,28 (AA'BB', 4H, Py) |
| 48 | 4-$SO_2NH_2$ | 26 | Rechromatographie an XAD—2 mit Wasser | ($CF_3COOD$): 3,55 und 3,82 (AB, J=19Hz, 2H, $SCH_2$); 4,23 (s, 3H, $OCH_3$); 5,30—6,33 (m, 4H, $CH_2$-Py und 2 Lactam-H); 7,40 (s, 1H, Thiazol); 8,65 und 9,15 (AA'BB', J=7Hz, 4H, Py) |
| 49 | 4-$CH_2CH_2SO_3K$ | 42 | Rechromatographie an Adsorptionsharz HP 20, Mitsubishi Co. | ($D_6$-DMSO): 2,30—4,05 (m, 6H, S—$CH_2$ und $CH_2$—$CH_2$); 3,75 (s, 3H, $OCH_3$);, 4,75—5,72 (m, 4H, $CH_2$-Py und 2 Lactam-H); 6,63 (s, 1H, Thiazol); 7,12 (bs, 2H, $NH_2$); 7,96 und 9,25 (AA'BB', J=7Hz, 4H, Py); 9,45 (d, J=8Hz, NH) |

Analog Beispiel 1, Variante b, werden die nachfolgend aufgeführten Verbindungen erhalten, die der allgemeinen Formel I mit $R^1$ = Wasserstoff und $R^2$ = Methyl entsprechen und als Rest A den in der zweiten Spalte der Tabelle 4 angegebenen Substituenten tragen.

TABELLE 4

| Beispiel | A | Ausbeute % d.Th. | Bemerkung | $^1H$—NMR: δ (ppm) |
|---|---|---|---|---|
| 50 | 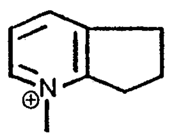 | 48 | Rechromatographie mit Aceton/Wasser 4:1 Reaktionszeit 3 Tage 23°C und 2 Std. 50°C mit Zusastz von 30% Aceton zum Reaktionsmedium | ($CF_3COOD$): 2,40—2,70 (m, 2H, Cyclopenten-H); 3,22—4,23 (m, 6H, 4-Cyclopenten-H und S—$CH_2$); 4,23 (s, 3H, $OCH_3$); 5,28—6,36 (m, 4H, $CH_2$-Py und 2 Lactam-H); 7,37 (s, 1H, Thiazol); 7,66—8,58 (m, 4H, Py) |
| 51 | 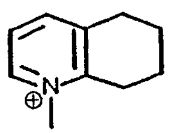 | 38 | Rechromatographie mit Aceton/Wasser 4:1 Reaktionszeit 3 Tage 23°C und 2 Std. 50°C mit Zusatz von 30% Aceton zum Reaktionsmedium | ($CF_3COOD$): 2,10 (m, 4H, Cyclohexen-H); 3,18 (m, 4H, Cyclohexen-H); 3,50 und 3,70 (AB, J=19Hz, 2H, S—$CH_2$); 4,25 (s, 3H, $OCH_3$); 5,38 (d, J=5Hz, $C_6$-Lactam-H); 5,55 und 5,80 (AB, 2H, $CH_2$-Py); 6,08 (d, J=5Hz, $C_7$-Lactam-H); 7,39 (s, 1H, Thiazol); 7,65—8,58 (m, 3H, Py) |

Beispiel 52

7-[2-(2-Aminothiazol-4-yl)-2-syn-äthoxyimino-acetamido]-3-(1-pyridiniummethyl)-ceph-3-em-4-carboxylat

Ein Gemisch aus 7,25 g (15 mmol) 7-[2-(2-Aminothiazol-4-yl)-2-syn-äthoxyimino-acetamido]-cephalosporansäure, 1,38 g (16,5 mmol) Natriumhydrogencarbonat, 41,5 g (250 mmol) Kaliumjodid, 5,92 g (75 mmol) Pyridin und 20 ml Wasser wird 5 Stunden bei 60°C gerührt. Der nach dem Gefriertrocknen erhaltene Rückstand wird in wenig Aceton/Wasser aufgenommen und an Kieselgel (Merck 0,063—0,20 mm, 40 × 4 cm-Säule) chromatographiert. Mit Aceton/Wasser (20:1) wird Kaliumjodid, mit Aceton/Wasser (4:1) dann die Titelverbindung eluiert. Das Rohprodukt wird an Kieselgel chromatographiert (Merck Lobar-Säule, ca. 1 bar, Aceton/Wasser 4:1). Durch Gefriertrocknen der Produktfraktionen erhält man 3,4 g der Titelverbindung in Form eines farblosen Feststoffs.

$^1$H—NMR ($D_6$-DMSO): δ = 1,21 (t, J=7Hz, 3H, $CH_2$—$\underline{CH_3}$); 4,08 (q, J=7Hz, 2H, $\underline{CH_2}$—$CH_3$); 4,85—5,73 (m, 4H, $CH_2$Py und 2 Lactam-H); 6,65 (s, 1H, Thiazol), 7,12 (bs, 2H, $H_2$N-Thiazol); 8,08—9,47 ppm (m, 6H, Py und Amid)

IR (KBr): 1775cm$^{-1}$ (Lactam-CO).

Die folgenden Verbindungen werden in Analogie zu Beispiel 52 hergestellt:

Beispiel 53

7-[2-(2-Aminothiazol-4-yl)-2-syn-äthoxyimino-acetamido]3-[(3-methyl-1-pyridinium)methyl]-ceph-3-em-4-carboxylat

Farbloser Feststoff, Ausbeute 47% d.Th.

$^1$H—NMR ($D_6$-DMSO): δ = 1,19 (t, J=7Hz, 3H, $CH_2$—$\underline{CH_3}$); 2,48 (m, DMSO und Py-$CH_3$); 3,29 (bs, $H_2O$ + S—$CH_2$); 4,08 (q, J=7Hz, 2H, $CH_2$—$CH_3$); 4,85—5,71 (m, 4H, $CH_2$Py und 2 Lactam-H); 6,63 (s, 1H, Thiazol); 7,08 (bs, 2H, $H_2$N-Thiazol); 7,85—9,46 ppm (m, 5H, Py und Amid).

Beispiel 54

7-[2-(2-Aminothiazol-4-yl)-2-syn-äthoxyimino-acetamido]-3-[(4-hydroxymethyl-1-pyridinium)methyl]-ceph-3-em-4-carboxylat

Farbloser Feststoff, Ausbeute 52% d.Th.

$^1$H—NMR ($D_6$-DMSO): δ = 1,21 (t, J=7Hz, 3H, $CH_2$—$\underline{CH_3}$); 3,31 (bs, $H_2O$ + S—$CH_2$); 4,10—5,72 (m, 9H, $CH_2CH_3$, $CH_2OH$, $CH_2$Py und 2 Lactam-H); 6,63 (s, 1H, Thiazol); 6,90—7,30 (m, 3H, $H_2$N und 1 Py-H); 7,92—9,45 (m, 4H, 3Py-H und Amid).

Beispiel 55

7-[2-(2-Amino-5-chlorthiazol-4-yl)-2-syn-methoxyimino-acetamido]-3-[(2-methyl-1-pyridinium)methyl]-ceph-3-em-4-carboxylat

Ein Gemisch aus 0,98 g (2 mmol) 7-[2-(2-Amino-5-chlorthiazol-4-yl)-2-syn-methoxyimino-acetamido]-cephalosporansäure, 0,18 g (2,2 mmol) Natrium-hydrogencarbonat, 6,6 g (40 mmol) Kaliumjodid, 1,8 g (20 mmol) 2-Picolin und 8 ml Wasser wird 5 Stunden bei 60°C gerührt. Dabei hält man den pH-Wert durch gelegentliche Zugabe von Natriumhydrogencarbonat zwischen 6,5 und 7. Es wird mit 70 ml Aceton verdünnt und an Kieselgel (Merck 0,063—0,20 mm, Säule 10 × 2 cm) chromatographiert. Mit Aceton/Wasser (7:1) wird Kaliumjodid, mit Aceton/Wasser (3:1) dann die Titelverbindung eluiert. Das Rohprodukt (0,2 g) wird an Kieselgel rechromatographiert (Merck "Lobar"-Säule B, ca. 1 bar, Aceton/Wasser 3:1). Durch Gefriertrocknen der Produktfraktionen erhält man 0,13 g der gewünschten Verbindung in Form eines farblosen Feststoffes neben geringen Mengen des Δ 2-Isomeren.

$^1$H—NMR ($CF_3CO_2D$): δ = 2,98 (s, 3H, Py—$CH_3$); 3,51 und 3,74 (AB, J=19Hz, 2H, S—$CH_2$); 4,20 (s, 3H, $OCH_3$); 5,34—6,11 (m, 4H, $CH_2$-Py und 2 Lactam-H); 7,77—8,73 ppm (m, 4H, Py).

IR (KBr): 1770 cm$^{-1}$ (Lactam-CO).

Die folgende Verbindung wird in Analogie zu Beispiel 55 hergestellt.

Beispiel 56

7-[2-(2-Amino-5-chlorthiazol-4-yl)-2-syn-methoxyiminoacetamido]-3-[(4-methyl-1-pyridinium)-methyl]-ceph-3-em-4-carboxylat

$^1$H—NMR ($CF_3CO_2D$): δ = 2,75 (s, 3H, Py-$CH_3$); 3,49 und 3,77 (AB, J=19Hz, 2H, S—$CH_2$); 4,19 (s, 3H, $OCH_3$); 5,16—6,20 (m, 4H, $CH_2$—Py und 2 Lactam-H); 7,86 und 8,76 ppm (AA'BB', J=6Hz, 4H, Py).

Bemerkung: Bei der Rechromatographie werden geringe Mengen Δ 2-Isomeres erhalten.

Beispiel 57

7-[2-(2-Aminothiazol-4-yl)-2-syn-methoxyimino-acetamido]-3-[(4-äthyl-1-pyridinium)-methyl]-ceph-3-em-4-carboxylat (Verfahren b)

*a) 7-tert-Butoxycarbonylaminocephalosporansäure*

Eine Mischung aus 12,8 g (50 mmol) 7-Aminocephalosporansäure, 20,3 g (0,1 mol) Bistrimethylsilylacetamid, 22 g (0,1 mol) Di-tert-butyldicarbonat und 150 ml Methylendichlorid wird 10 Tage bei Raumtemperatur belassen. Die Lösung wird sodann nach Zugabe von 200 ml Eiswasser 3 Stunden gerührt. Von nicht umgesetzter 7-Aminocephalosporansäure wird abgesaugt, die organische Phase abgetrennt und die Wasserphase noch zweimal mit Methylenchlorid extrahiert. Die organische Phase wird mit Wasser

mehrmals gewaschen und mit Magnesiumsulfat getrocknet. Nach Abdempfen des Lösungsmittels verbleiben 11 g (59% d.Th.) amorphes gelbliches Produkt.

NMR ($D_6$-DMSO): δ = 1,41 (s, 9H, tert.Butyl); 2,00 (s, 3H, Acetyl); 2,80—3,61 3,6 (2H, $SCH_2$ + $H_2O$); 4,50—5,07 (m, 3H, $C_6$-Lactam-H) und $CH_2OAc$); 5,41 (dd, J=4,5 und 9Hz, $C_7$-Lactam-H); 7,90 ppm (d, J=9Hz; NH).

IR (KBr): 1785 $cm^{-1}$ (Lactam-CO), 1725 $cm^{-1}$ (Ester).

*b) 7-tert-Butoxycarbonylamino-3-[(4-äthyl-1-pyridinium)methyl]-ceph-3-em-4-carboxylat.*

Ein Gemisch aus 7,44 g (20 mmol) 7-tert-Butoxy-carbonylaminocephalosporansäure, 66 g (0,4 mol) Kaliumjodid, 15 g (0,14 mol) 4-Äthylpyridin und 30 ml Wasser werden 6 Std. bei 75°C gerührt. Das Gemisch wird in Analogie zu Beispiel 1 über Kieselgel chromatographiert. Mit Aceton/Wasser (3:1) wird die Titelverbindung eluiert. Ausbeute 2,4 g (30% d.Th.) amorphes Produkt nach der Gefriertrocknung.

NMR ($D_6$-DMSO): δ = 1,05—1,48 (s, t, 9H, tert. Butyl und 3H, $CH_2$—$CH_3$); 2,70—3,72 (2H, $SCH_2$ und $H_2O$); 4,02—5,62 (m, 6H, $CH_2CH_3$, 2 Lactam-H und $CH_2N$); 7,63—9,44 ppm (m, 5H, NH und 4 Py-H).

IR (KBr): 1780 (Lactam-CO), 1710 $cm^{-1}$ (Ester).

*c) 7-Amino-3-[(4-äthyl-1-pyridinium)-methyl]-ceph-3-em-4-carboxylat*

Eine Lösung von 2,10 g (5 mmol) 7-tert-Butoxycarbonylamino-3-[(4-äthyl-1-pyridinium)-methyl]-ceph-3-em-4-carboxylat in 20 ml Trifluoressigsäure und 0,2 ml Anisol wird 30 min bei 10—20°C belassen. Nach Abdestillieren der Trifluoressigsäure wird der Rückstand durch mehrmaliges Verreiben mit Äther zur Kristallisation gebracht. Es wird in 5 ml Wasser mit Hilfe von Natriumbicarbonat gelöst und die Lösung über Servachrom XAD-2-Adsorberharz (2 × 40 cm-Säule) mit Wasser chromatographiert. Nach einem Vorlauf von 400 ml wird die Titelverbindung eluiert. Die Produktfraktionen werden gefriergetrocknet und man erhält 1,65 g (78% d.Th.) eines farblosen Feststoffes.

NMR ($CF_3COOD$): δ = 1,47 (t, J=7Hz, 3H, $CH_2CH_3$); 3,03 (q, J=7Hz, 2H, $CH_2CH_3$); 3,60 und 3,78 (AB, J=19Hz, 2H, $SCH_2$), 5,25—5,72 (m, 4H, 2 Lactam-H und 5,25 $CH_2N$), 7,75 und 8,63 ppm (AA'BB', J=6Hz, 4Py-H).

IR (KBr): 1785 $cm^{-1}$ (Lactam-CO).

*d) 7-[2-(2-Aminothiazol-4-yl)-2-syn-methoxyiminoacetamido]-3-[(4-äthyl-1-pyridinium)-methyl]-ceph-3-em-4-carboxylat*

1 g (5 mmol) 2-(2-Amino-1,3-thiazol-4-yl)-2-syn-methoxyimino-essigsäure werden in 20 ml Methylendichlorid suspendiert. Nach Zugabe von 0,93 ml (10 mMol) N,N-Dimethylacetamid wird auf −5° gekühlt und unter Rühren eine Lösung von 0,75 g (7,5 mMol) Phosgen in 5 ml Toluol zugetropft. Es wird 2 Stunden bei −5°C nachgerührt und nach Abkühlen auf −10°C ein Gemisch aus 1,60 g (3,8 mMol) 7-Amino-3-[(4-äthyl-1-pyridinium)-methyl]-ceph-3-em-4-carboxylat, 1,1 ml (8 mMol) Triäthylamin, 2 g (10 mMol) Bistrimethylsilylacetamid und 20 ml N,N-Dimethylacetamid, das auf −10°C gekühlt war, zugegeben. Es wird 2 Std. bei −10°C nachgerührt, 30 ml Eiswasser zugegeben und weitere 30 Minuten bei 0—5°C sowie 1 Std. ohne Kühlung nachgerührt. Das Lösungsmittel wird im Vakuum entfernt und der Rückstand in Analogie zu Beispiel 1 über Kieselgel mit Aceton/Wasser (3:1) chromatographiert. Durch Gefriertrocknen wird die Titelverbindung in 60% Ausbeute erhalten. Sie ist in allen Eigenschaften mit der Verbindung aus Beispiel 1 identisch.

Beispiele 58—107

In Analogie zu Beispiel 57 d werden die Verbindungen der Tabellen 3 und 4 aus den entsprechenden 7-Amino-ceph-3-em-4-carboxylat-Derivaten (allgemeine Formel III, hergestellt analog Beispiel 57 b, c) und 2-(2-Amino-1,3-thiazol-4-yl)-2-syn-methoxyimino-essigsäure dargestellt. Die Verbindungen sind in allen Eigenschaften mit denen aus den Tabellen 3 und 4 identisch.

Beispiel 108

3-[(4-Cyclopropyl-1-pyridinium)methyl]-7-[2-syn-methoximino-2-(2-aminothiazol-4-yl)acetamido]-ceph-3-em-4-carboxylat (Verfahren a)

Ein Gemisch aus 4,55 g (10 mmol) 7-[2-Aminothiazol-4-yl)-2-syn-methoximino-acetamido]-cephalosporansäure, 1 g (10 mmol) Kaliumhydrogencarbonat und 2,38 g (20 mmol) 4-Cyclopropylpyridin in 25 ml Wasser wird durch Zugabe von 1,5 ml 2 n Essigsäure auf pH 6,5 gestellt und sodann 3 Stunden unter Rühren auf 60—63°C erhitzt.

Nach dem Erkalten wird der Ansatz mit 50 ml Aceton verdünnt und über 400 g Kieselgel (Merck 0,063—0.2 mm) mit Aceton/Wasser (2:1) chromatographiert. Nach einem Vorlauf von 800 ml wird die Titelverbindung eluiert. Das gefriergetrocknete Rohprodukt wird über eine "Lobar C"-Säule (Merck) mit Aceton/Wasser (2:1) rechromatographiert. Durch Gefriertrocknen der Produktfraktionen erhält man 1 g (19,5% d.Th.) eines farblosen amorphen Feststoffs.

$^1H$—NMR ($CF_3CO_2D$): δ = 1,17—2,51 (m, 5H, Cyclopropyl); 3,48 und 3,76 (AB, J=19Hz, 2H, $SCH_2$); 4,24 (s, 3H, $OCH_3$); 5,12—6,19 (m, 4H, $CH_2Py$ und 2 Lactam-H); 7,41 (s, 1H, Thiazol); 7,65 und 8,68 ppm (AA'BB', J=7Hz, 4H, Py).

(Verfahren b)

Ein Gemisch aus 5 g (20 mmol) 2-(2-Amino-1,3-thiazol-4-yl)-2-syn-methoximino-essigsäure, 3,8 g (25

mmol) 1-Hydroxy-1H-benzotriazol-Hydrat, 5,7 g (27,5 mmol) Dicyclohexylcarbodiimid und 125 ml N,N-Dimethylformamid wird 3 Stunden bei Raumtemperatur gerührt. Die Mischung wird filtriert, auf 0°C gekühlt und in eine Lösung von 10,1 g (25 mmol) 7-Amino-3-[(4-cyclopropyl-1-pyridinium)methyl]-ceph-3-em-4-carboxylat-Dihydrochlorid, 50 ml N,N-Dimethylformamid und 8 ml (63,5 mmol) N,N-Dimethyl-anilin gegeben. Es wird über Nacht bei Raumtemperature belassen, von Niederschlag abgesaugt und das Filtrat unter Rühren in 1,5l Diäthyläther eingetropft. Der amorphe Niederschlag wird mit Aceton verrührt, abgesaugt und mit Aceton gewaschen. Dieses Rohprodukt wird in 200 ml Wasser gelöst, von wenig Ungelöstem wird filtriert und die wässrige Lösung gefriergetrocknet.

Man erhält 8,8 g (68% d.Th.) eines farblosen Feststoffs. Die Verbindung ist in allen Eigenschaften mit der oben nach Verfahren a) erhaltenen identisch.

Beispiel 109

3-[(3,4-Dihydro-2H-pyrano[3,2-c]pyridinium)methyl]-7-[2-syn-methoximino-2-(2-aminothiazol-4-yl)acetamido]-ceph-3-em-4-carboxylat

Ein Gemisch aus 6,83 g (15 mmol) 7-[2-Aminothiazol-4-yl)-2-syn-methoximino-acetamido]-cephalosporansäure, 1,5 g (15 mmol) Kaliumhydrogencarbonat, 24 g (0,25 mol) Kaliumrhodanid, 25 ml Wasser und 3,4 g (25 mmol) 3,4-Dihydro-2H-pyrano[3,2-c]pyridin (H. Sliwa und G. Cordonnier, J.Het. Chemistry *12*, 809 (1975)), dessen pH-Wert durch Zugabe von 2,4 g 85%iger Phosphorsäure auf pH. 6,8 eingestellt wurde, wird 2 Stunden bei 68—70°C gerührt. Nach dem Erkalten werden 200 ml Aceton zugegeben und die Mischung über 400 g Kieselgel (Merck 0,063—0,2 mm) chromatographiert. Mit Aceton/Wasser (8:1) werden die Salze, mit Aceton/Wasser (2:1) das Produkt eluiert. Die Produktfraktionen werden lyophilisiert und das Lyophilisat über eine "Lobar-C"-Säule (Merck AG) mit Aceton/Wasser (2:1) rechromatographiert. Durch Gefriertrocknen der Produktfraktionen erhält man 0,9 g der Titelverbindung in Form eines farblosen Feststoffs.

$^1H$—NMR ($CF_3CO_2D$): δ = 2,00—2,45 (m, 2H, Pyran-H); 2.75—3.30 (m, 2H, Pyran-H), 3,52 und 3,75 (AB, J=19Hz, 2H, $SCH_2$); 4.25 (s, 3H, $OCH_3$); 4.40—4.85 (m, 2H, Pyran-H); 5,15—6.20 (m, 4H, $CH_2Py$ und 2-Lactam-H); 7,35—7,45 (m, 1Py-H und 1-Thiazol-H); 8.30—8,65 ppm (m, 2H, Py).

Analog Beispiel 109 werden die nachfolgend aufgeführten Verbindungen erhalten, die der allgemeinen Formel I mit $R^1$ = Wasserstoff und $R^2$ = Methyl entsprechen und die im Pyridiniumrest (A in Formel I) die in der zweiten Spalte der Tabelle 5 angegebenen Substituenten tragen. Die Zahl der Substituenten gibt jeweils die genaue Stellung des oder der Substituenten im Pyridiniumrest an.

TABELLE 5

| Beispiel | Substituent | Ausbeute % d.Th. | $^1H$—NMR: δ (ppm) |
|---|---|---|---|
| 110 | 3-iso-$C_3H_7$ | 22 | ($CF_3CO_2D$): 1,44 (d, J=7Hz, 6H, $CH(CH_3)_2$); 3,04—4,08 (m, 3H, $CH(CH_3)_2$ und $SCH_2$); 4,22 (s, 3H, $OCH_3$); 5,26—5,29 (m, 4H, $CH_2$-Py und 2 Lactam-H); 7,38 (s, 1H, Thiazol); 7,91—8,84 (m, 4H, Py) |
| 111 | 2-$CH_3$-5-$CH_3$ | 23 | ($CF_3CO_2D$): 2,59 (s, 3H, $PyCH_3$); 2,93 (s, 3H, $PyCH_3$); 3,46 und 3,66 (AB, J=19Hz, 2H, $SCH_2$); 4,25 (s, 3H, $OCH_3$); 5,36—6,13 (m, 4H, $CH_2Py$ und 2 Lactam-H); 7,40 (s, 1H, Thiazol); 7,77—8,65 (m, 3H, Py) |
| 112 | 2-$CH_3$-4-$CH_3$ | 17 | ($CF_3CO_2D$): 2,70 (s, 3H, $CH_3$); 2,91 (s, 3H, $CH_3$); 3,47 und 3,67 (AB, J=19Hz, 2H, $SCH_2$); 4,24 (s, 3H, $OC_3$); 5,34—6,11 (m, 4H, $CH_2Py$ und 2 Lactam-H); 7,37 (s, 1H, Thiazol); 7,65—8,56 (m, 3H, Py) |
| 113 | 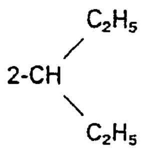 | 28 | ($CF_3CO_2D$): 0,80—2,24 (m, 10H, $CH(C_2H_5)_2$); 3,17—3,97 (m, 3H, $CH(C_2H_5)_2$ und $SCH_2$); 4,25 (s, 3H, $OCH_3$); 5,14—6,12 (m, 4H, $CH_2Py$ und 2 Lactam-H); 7,40 (s, 1H, Thiazol); 7,80—8,90 (m, 4H, Py) |
| 114 | 3-$CH_3$-4-$C_2H_5$ | 18 | ($CF_3CO_2D$): 1,44 (t, J=7Hz, 3H, $CH_2CH_3$); 2,59 (s, 3H, $PyCH_3$); 3,03 (q, J=7Hz, $CH_2CH_3$); 3,50 und 3,76 (AB, J=19Hz, 2H, $SCH_2$); 4,23 (s, 3H, $OCH_3$); 5,16—6,26 (m, 4H, $CH_2Py$ und 2 Lactam-H); 7,41 (s, 1H, Thiazol); 7,93 und 8,66 (AA'BB', J=6Hz, 3H, Py) |
| 115 | 4-$CH_2C_6H_5$ | 8 | ($CF_3CO_2D$): 3,46 und 3,77 (AB, J=18Hz, 2H, $SCH_2$); 4,22 (s, 3H, $OCH_3$); 4,32 (s, 2H, $CH_2C_6H_5$); 5,13—6,19 (m, 4H, $CH_2$-Py und 2 Lactam-H); 7,05—7,46 (m, 6H, $C_6H_5$ und Thiazol); 7,81 und 8,78 (AA'BB', J=6Hz, 4H, Py) |
| 116 | 4-$CH_2$-p-$C_6H_4Cl$ | 7 | ($CF_3CO_2D$): 3,47 und 3,78 (AB, J=18Hz, 2H, $SCH_2$); 4,24 (s, 3H, $OCH_3$); 4,33 (s, 2H, $CH_2C_6H_4$); 5,18—6,23 (m, 4H, $CH_2Py$ und 2 Lactam-H); 7,05—7,46 (m, 5H, $C_6H_4$ und Thiazol); 7,87 und 8,83 (AA'BB', J=6Hz, 4H, Py) |
| 117 | 3 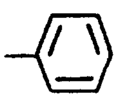 | 11 | ($CF_3CO_2D$): 3,57 und 3,85 (AB, J=19Hz, 2H, $SCH_2$); 4,22 (s, 3H, $OCH_3$); 5,36—6,39 (m, 4H, $CH_2Py$ und 2 Lactam-H); 7,39 (s, 1H, Thiazol); 7,63 (s, 5H, $C_6H_5$); 8,03—9,20 (m, 4H, Py) |
| 118 | 3-$CH_2$—$CH=CH_2$ | 21 | ($CF_3CQ_2D$): 3,23—4,07 (m, 4H, CH—$CH_2$ und $SCH_2$); 4,23 (s, 3H, $OCH_3$); 5,09—6,31 (m, 7H, $CH=CH_2$, $CH_2Py$ und 2 Lactam-H); 7,38 (s, 1H Thiazol); 7,91—8,93 (m, 4H, Py) |
| 119 | 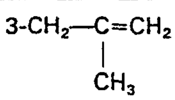 | 20 | ($CF_3CO_2D$): 1,79 (s, 3H, $CCH_3$); 3,21—4,23 (m, 7H, $SCH_2$, $CH_2C$ und $OCH_3$); 4,80—6,31 (m, 6H, $C=CH_2$, $CH_2Py$ und 2 Lactam-H); 7,40 (s, 1H, Thiazol); 7,92—8,91 (m, 4H, Py) |
| 120 | 3-CH=CH—$CH_3$ | 23 | ($CF_3CO_2D$): 2,06 (d, J=6Hz, 3H, CH—$CH_3$); 3,51 und 3,80 (AB, J=19Hz, 2H, $SCH_2$); 4,23 (s, 3H, $OCH_3$); 5,22—6,79 (m, 6H, CH=CH, $CH_2Py$ und 2 Lactam-H); 7,42 (s, 1H, Thiazol); 7,86—8,82 (m, 4H, Py) |

TABELLE 5 — cont.

| Beispiel | Substituent | Ausbeute % d.Th. | $^1$H—NMR: δ (ppm) |
|---|---|---|---|
| 121 | 4-$CH_2$—$CH_2$—CH=$CH_2$ | 23 | ($CF_3CO_2D$): 2,40—3,27 (m, 4H, $CH_2$—$CH_2$); 3,48 und 3,78 (AB, J=19Hz, 2H, $SCH_2$); 4,24 (s, 3H, $OCH_3$); 4,90—6,24 (m, 7H, CH=$CH_2$, $CH_2$Py und 2 Lactam-H); 7,41 (s, 1H, Thiazol); 7,96 und 8,81 (AA'BB', J=6Hz, 4H, Py) |
| 122 | 5-OH-2-$CH_3$ | 13 | ($CF_3CO_2D$): 2,86 (s, 3H, $PyCH_3$); 3,51 und 3,72 (AB, J=18Hz, 2H, $SCH_2$); 4,26 (s, 3H, $OCH_3$); 5,37—6,13 (m, 4H, $CH_2$Py und 2 Lactam-H); 7,41 (s, 1H, Thiazol); 7,66—8,39 (m, 3H, Py) |
| 123 | 3-OH-4-$CH_3$ | 22 | ($CF_3CO_2D$): 2,60 (s, 3H, $CH_3$); 3,45 und 3,80 (AB, J=19Hz, 2H, $SCH_2$); 4,23 (s, 3H, $OCH_3$); 5,15—6,25 (m, 4H, $CH_2$Py und 2 Lactam-H); 7,41 (s, 1H, Thiazol); 7,70—8,75 (m, 3H, Py) |
| 124 | 4-CH—$C_2H_5$<br>  \|<br> OH | 18 | ($CF_3CO_2D$): 1,13 (t, J=7Hz, 3H, $CH_3$); 1,90—2,28 (m, 2H, $CH_2CH_3$); 3,53 und 3,81 (AB, J=19Hz, 2H, $SCH_2$); 4,23 (s, 3H, $OCH_3$); 5,07—6,27 (m, 5H, CHOH, $CH_2$Py und 2 Lactam-H); 7,38 (s, 1H, Thiazol); 8,15 und 8,94 (AA'BB', J=6Hz, 4H, Py) |
| 125 | 3-CH—$C_2H_5$<br>  \|<br> OH | 14 | ($CF_3CO_2D$): 1,11 (t, J=7Hz, 3H, $CH_3$); 1,93—2,28 (m, 2H, $CH_2CH_3$); 3,55 und 3,82 (AB, J=19Hz, 2H, $SCH_2$); 4,23 (s, 3H, $OCH_3$); 5,07—6,34 (m, 5H, CHOH, $CH_2$Py und 2 Lactam-H); 7,39 (s, 1H, Thiazol); 8,01—9,14 (m, 4H, Py) |
| 126 | $CH_3$<br>\|<br>3-$CH_2$—C—OH<br>\|<br>$CH_3$ | 28 | ($CF_3CO_2D$): 1,47 (s, 6H, $C(CH_3)_2$); 3,23 (bs, 2H, $PyCH_2$); 3,54 und 3,81 (AB, J=19Hz, 2H, $SCH_2$); 4,24 (s, 3H, $OCH_3$); 5,29—6,33 (m, 4H, $CH_2$Py und 2 Lactam-H); 7,41 (s, 1H, Thiazol); 7,96—9,00 (m, 4H, Py) |
| 127 | 2-$CH_2$OH-4-$CH_3$ | 21 | ($CF_3CO_2D$): 2,75 (s, 3H, $CH_3$); 3,45 und 3,75 (AB, J=19Hz, 2H, $SCH_2$); 4,23 (s, 3H, $OCH_3$); 5,15—6,20 (m, 6H, $CH_2$Py, $CH_2$OH und 2 Lactam-H); 7,41 (s, 1H, Thiazol); 7,60—8,75 (m, 3H, Py) |
| 128 | 2-$CH(CH_2OH)_2$ | 8 | ($CF_3CO_2D$): 3,35—4,57 (m, 10H, CH, $SCH_2$, 2$CH_2$OH, $OCH_3$); 5,25—6,25 (m, 4H, $CH_2$Py, 2 Lactam-H); 7,42 (s, 1H, Thiazol); 7,85—8,95 (m, 4H, Py) |
| 129 | 4-$CH_2CH(OH)CH_3$ | 14 | ($CF_3CO_2D$): 1,52 (d, J=7Hz, 3H, $CH_3$); 3.10—4.00 (m, 4H, $PyCH_2$ und $SCH_2$); 4,00—4,60 (m, 4H, $OCH_3$-Singulett und CH); 5,25—6,35 (m, 4H, $CH_2$Py und 2 Lactam-H); 7,41 (s, 1H, Thiazol); 8,07 und 8,90 (AA'BB', J=6Hz, 4H, Py) |
| 130 | 4-$CH_2$—$CH_2$—$CH_2$—OH | 14 | ($CF_3CO_2D$): 2,02—2,48 (m, 2H, $CH_2CH_2$); 2,97—4,23 (m, 9H, $PyCH_2$, $CH_2O$, $SCH_2$ und $OCH_3$); 5,27—6,27 (m, 4H, $CH_2$Py und 2 Lactam-H); 7,39 (s, 1H, Thiazol); 7,97 und 8,85 (AA'BB', J=6Hz, 4H, Py) |
| 131 | 3 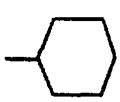 | 27 | ($CF_3CO_2D$): 1,29—2,17 (m, 10H, Cyclohexyl); 2,67—4,08 (m, 3H, Cyclohexyl und $SCH_2$); 4,23 (s, 3H, $OCH_3$); 5,22—6,39 (m, 4H, $CH_2$Py und 2 Lactam-H); 7,39 (s, 1H, Thiazol); 8,03—9,21 (m, 4H, Py) |

TABELLE 5 — cont.

| Beispiel | Substituent | Ausbeute % d.Th. | $^1$H—NMR: δ (ppm) |
|---|---|---|---|
| 132 | 4 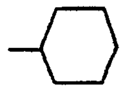 | 28 | ($CF_3CO_2D$): 1,23—2,24 (m, 10H, $CH_2$-Cyclohexyl); 2,82—4,10 (m, 3H, CH-Cyclohexyl, $SCH_2$); 4,23 (s, 3H, $OCH_3$); 5,18—6,23 (m, 4H, $CH_2$Py und 2 Lactam-H); 7,39 (s, 1H, Thiazol); 7,93 und 8,82 (AA'BB', J=6Hz, 4H, Py) |
| 133 | 3 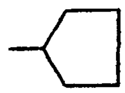 | 19 | ($CF_3CO_2D$): 1,38—4,23 (m, 14H, Cyclopentyl, $SCH_2$ und $OCH_3$); 5,23—6,30 (m, 4H, $CH_2$Py und 2 Lactam-H); 7,39 (s, 1H, Thiazol); 7,88—8,83 (m, 4H, Py) |
| 134 | 4 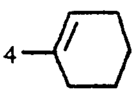 | 26 | ($CF_3CO_2D$): 1,71—2,68 (m, 8H, Cyclohexenyl), 3,53—3,77 (AB, J=19Hz, 2H, $SCH_2$); 4,23 (s, 3H, $OCH_3$), 5,14—6,19 (m, 4H, $CH_2$Py und 2 Lactam-H); 7,16 (bs, 1H, Cyclohexenyl); 7,40 (s, 1H, Thiazol); 8,03 und 8,69 (AA'BB', J=7Hz, 4H, Py) |
| 135 | 3 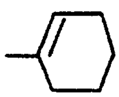 | 25 | ($CF_3CO_2D$): 1,69—2,04 (bs, 4H, Cyclohexenyl); 2,21—2,60 (m, 4H, Cyclohexenyl); 3,51 und 3,80 (AB, J=19Hz, 2H, $SCH_2$); 4,23 (s, 3H, $OCH_3$); 5,24—6,28 (m, 4H, $CH_2$Py und 2 Lactam-H); 6,51—6,73 (m, 1H, Cyclohexenyl); 7,37 (s, 1H, Thiazol); 7,85—8,93 (m, 4H, Py) |
| 136 | 4 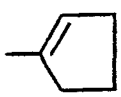 | 20 | ($CF_3CO_2D$): 1,99—3,03 (m, 6H, Cyclopentenyl); 3,50 und 3,77 (AB, J=19Hz, 2H, $SCH_2$); 4,24 (s, 3H, $OCH_3$); 5,15—6,19 (m, 4H, $CH_2$Py und 2 Lactam-H); 7,22 (bs, 1H, Cyclopentenyl); 7,41 (s, 1H, Thiazol); 7,99 und 8,70 ((AA'BB', J=6Hz, 4H, Py) |
| 137 | 3 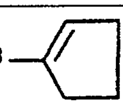 | 18 | ($CF_3CO_2D$): 2,03—3,03 (m, 6H, Cyclopentenyl); 3,53 und 3,81 (AB, J=19Hz, 2H, $SCH_2$); 4,23 (s, 3H, $OCH_3$); 5,28—6,32 (m, 4H, $CH_2$Py und 2 Lactam-H); 6,79 (bs, 1H, Cyclopentenyl); 7,41 (s, 1H, Thiazol); 7,89—8,97 (m, 4H, Py) |
| 138 | 3 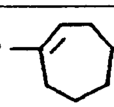 | 23 | ($CF_3CO_2D$): 1,55—2,06 (m, 6H, Cycloheptenyl); 2,33—2,84 (m, 4H, Cycloheptenyl); 3,51 und 3,80 (AB, J=19Hz, 2H, $SCH_2$); 4,23 (s, 3H, $OCH_3$); 5,24—6,61 (m, 5H, $CH_2$Py, 2 Lactam-H und Cycloheptenyl-H); 7,39 (s, 1H, Thiazol); 7,85—8,86 (m, 4H, Py) |
| 139 | 3 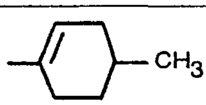 | 16 | ($CF_3CO_2D$): 1,04—2,69 (m, 10H, Methylcyclohexenyl); 3,52 und 3,80 (AB, J=19Hz, 2H, $SCH_2$); 4,23 (s, 3H, $OCH_3$); 5,24—6,32 (m, 4H, $CH_2$Py und 2 Lactam-H); 6,62 (m, 1H, Methylcyclohexenyl); 7,40 (s, 1H, Thiazol); 7,85—8,94 (m, 4H, Py) |
| 140 | 3 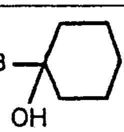 | 20 | ($CF_3CO_2D$): 1,68—2,21 (bs, 10H, Cyclohexyl); 3,55 und 3,79 (AB, J=19Hz, 2H, $SCH_2$); 4,23 (s, 3H, $OCH_3$); 5,34—6,27 (m, 4H, $CH_2$Py und 2 Lactam-H); 7,37 (s, 1H, Thiazol); 7,37—9,29 (m, 4H, Py) |
| 141 | 3 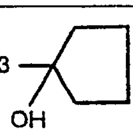 | 18 | ($CF_3CO_2D$): 2,19 (bs, 8H, Cyclopentyl); 3,54 und 3,82 (AB, J=19Hz, 2H, $SCH_2$); 4,23 (s, 3H, $OCH_3$); 5,35—6,30 (m, 4H, $CH_2$Py und 2 Lactam-H); 7,37 (s, 1H, Thiazol); 7,97—9,30 (m, 4H, Py) |

TABELLE 5 — cont.

| Beispiel | Substituent | Ausbeute % d.Th. | $^1$H—NMR: δ (ppm) |
|---|---|---|---|
| 142 | 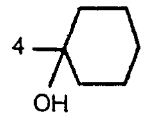 | 22 | ($CF_3CO_2D$): 1,95 (bs, 10H, Cyclohexyl); 3,51 und 3,83 (AB, J=19Hz, 2H, $SCH_2$); 4,24 (s, 3H, $OCH_3$); 5,24—6,26 (m, 4H, $CH_2$Py und 2 Lactam-H); 7,39 (s, 1H, Thiazol); 8,27 und 8,93 (AA'BB', J=6Hz, 4H, Py) |
| 143 | 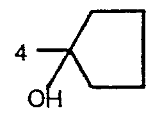 | 15 | ($CF_3CO_2D$): 2,17 (bs, 8H, Cyclopentyl); 3,52 und 3,82 (AB, J=19Hz, 2H, $SCH_2$); 4,23 (s, 3H, $OCH_3$); 5,26—6,28 (m, 4H, $CH_2$Py und 2 Lactam-H); 7,39 (s, 1H, Thiazol); 8,25 und 8,91 (AA'BB', J=7Hz, 4H, Py) |
| 144 | 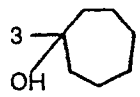 | 21 | ($CF_3CO_2D$): 1,84 (bs, 8H, Cycloheptyl); 2,17 (bs, 4H, Cycloheptyl); 3,54 und 3,81 (AB, J=19Hz, 2H, $SCH_2$); 4,23 (s, 3H, $OCH_3$); 5,35—6,29 (m, 4H, $CH_2$Py und 2 Lactam-H); 7,38 (s, 1H, Thiazol); 7,97—9,28 (m, 4H, Py) |
| 145 | 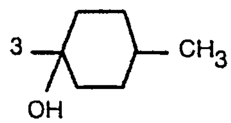 | 16 | ($CF_3CO_2D$): 0,98—2,48 (m, 12H, Methylcyclohexyl); 3,56 und 3,80 (AB, J=19Hz, 2H, $SCH_2$); 4,23 (s, 3H, $OCH_3$); 5,36—6,30 (m, 4H, $CH_2$Py und 2 Lactam-H); 7,39 (s, 1H, Thiazol); 7,92—9,32 (m, 4H, Py) |
| 146 | 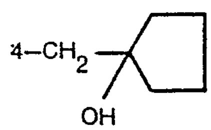 | 17 | ($CF_3CO_2D$): 1,91 (bs, 8H, Cyclopentyl); 3,39 (bs, 2H, $PyCH_2$); 3,58 und 3,82 (AB, J=19Hz, 2H, $SCH_2$); 4,24 (s, 3H, $OCH_3$); 5,23—6,27 (m, 4H, $CH_2$Py und 2 Lactam-H); 7,42 (s, 1H, Thiazol); 8,08 und 8,87 (AA'BB', J=6Hz, 4H, Py) |
| 147 | 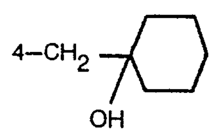 | 22 | ($CF_3CO_2D$): 1,71 (bs, 10H, Cyclohexyl); 3,27 (bs, 2H, $PyCH_2$); 3,59 und 3,81 (AB, J=19Hz, 2H, $SCH_2$); 4.23 (s, 3H, $OCH_3$); 5,19—6,25 (m, 4H, $CH_2$Py und 2 Lactam-H); 7,42 (s, 1H, Thiazol); 8,03 und 8,86 (AA'BB', J=6Hz, 4H, Py) |
| 148 | 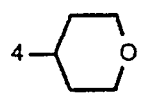 | 21 | ($CF_3CO_2D$): 1,83—2,30 (m, 4H, Pyranyl); 3,03—4,66) (m, 10H, Pyranyl, $SCH_2$ und $OCH_3$); 5,21—6,26 (m, 4H, $CH_2$Py und 2 Lactam-H); 7,39 (s, 1H, Thiazol); 8,01 und 8,93 (AA'BB', J=6Hz, 4H, Py) |
| 149 | 4-$OCH_3$-3-$CH_3$ | 18 | ($CF_3CO_2D$): 2,40 (s, 3H, $CH_3$); 3,45 und 3,77 (AB, J=19Hz, 2H, $SCH_2$); 4,24 (bs, 6H, 2×$OCH_3$); 5,03—6,20 (m, 4H, $CH_2$Py und 2 Lactam-H); 7,41 (s, 1H, Thiazol); 7,38 (d, J=7Hz, 1H, Py); 8,35—8,85 (m, 2H, Py) |
| 150 | 3-$OCH_3$-4-$CH_3$ | 23 | ($CF_3CO_2D$): 2,60 (s, 3H, $CH_3$); 3,45 und 3,82 (AB, J=19Hz, 2H, $SCH_2$); 4,13 (s, 3H, $OCH_3$); 4,23 (s, 3H, $OCH_3$); 5,15—6,30 (m, 4H, $CH_2$Py und 2 Lactam-H); 7,41 (s, 2H, Thiazol); 7,72—8,60 (m, 3H, Py) |
| 151 | 3-$OCH_3$ | 21 | ($CF_3CO_2D$): 3,50 und 3,83 (AB, J=19Hz, 2H, $SCH_2$); 4,12 (s, 3H, $OCH_3$); 4,24 (s, 3H, $OCH_3$); 5,17—6,40 (m, 4H, $CH_2$Py und 2 Lactam-H); 7,42 (s, 1H, Thiazol); 7,95—8,80 (m, 4H, Py) |
| 152 | 4-$OC_2H_5$ | 18 | ($CF_3CO_2D$): 1,60 (t, J=7Hz, 3H, $CH_3$); 3,45 und 3,80 (AB, J=19Hz, 2H, $SCH_2$); 4,24 (s, 3H, $OCH_3$); 4,30 (q, J=7Hz, $CH_2CH_3$); 5,00—6,20 (m, 4H, $CH_2$Py und 2 Lactam-H); 7,41 (s, 1H, Thiazol); 7,40 und 8,70 (AA'BB', J=6Hz, 4H, Py) |

TABELLE 5 — cont.

| Beispiel | Substituent | Ausbeute % d.Th. | $^1H$—NMR: δ (ppm) |
|---|---|---|---|
| 153 | 3-$OC_2H_5$ | 24 | ($CF_3CO_2D$): 1,56 (t, J=7Hz, 3H, $CH_3$); 3,46 und 3,82 (AB, J=19Hz, 2H, $SCH_2$); 4,30 (q, J=7Hz, $CH_2CH_3$); 4,24 (s, 3H, $OCH_3$); 5,15—6,35 (m, 4H, $CH_2Py$ und 2 Lactam-H); 7,41 (s, 1H, Thiazol); 7,90—8,75 (m, 4H, Py) |
| 154 | 3-$OCH(CH_3)_2$ | 17 | ($CF_3CO_2D$): 1,45 und 1,55 (d, J=6Hz, 6H, 2$CH_3$); 3,45 und 3,85 (AB, J=19Hz, 2H, $SCH_2$); 4,24 (s, 3H, $OCH_3$); 4,85 (m, 1H, CH); 5,15—6,35 (m, 4H, $CH_2Py$ und 2 Lactam-H); 7,41 (s, 1H, Thiazol); 7,85—8,70 (m, 4H, Py) |
| 155 | 3-$OCH_2CH_2CH_3$ | 19 | ($CF_3CO_2D$): 1,11 (t, J=7Hz, 3H, $CH_3$) 1,62—2,35 (m, 2H, $CH_2$); 3,50 und 3,82 (AB, J=19Hz, 2H, $SCH_2$); 4,00—4,45 (m, 5H, $CH_2$ und $OCH_3$); 5,15—6,35 (m, 4H, $CH_2Py$ und 2 Lactam-H); 7,41 (s, 1H, Thiazol); 7,90—8,75 (m, 4H, Py) |
| 156 | 2-$CH_2$—$CH_2$—O—$CH_3$ | 18 | ($CF_3CO_2D$): 3,24—4,50 (m, 10H, $CH_2$—$CH_2$, $OCH_3$ und $OCH_3$); 5,05—6,15 (m, 4H, $CH_2Py$ und 2 Lactam-H); 7,40 (s, 1H, Thiazol); 7,80—8,83 (m, 4H, Py) |
| 157 | 4-$CH_2$—$CH_2OC_2H_5$ | 12 | ($CF_3CO_2D$): 1,33 (t, J=7Hz, 3H, $CH_3$); 3,27—4,40 (m, 11H, 3$CH_2$, $SCH_2$, $OCH_3$); 5,15—6,30 (m, 4H, $CH_2Py$, 2 Lactam-H); 7,42 (s, 1H, Thiazol); 8,07 und 8,90 (AA'BB', J=6Hz, 4H, Py) |
| 158 | 2-$CH_2$—$CH_2$—O—$C_2H_5$ | 20 | ($CF_3CO_2D$): 1,33 (t, J=7Hz, 3H, $CH_2CH_3$); 3,49—4,26 (m, 11H, 3$CH_2$, $SCH_2$, $OCH_3$); 5,38—6,20 (m, 4H, $CH_2Py$ und 2 Lactam-H); 7,42 (s, 1H, Thiazol); 7,88—8,86 (m, 4H, Py) |
| 159 | 4-$COCH_3$ | 17 | ($CF_3CO_2D$): 2,90 (s, 3H, $CH_3$); 3,55 und 3,85 (AB, J=19Hz, 2H, $SCH_2$); 4,24 (s, 3H, $OCH_3$); 5,25—6,50 (m, 4H, $CH_2Py$ und 2 Lactam-H); 7,41 (s, 1H, Thiazol); 8,57 und 9,30 (AA'BB', J=6Hz, 4H, Py) |
| 160 | 4-$CH_2COCH_3$ | 12 | ($CF_3CO_2D$): 2,53 (s, 3H, $CH_3$); 5,30 und 3,85 (AB, J=19Hz, 2H, $SCH_2$); 4,00—4,45 (m, 5H, $CH_2$ und $OCH_3$); 5,20—6,35 (m, 4H, $CH_2Py$ und 2 Lactam-H); 7,41 (s, 1H, Thiazol); 8,02 und 8,95 (AA'BB', J=6Hz, 4H, Py) |
| 161 | 4-$CH_2$—$CH_2$—$CH_2$-Cl | 28 | ($CF_3CO_2D$): 2,15—2,48 (m, 2H, $CH_2CH_2$); 3,04—4,23 (m, 9H, $PyCH_2$, $CH_2Cl$, $SCH_2$, $OCH_3$); 5,21—6,26 (m, 4H, $CH_2Py$ und 2-Lactam-H); 7,39 (s, 1H, Thiazol); 7,98 und 8,87 (AA'BB', J=6Hz, 4H, Py) |
| 162 | 3-$CH_2$—$CH_2$—$CH_2$-Cl | 16 | ($CF_3CO_2D$): 2,13—3,77 (m, 8H, Py-$CH_2$—$CH_2$—$CH_2$ *und* $SCH_2$); 4,23 (s, 3H, $OCH_3$); 5,26—6,33 (m, 4H, $CH_2Py$ und 2 Lactam-H); 7,42 (s, 1H, Thiazol); 7,94—8,96 (m, 4H, Py) |
| 163 | 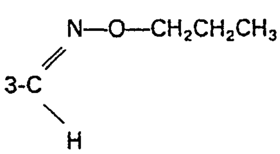 | 29 | ($CF_3CO_2D$): 1,03 (t, J=7Hz, 3H, $CH_2CH_3$); 1,66—2,01 (m, 2H, $CH_2CH_3$); 3,24—4,45 (m, 7H, $SCH_2$, $OCH_2$ und $OCH_3$); 5,30—6,37 (m, 4H, $CH_2Py$ und 2 Lactam-H); 7,41 (s, 1H, Thiazol); 7,33—9,20 (m, 5H, Py und CH) |

TABELLE 5 — cont.

| Beispiel | Substituent | Ausbeute % d.Th. | $^1$H—NMR: δ (ppm) |
|---|---|---|---|
| 164 | 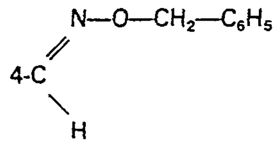 | 20 | ($CF_3CO_2D$): 3,53 und 3,82 (AB, J=19Hz, 2H, $SCH_2$); 4,24 (s, 3H, $OCH_3$); 5,23—6,26 (m, 6H, $CH_2$Py, 2 Lactam-H und $OCH_2$); 7,37 (s, 5H, $C_6H_5$); 8,25 und 8,88 (AA'BB', J=7Hz, 4H, Py); 8,28 (s, 1H, CH) |
| 165 | 3-$CF_3$ | 9 | ($CF_3CO_2D$): 3,55 und 3,90 (AB, J=19Hz, 2H, $SCH_2$); 4,23 (s, 3H, $OCH_3$); 5,15—6,45 (m, 4H, $CH_2$Py und 2 Lactam-H); 7,41 (s, 1H, Thiazol); 8,15—9,52 (m, 4H, Py) |
| 166 | 3-Br-4-$CH_3$ | 17 | ($CF_3CO_2D$): 2,80 (s, 3H, $CH_3$); 3,50 und 3,85 (AB, J=19Hz, 2H, $SCH_2$); 4,23 (s, 3H, $OCH_3$); 5,10—6,40 (m, 4H, $CH_2$Py und 2 Lactam-H); 7,41 (s, 1H, Thiazol); 7,89 (d, J=6Hz, 1H, Py); 8,70—9,20 (2H, Py) |
| 167 | 3 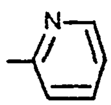 | 16 | ($CF_3CO_2D$): 3,60 und 3,95 (AB, J=19Hz, 2H, $SCH_2$) 4,23 (s, 3H, $OCH_3$); 5,25—6,55 (m, 4H, $CH_2$Py und 2 Lactam-H); 7,40 (s, 1H, Thiazol); 8,2—9,85 (m, 8H, Py) |
| 168 | 3-$CH_2$—CN | 14 | ($CF_3CO_2D$): 3,57 und 3,84 (AB, J=19Hz, 2H, $SCH_2$); 4,23 (s, 3H, $OCH_3$); 4,33 (s, 2H, $CH_2CN$); 5,36—6,41 (m, 4H, $CH_2$Py und 2 Lactam-H); 7,40 (s, 1H, Thiazol); 8,08—9,21 (m, 4H, Py) |
| 169 | 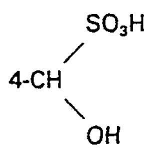 | 15 | ($CF_3CO_2D$): 3,30—4,11 (m, 2H, $SCH_2$); 4,23 (s, 3H, $OCH_3$); 5,36—6,46 (m, 5H, $CH_2$Py, 2 Lactam-H und CH—OH); 7,42 (s, 1H, Thiazol); 8,73 und 9,30 (AA'BB', J=6Hz, 4H, Py) |
| 170 | 4-CH=CH—$CO_2CH_3$ | 21 | ($CF_3CO_2D$): 3,55 und 3,83 (AB, J=19Hz, 2H, $SCH_2$); 4,04 (s, 3H, $CO_2CH_3$); 4,24 (s, 3H, $OCH_3$); 5,28—6,36 (m, 4H, $CH_2$Py und 2 Lactam-H); 7,15 und 7,84 (AB, J=16Hz, 2H, CH=CH); 7,41 (s, 1H, Thiazol); 8,25 und 9,01 (AA'BB', J=6Hz, 4H, Py) |
| 171 | 3-CH=CH—$CO_2CH_3$ | 16 | ($CF_3CO_2D$): 3,57 und 3,84 (AB, J=19Hz, 2H, $SCH_2$); 4,03 (s, 3H, $CO_2CH_3$); 4,23 (s, 3H, $OCH_3$); 5,36—6,39 (m, 4H, $CH_2$Py und 2 Lactam-H); 6,98 und 7,86 (AB, J=16Hz, 2H, CH=CH); 7,40 (s, 1H, Thiazol); 8,07—9,21 (m, 4H, Py) |

Analog Beispiel 109 werden die nachfolgend aufgeführten Verbindungen erhalten, die der allgemeinen Formel I mit $R^1$ = Wasserstoff und $R^2$ = Methyl entsprechen und als Rest A den in der zweiten Spalte der Tabelle 6 angegebenen Substituenten tragen.

TABELLE 6

| Beispiel | A | Ausbeute % d.Th. | $^1$H—NMR: δ (ppm) |
|---|---|---|---|
| 172 | 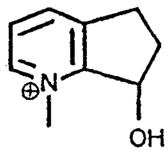 | 21 | ($CF_3CO_2D$): 2,1—3,9 (m, 6H, 4 Cyclopenten-H, $SCH_2$); 4,23 (s, 3H, $OCH_3$); 5,1—6,4 (m, 5H, $CH_2Py$, Cyclopenten-H, 2 Lactam-H); 7,42 (s, 1H, Thiazol); 7,8—8,9 (m, 3H, Py) |
| 173 | 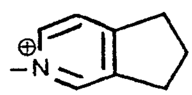 | 17 | ($CF_3CO_2D$): 2,3—3,9 (m, 8H, 6 Cyclopenten-H, $SCH_2$); 4,23 (s, 3H, $OCH_3$); 5,05—6,3 (m, 4H, $CH_2Py$, 2 Lactam-H); 7,40 (s, 1H, Thiazol); 7,7—8,8 (m, 3H, Py) |
| 174 | 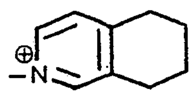 | 15 | ($CF_3CO_2D$): 1,8—2,3 (m, 4H, Cyclohexen-H); 2,7—3,7 (m, 6H, $SCH_2$, Cyclohexen-H); 4,23 (s, 3H, $OCH_3$); 5,05—6,3 (m, 4H, $CH_2Py$, 2 Lactam-H); 7,41 (s, 1H, Thiazol); 7,6—8,7 (m, 3H, Py) |
| 175 | 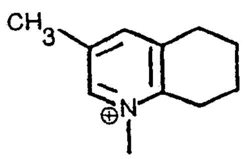 | 18 | ($CF_3CO_2D$): 1,8—2,35 (m, 4 Cyclohexen-H); 2,55 (s, 3H, $CH_3$); 2,9—3,7 (m, 6H, $SCH_2$ und 4 Cyclohexen-H); 4,24 (s, 3H, $OCH_3$), 5,3—6,25 (m, 4H, $CH_2Py$, 2 Lactam-H); 7,41 (s, 1H, Thiazol); 8,12 und 8,42 (je 1H, Bs, Py) |
| 176 | 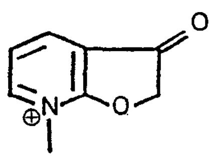 | 4 | ($CF_3CO_2D$): 3,51 und 3,74 (AB, J=19Hz, 2H, $SCH_2$); 4,24 (s, 3H, $OCH_3$); 5,35 (s, 2H, $CH_2$); 5,1—6,3 (m, 4H, $CH_2Py$ und 2 Lactam-H); 7,41 (s, 1H, Thiazol); 7,65—9,05 (m, 3H, Py) |
| 177 | 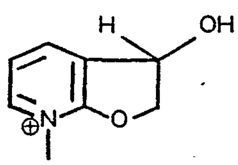 | 3 | ($CF_3CO_2D$): 3,52 und 3,75 (AB, J=19Hz, 2H, $SCH_2$); 4,23 (s, 3H, $OCH_3$); 5,0—6,3 (m, 7H, $CH_2Py$, 2 Lactam-H, 3 Furan-H); 7,41 (s, 1H, Thiazol); 7,3—8,9 (m, 3H, Py) |
| 178 | 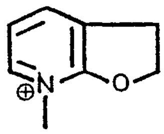 | 5 | ($CF_3CO_2D$): 3,3—3,8 (m, 4H, $SCH_2$ und 2 Furan-H); 4,23 (s, 3H, $OCH_3$); 4,9—6,4 (m, 6H, $CH_2Py$, 2 Lactam-H, 2 Furan-H, $OCH_2$); 7,42 (s, 1H, Thiazol); 7,1—8,4 (m, 3H, Py) |
| 179 | 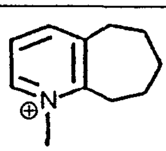 | 21 | ($CF_3CO_2D$): 1,6—2,35 (m, 6 Cyclohepten-H); 3,0—3,95 (m, 6H, 4 Cyclohepten-H und $SCH_2$) 4,25 (s, 3H, $OCH_3$); 5,25—6,35 (m, 4H, $CH_2Py$ und 2 Lactam-H); 7,42 (s, 1H, Thiazol); 7,6—8,73 (m, 3H, Py) |

Analog Beispiel 109 werden die nachfolgend aufgeführten Verbindugen erhalten, die der allgemeinen Formel I mit $R^1$ = Chlor und $R^2$ = Methyl entsprechen und die im Pyridiniumrest (A in Formel I) die in der zweiten Spalte der Tabelle 7 angegebenen Substituenten tragen. Im Falle des Beispiels 189 bedeutet der in Spalte 2 stehende Rest den Rest A in Formel I.

TABELLE 7

| Beispiel | Substituent | Ausbeute % d.Th. | $^1$H—NMR: δ (ppm) |
|---|---|---|---|
| 180 | 3-$CH_3$ | 13 | ($CF_3CO_2D$): 2,65 (s, 3H, $CH_3$); 3,68 und 4,10 (AB, J=19Hz, 2H, $SCH_2$); 4,20 (s, 3H, $OCH_3$); 5,15—6,30 (m, 4H, $CH_2$Py und 2 Lactam-H); 7,8—9,0 (m, 4H, Py) |
| 181 | 3,4-Di-$CH_3$ | 15 | ($CF_3CO_2D$): 2,55 (s, 3H, $CH_3$); 2,65 (s, 3H, $CH_3$); 3,43 und 3,75 (AB, J=19Hz, 2H, $SCH_2$); 4,20 (s, 3H, $OCH_3$); 5,1—6,3 (m, 4H, $CH_2$Py und 2 Lactam-H); 7,6—8,8 (m, 3H, Py) |
| 182 | | | |
| 183 | 4-$CH_2OH$ | 21 | ($CF_3CO_2D$): 3,50 und 3,82 (AB, J=19Hz, 2H, $SCH_2$); 4,22 (s, 3H, $OCH_3$); 5,25 (s, $CH_2OH$); 5,0—6,2 (m, 4H, $CH_2$Py und 2 Lactam-H); 8,0—9,1 (m, 3H, Py) |
| 184 | 4-$CH_2OCH_3$ | 24 | ($CF_3CO_2D$): 3,2—4,1 (AB, J=19Hz, 2H, $SCH_2$); 3,73 (s, 3H, $OCH_3$); 4,21 (s, 3H, $OCH_3$); 5,00 (s, 2H, $CH_2$—$OCH_3$); 5,2—6,3 (m, 4H, $CH_2$Py und 2 Lactam-H); 8,0—9,1 (AA'BB', J=6Hz, 4H, Py) |
| 185 | 4-$CONH_2$ | 17 | ($CF_3CO_2D$): 3,52 und 3,82 (AB, J=19Hz, $SCH_2$); 4,21 (s, 3H, $OCH_3$); 5,40 (d, 1H, J=5Hz, C-6-H); 5,3—6,5 (AB, 2H, $CH_2$Py); 6,08 (d, 1H, J=5Hz, C-7-H); 8,57—9,23 (AA'BB', J=6Hz, 4H, Py) |
| 186 | 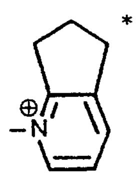 * Rest A in Formel I | 20 | ($CF_3CO_2D$): 2,2—2,8 (m, 2H, Cyclopenten-H); 3,1—4,2 (m, 6H, 4 Cyclopenten-H, $SCH_2$); 4,21 (s, 3H, $OCH_3$); 5,2—6,2 (m, 4H, $CH_2$Py, 2 Lactam-H); 7,6—8,6 (m, 3H, Py) |

Beispiel 187—266

In Analogie zu Beispiel 108 (Verfahren b) werden die Verbindungen der Tabellen 5, 6 und 7 aus den entsprechenden 7-Amino-ceph-3-em-4-carbonsäure-Derivaten der allgemeinen Formel III und den 2-(2-Aminothiazol-4-yl)-2-syn-oxyiminoessigsäuren der allgemeinen Formel IV dargestellt. Die Verbindungen sind in allen Eigenschaften mit denen aus den Tabellen 5, 6 und 7 identisch.

Beispiel 267

3-[(2,3-cyclopenteno-1-pyridinium)methyl]-7-[2-syn-hydroxyimino-2-(2-aminothiazol-4-yl)acetamido]-ceph-3-em-4-carboxylat

Aus 4,3 g (10 mmol) 2-(2-Trityl-amino-1,3-thiazol-4-yl)-2-syn-hydroxy-iminoessigsäure, 1,68 g (11 mmol) 1-Hydroxy-1H-benzotriazol-Hydrat und 2,48 g (12 mmol) Dicyclohexyl-carbodiimid in 70 ml N,N-Dimethylformamid wird in Analogie zu Beispiel 108 b eine Lösung des Aktivesters bereitet. Nach 3 Stunden wird dieses Gemisch in eine auf −40°C gekühlte Lösung von 4,45 g (11 mmol) 7-Amino-3-[(2,3-Cyclopenteno-1-pyridinium)methyl]-ceph-3-em-4-carboxylat-Dihydrochlorid, 50 ml N,N-Dimethylformamid und 4 ml (32 mmol) N,N-Dimethylanilin eingerührt und über Nacht bei Raumtemperatur belassen. Vom Dicyclohexylharnstoff wird filtriert und das gelbbraun gefärbte Filtrat in 1 l Diäthyläther eingetropft. Vom Ungelösten wird dekantiert, der Niederschlag mit Aceton verrührt, abgesaugt, mit Aceton gewaschen und getrocknet. Das Rohrodukt (3,5 g) wird in 30 ml Trifluoressigsäure gelöst und 25 Minuten bei Raumtemperatur gerührt. Die flüchtigen Anteile werden im Vakuum entfernt, der Rückstand mit Äther/n-Pentan (2:1) digeriert, abgesaugt und mit demselben Gemisch gewaschen. Der Niederschlag wird sodann in 5 ml Wasser unter Zusatz von Natriumbicarbonat gelöst und über eine "Lobar C" — Säule (Merck) mit Aceton/Wasser (2:1) chromatographiert. Durch Gefriertrocknen der Produktfraktionen erhält man 1,1 g (22% d.Th.) eines farblosen amorphen Feststoffs.

$^1$H-NMR ($CF_3CO_2D$): δ = 2,2—2,8 (m, 2H, Cyclopenten-H); 3,1—4,1 (m, 6H, Cyclopenten-H und $SCH_2$); 5,2—6,3 (m, 4H, $CH_2Py$ und 2 Lactam-H); 7,48 (s, 1H, Thiazol); 7,6—8,6 ppm (m, 3H, Py).

Beispiel 268

3-[(4-Cyclopropyl-1-pyridinium)methyl]-7-[2-syn-hydroxyimino-2-(2-aminothiazol-4-yl)acetamido]-ceph-3-em-4-carboxylat

Aus 2-(2-Tritylamino-1,3-thiazol-4-yl)-2-syn-hydroxyiminoessigsäure und 7-Amino-3-[(4-Cyclopropyl-1-pyridinium)methyl]-ceph-3-em-4-carboxylat-Dihydrochlorid nach dem HOBT-Verfahren in Analogie zu Beispiel 272. Nach Abspaltung der Tritylschutzgruppe mit Trifluoressigsäure und Chromatographie wird die Titelverbindung in Form eines farblosen amorphen Feststoffs in 25% Ausbeute erhalten.

$^1$H-NMR ($CF_3CO_2D$): δ = 1,03—2,6 (m, 5H, Cyclopropyl); 3,40 und 3,82 (AB, J=19Hz, 2H, $SCH_2$); 5,0—6,25 (m, 4H, $CH_2Py$ und 2 Lactam-H); 7,50 (s, 1H, Thiazol); 7,64 und 8,66 ppm (AA', BB', J=7Hz, 4H, Py)

Die folgenden Verbindungen werden in Analogie zu Beispiel 272 aus den 7-Amino-ceph-3-em-4-carboxylat-Dihydrochloriden der allgemeinen Formel III und den entsprechenden 2-(2-Amino-1,3-thiazol-4-yl)-2-syn-oximinoessigsäuren der allgemeinen Formel IV ($R^4$ = H) hergestellt.

Beispiel 269

3-[(4-Cyclopropyl-1-pyridinium)methyl]-7-[2-syn-aethoxyimino-2-(2-aminothiazol-4-yl)acetamido]-ceph-3-em-4-carboxylat

Farbloser Feststoff, Ausbeute 28% d.Th.

$^1$H-NMR ($CF_3CO_2D$): δ = 1,1—2,6 (m, 8H, Cyclopropyl und $CH_2\underline{CH_3}$ bei 1,43); 3,43 und 3,76 (AB, J=19Hz, 2H, $SCH_2$) 4,53 (q, J=7Hz, 2H, $\underline{CH_2}CH_3$); 5,1—6,2 (m, 4H, $CH_2Py$ und 2 Lactam-H); 7,42 (s, 1H, Thiazol); 7,63 und 8,66 ppm (AA', BB', J=7Hz, 4H, Py)

Beispiel 270

3-[(4-Cyclopropyl-1-pyridinium)methyl]-7-[2-(2-amino-5-bromthiazol-4-yl)-2-syn-methoxyimino-acetamido]-ceph-3-em-4-carboxylat

Hellgelber Feststoff, Ausbeute 18% d.Th.

$^1$H-NMR ($CF_3CO_2D$): δ = 1,05—2,55 (m, 5H, Cyclopropyl); 3,39 und 3,82 (AB, J=19Hz, 2H, $SCH_2$); 4,22 (s, 3H, $OCH_3$); 5,05—6,23 (m, 4H, $CH_2Py$ und 2 Lactam-H); 7,58 und 8,66 ppm (AA', BB', J=7Hz, 4H, Py)

Beispiel 271

3-[(4-Cyclopropyl-1-pyridinium)methyl]-7-[2-syn-aethoxy-imino-2-(2-amino-5-chlorthiazol-4-yl)acetamido]-ceph-3-em-4-carboxylat

Farbloser Feststoff, Ausbeute 20% d.Th.

$^1$H-NMR ($CF_3CO_2D$): δ = 1,06—2,48 (m, 8H, Cyclopropyl und $—CH_2\underline{CH_3}$ bei 1,42); 3,42 und 3,82 (AB, J=19Hz, 2H, $SCH_2$); 4,51 (q, J=7Hz, $\underline{CH_2}—CH_3$); 5,03—6,23 (m, 4H, $CH_2Py$ und 2 Lactam-H); 7,63 und 8,66 ppm (AA'BB', J=7Hz, 4H, Py)

Beispiel 272

3-[(2,3-Cyclopenteno-1-pyridinium)methyl]-7-[2-syn-isopropyloxyimino-2-(2-aminothiazol-4-yl)acetamido]-ceph-3-em-4-carboxylat

Farbloser Feststoff, Ausbeute 23% d.Th.

$^1$H-NMR ($CF_3CO_2D$): δ = 1,47 und 1,57 (d, J=6Hz, 6H, 2 $CH_3$); 2,1—2,8 (m, 2H, Cyclopenten-H); 3,0—4,2 (m, 6H, 4 Cyclopenten-H und $SCH_2$); 4,8 (m, 1H, CH); 5,1—6,4 (m, 4H, $CH_2Py$ und 2-Lactam-H); 7,41 (s, 1H, Thiazol); 7,5—8,6 ppm (m, 3H, Py)

Beispiel 273

3-[(2,3-Cyclopenteno-1-pyridinium)methyl]-7-[2-syn-propyloxyimino-2-(2-aminothiazol-4-yl)acetamido]-ceph-3-em-4-carboxylat

Farbloser Feststoff, Ausbeute 21% d.Th.

$^1$H-NMR ($CF_3CO_2D$): δ = 1,08 (t, J=6Hz, 3H, $CH_3$); 1,6—2,8 (m, 4H, $CH_2$ und 2 Cyclopenten-H); 3,1—4,2 (m, 6H, 4 Cyclopenten-H und $SCH_2$); 4,53 (t, J=6Hz, $OCH_2$); 5,1—6,3 (m, 4H, $CH_2Py$ und 2 Lactam-H); 7,42 (s, 1H, Thiazol); 7,4—8,5 ppm (m, 3H, Py)

Beispiel 274

3-[(2,3-Cyclopenteno-1-pyridinium)]methyl-7-[2-syn-aethoxyimino-2-(2-amino-5-chlorthiazol-4-yl)acetamido]-ceph-3-em-4-carboxylat

Farbloser Feststoff, Ausbeute 16% d.Th.

$^1$H-NMR: ($CF_3CO_2D$): δ = 1,42 (t, J=7Hz, 3H, $CH_2\underline{CH_3}$); 2,2—2,8 (m, 2 Cyclopenten-H); 3,1—3,85 (m, 6H, 4 Cyclopenten-H und $SCH_2$); 4,50 (q, J=7Hz, 2H, $\underline{CH_2}CH_3$); 5,15—6,25 (m, 4H, $CH_2Py$ und 2 Lactam-H); 7,6—8,7 (m, 3H, Py)

Beispiel 275

3-[(4-Cyclopropyl-1-pyridinium)methyl]-7-[2-(2-amino-5-chlorthiazol-4-yl)-2-syn-methoxyimino-acetamido]-ceph-3-em-4-carboxylat

Farbloser Feststoff, Ausbeute 45% d.Th.

$^1$H-NMR: ($CF_3CO_2D$): δ = 1,03—2,55 (m, 5H, Cyclopropyl); 3,37 und 3,81 (AB, J=18Hz, 2H, $SCH_2$); 4,22 (s, 3H, $OCH_3$); 5,03—6,25 (m, 4H, $CH_2$Py und 2 Lactam-H); 7,56 und 8,64 ppm (AA'BB', J=7Hz, 4H, Py)

Beispiel 276

3-[(2,3-Cyclopenteno-1-pyridinium)methyl]-7-[2-syn-aethoxyimino-2-(2-aminothiazol-4-yl)-acetamido]ceph-3-em-4-carboxylat

Farbloser Feststoff, Ausbeute 57% d.Th.

$^1$H-NMR: ($CF_3CO_2D$): δ = 1,43 (t, J=7Hz, 3H, $CH_2CH_3$); 2,2—2,8 (m, 2 Cyclopenten-H); 3,05—3,95 (m, 6H, 4 Cyclopenten-H und $SCH_2$); 4,51 (q, J=7Hz, 2H, $CH_2$, $CH_3$); 5,05—6,26 (m, 4H, $CH_2$Py und 2 Lactam-H); 7,42 (s, 1H, Thiazol); 7,5—8,75 (m, 3H, Py)

**Patentansprüche**

1. Cephemderivate der allgemeinen Formel I

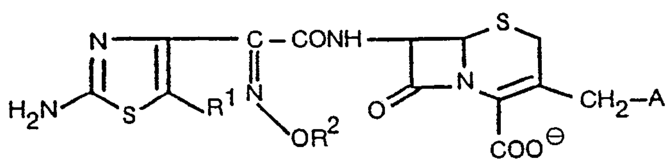

(I)

und deren physiologisch verträgliche Säureadditionssalze, worin bedeuten

$R^1$ Wasserstoff oder Halogen,

$R^2$ Wasserstoff oder $C_1$—$C_6$-Alkyl,

A einen Pyridiniumrest

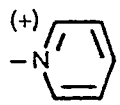

der ein- oder mehrfach, gleich oder verschieden substituiert sein kann

durch $C_1$—$C_6$-Alkyl, das ein- oder mehrfach substituiert sein kann durch Hydroxy, Carboxy, $C_1$—$C_6$-Alkoxycarbonyl, Formyl, $C_1$—$C_6$-Alkylcarbonyl, Carbamoyl, N-Hydroxy-carbamoyl, Sulfo, $C_1$—$C_6$-Alkoxy, Hydroxy-$C_1$—$C_6$-alkoxy, $C_1$—$C_6$-Alkylthio, $C_1$—$C_6$-Alkylsulfinyl, $C_1$—$C_6$-Alkylsulfonyl, $C_2$—$C_6$-Alkenyloxy, $C_2$—$C_6$-Alkenylthio, $C_2$—$C_6$-Alkenylsulfinyl oder $C_2$—$C_6$-Alkenylsulfonyl und wobei 2 Alkylgruppen auch zu einem gegebenenfalls durch $C_1$—$C_6$-Alkyl, $C_1$—$C_4$-Alkoxy, Hydroxymethyl, Halogen, Hydroxy, Oxo, Hydroximino, Exomethylen, Carboxy, $C_1$—$C_6$-Alkoxycarbonyl, Cyano oder Carbamoyl substituierten Di- bis Decamethylen-Ring geschlossen sein können, in dem ein C-Atom durch ein Sauerstoff- oder Schwefelatom ersetzt sein kann und weiterhin auch noch eine oder zwei Doppelbindungen enthalten sein können,

durch Cyano-$C_1$—$C_3$-Alkyl, Epoxy-$C_2$—$C_6$-Alkyl, Trifluormethyl oder Pentafluoräthyl,

durch Hydroxyiminomethyl oder $C_1$—$C_4$-Alkoxyiminomethyl,

durch $C_2$—$C_6$-Alkenyl, das durch Hydroxy substituiert sein kann,

durch $C_2$—$C_6$-Alkinyl,

durch $C_3$—$C_7$-Cycloalkyl oder $C_3$—$C_7$-Cycloalkylmethyl, wobei sich die Kohlenstoffzahl auf den Cycloalkylrest bezieht und wobei in beiden Substituenten der Ring auch substituiert sein kann durch Hydroxy, Halogen, Carboxy, $C_1$—$C_6$-Alkoxycarbonyl oder Cyano,

durch $C_4$—$C_7$-Cycloalkenyl,

durch $C_1$—$C_6$-Alkoxy, das durch Hydroxy, Carboxy oder $C_1$—$C_6$-Alkoxycarbonyl substituiert sein kann,

durch Epoxy-$C_2$—$C_6$-Alkoxy,

durch $C_2$—$C_6$-Alkenyloxy oder $C_2$—$C_6$-Alkinyloxy,

durch Halogen, Cyano, Hydroxy oder Mercapto,

durch $C_1$—$C_6$-Alkylthio, $C_1$—$C_6$-Alkylsulfinyl oder $C_1$—$C_6$-Alkylsulfonyl, die im Alkylteil durch Hydroxy substituiert sein können,

durch Methylthio, Methylsulfinyl oder Methylsulfonyl, die im Methylteil substituiert sind durch Carboxy oder $C_1$—$C_6$-Alkyloxycarbonyl,

durch $C_2$—$C_6$-Alkenylthio, $C_2$—$C_6$-Alkenylsulfinyl oder $C_2$—$C_6$-Alkenylsulfonyl,

durch Phenyl oder Benzyl, die auch durch Halogen substituiert sein können,

durch 2'-Thienyl oder 3'-Thienyl,

durch Formyl,

durch $C_1$—$C_6$-Alkylcarbonyl, das auch durch Hydroxy substituiert sein kann,

durch Benzoyl, durch $C_1$—$C_6$-Alkylcarbonylamino,

durch Carboxy oder $C_1$—$C_6$-Alkoxycarbonyl,

durch Carbamoyl, das am Stickstoff einmal substituiert sein kann durch $C_1$—$C_6$-Alkyl, Hydroxy-$C_1$—$C_6$-alkyl, $C_1$—$C_6$-Alkoxycarbonyl, $C_1$—$C_6$-Alkylcarbonyl, Carboxymethyl, $C_1$—$C_6$-Alkoxycarbonylmethyl, Aminocarbonylmethyl, $C_1$—$C_6$-Alkylaminocarbonyl, Carbamoyl, Hyroxy oder Pyridyl oder das am Stickstoff zweimal substituiert sein kann durch $C_1$—$C_6$-Alkyl,

durch Carbazoyl, das durch $C_1$—$C_4$-Alkyl substituiert sein kann, oder N-Carbamoylcarbazoyl,

durch Sulfamoyl, das am Stickstoff einmal substituiert sein kann durch $C_1$—$C_6$-Alkylaminocarbonyl,

durch Pyridyl oder 4-Pyridon-1-yl,

und in der die $R^2$O-Gruppe in syn-Position steht und wobei für den Fall, daß

A. $R^1$ = Wasserstoff und
$R^2$ = Methyl ist,
A nicht sein kann Pyridinium, Chinolinium oder Pyridinium, das einfach substituiert ist durch 3-Methyl, 4-Methyl, 4-Hydroxymethyl, 4-Carboxymethyl, 4-Trifluormethyl, 3-Chlor, 3-Brom, 3-Jod, 4-Carbamoyl, 4-N-Hydroxymethyl-carbamoyl, 4-methoxycarbonyl-carbamoyl, Hydroxycarbamoyl, N-Hydroxy-N-methyl-carbamoyl, N-Hydroxycarbamoylmethyl

B. $R^1$ = Chlor und
$R^2$ = Methyl oder Äthyl ist
A nicht Pyridinium sein kann.

2. Verfahren zur Herstellung von Cephemverbindungen der allgemeinen Formel I und ihrer physiologisch verträglichen Säureadditionssalze, dadurch gekennzeichnet, daß man eine Verbindung der allgemeinen Formel II

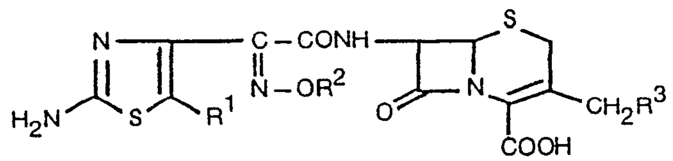

(II)

oder deren Salze, worin $R^1$ und $R^2$ die oben genannte Bedeutung haben und $R^3$ eine durch Pyridin oder substituierte Pyridine, die den Pyridiniumresten A der Formel I entsprechen, austauschbare Gruppe bedeutet, mit Pyridin oder einem solchen Pyridinderivat umsetzt, oder daß man eine 7-Amino-Cephemverbindung der allgemeinen Formel III

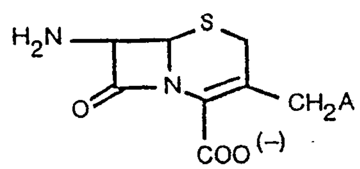

(III)

oder deren Säureadditionssalze, wobei die Aminogruppe auch in Form eines reaktionsfähigen Derivats vorliegen kann, mit einer 2-(2-Aminothiazol-4-yl)-2-syn-oximinoessigsäure der allgemeinen Formel IV

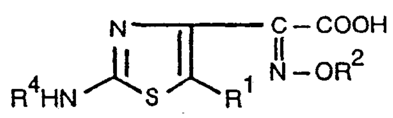

(IV)

worin $R^1$ und $R^2$ die obige Bedeutung besitzen und $R^4$ für Wasserstoff oder eine Aminoschutzgruppe steht, oder mit einem aktivierten Derivat dieser Verbindung umsetzt und α) eine gegebenenfalls vorhandene Schutzgruppe abspaltet und β) falls erforderlich, das erhaltene Produkt in ein physiologisch verträgliches Säureadditionssalz überführt.

3. Verfahren gemäß Anspruch 2, dadurch gekennzeichnet, daß der nucleophile Austausch des Substituenten $R^3$ in Gegenwart von Neutralsalzionen, insbesondere von Jodid oder Thiocyanationen erfolgt.

4. Gegen bakterielle Infektionen wirksame pharmazeutische Präparate, gekennzeichnet durch einen Gehalt an Cephemderivaten der allgemeinen Formel I.

5. Verfahren zur Herstellung von gegen bakterielle Infektionen wirksamen pharmazeutischen Präparaten, dadurch gekennzeichnet, daß ein Cephemderivat der allgemeinen Formel I gegebenenfalls mit pharmazeutisch üblichen Trägerstoffen oder Verdünnungsmitteln in eine pharmazeutisch geeignete Verabreichungsform gebracht wird.

6. 7-Amino-cephemverbindungen der allgemeinen Formel III

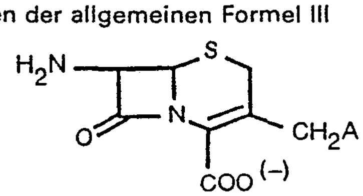

(III)

oder deren Säureadditionssalze, worin

A für einen Pyridiniumrest

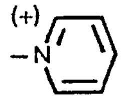

steht, der ein- oder mehrfach, gleich oder verschieden substituiert ist

durch $C_1$—$C_6$-Alkyl, das ein- oder mehrfach substituiert sein kann durch Hydroxy, Carboxy, $C_1$—$C_6$-Alkoxycarbonyl, Formyl, $C_1$—$C_6$-Alkylcarbonyl, Carbamoyl, N-Hydroxy-carbamoyl, Sulfo, $C_1$—$C_6$-Alkoxy, Hydroxy-$C_1$—$C_6$-alkoxy, $C_1$—$C_6$-Alkylthio, $C_1$—$C_6$-Alkylsulfinyl, $C_1$—$C_6$-Alkylsulfonyl, $C_2$—$C_6$-Alkenyloxy, $C_2$—$C_6$-Alkenylthio, $C_2$—$C_6$-Alkenylsulfinyl oder $C_2$—$C_6$-Alkenylsulfonyl und wobei 2 Alkylgruppen auch zu einem gegebenenfalls durch $C_1$—$C_6$-Alkyl, $C_1$—$C_4$-Alkoxy, Hydroxymethyl, Halogen, Hydroxy, Oxo, Hydroximino, Exomethylen, Carboxy, $C_1$—$C_6$-Alkoxycarbonyl, Cyano oder Carbamoyl substituierten Di- bis Decamethylen-Ring geschlossen sein können, in dem ein C-Atom durch ein Sauerstoff- oder Schwefelatom ersetzt sein kann und weiterhin auch noch eine oder zwei Doppelbindungen enthalten sein können,

durch Cyano-$C_1$—$C_3$-Alkyl, Epoxy-$C_2$—$C_6$-Alkyl, Trifluormethyl oder Pentafluoräthyl,

durch Hydroxyiminomethyl oder $C_1$—$C_4$-Alkoxyiminomethyl,

durch $C_2$—$C_6$-Alkenyl, das durch Hydroxy substituiert sein kann,

durch $C_2$—$C_6$-Alkinyl,

durch $C_3$—$C_7$-Cycloalkyl oder $C_3$—$C_7$-Cycloalkylmethyl, wobei sich die Kohlenstoffzahl auf den Cycloalkylrest bezieht und wobei in beiden Substituenten der Ring auch substituiert sein kann durch Hydroxy, Halogen, Carboxy, $C_1$—$C_6$-Alkoxycarbonyl oder Cyano,

durch $C_4$—$C_7$-Cycloalkenyl,

durch $C_1$—$C_6$-Alkoxy, das durch Hydroxy, Carboxy oder $C_1$—$C_6$-Alkoxycarbonyl substituiert sein kann,

durch Epoxy-$C_2$—$C_6$-Alkoxy,

durch $C_2$—$C_6$-Alkenyloxy oder $C_2$—$C_6$-Alkinyloxy,

durch Halogen, Cyano, Hydroxy oder Mercapto,

durch $C_1$—$C_6$-Alkylthio, $C_1$—$C_6$-Alkylsulfinyl oder $C_1$—$C_6$-Alkylsulfonyl, die im Alkylteil durch Hydroxy substituiert sein können,

durch Methylthio, Methylsulfinyl oder Methylsulfonyl, die im Methylteil substituiert sind durch Carboxy oder $C_1$—$C_6$-Alkoxycarbonyl,

durch $C_2$—$C_6$-Alkenylthio, $C_2$—$C_6$-Alkenylsulfinyl oder $C_2$—$C_6$-Alkenylsulfonyl,

durch Phenyl oder Benzyl, die auch durch Halogen substituiert sein können,

durch 2'-Thienyl oder 3'-Thienyl,

durch Formyl

durch $C_1$—$C_6$-Alkylcarbonyl, das auch durch Hydroxy substituiert sein kann,

durch Benzoyl, durch $C_1$—$C_6$-Alkylcarbonylamino,

durch Carboxy oder $C_1$—$C_6$-Alkoxycarbonyl,

durch Carbamoyl, das am Stickstoff einmal substituiert sein kann durch $C_1$—$C_6$-Alkyl, Hydroxy-$C_1$—$C_6$-alkyl, $C_1$—$C_6$-Alkoxycarbonyl, $C_1$—$C_6$-Alkylcarbonyl, Carboxymethyl, $C_1$—$C_6$-Alkoxycarbonylmethyl, Aminocarbonylmethyl, $C_1$—$C_6$-Alkylaminocarbonyl, Carbamoyl, Hyroxy oder Pyridyl oder das am Stickstoff zweimal substituiert sein kann durch $C_1$—$C_6$-Alkyl,

durch Carbazoyl, das durch $C_1$—$C_4$-Alkyl substituiert sein kann, oder N-Carbamoylcarbazoyl,

durch Sulfamoyl, das am Stickstoff einmal substituiert sein kann durch $C_1$—$C_6$-Alkylaminocarbonyl,

durch Pyridyl oder 4-Pyridon-1-yl,

und in der die $R^2$O-Gruppe in syn-Position steht und wobei für den Fall, daß

A. $R^1$ = Wasserstoff und
$R^2$ = Methyl ist,
A nicht sein kann Pyridinium, Chinolinium oder Pyridinium, das einfach substituiert ist durch 3-Methyl, 4-Methyl, 4-Hydroxymethyl, 4-Carboxymethyl, 4-Trifluormethyl, 3-Chlor, 3-Brom, 3-Jod, 4-Carbamoyl, 4-N-Hydroxymethyl-carbamoyl, 4-methoxycarbonyl-carbamoyl, Hydroxycarbamoyl, N-Hydroxy-N-methyl-carbamoyl, N-Hydroxycarbamoylmethyl

B. $R^1$ = Chlor und
$R^2$ = Methyl oder Äthyl ist
A nicht Pyridinium sein kann.

7. Cephemderivate nach Anspruch 1, worin bedeuten

$R_1$ Wasserstoff, $R_2$ $C_1$—$C_4$-Alkyl und

A einen Pyridiniumrest, der substituiert ist durch $C_1$—$C_4$-Alkoxy oder $C_3$—$C_6$-Cycloalkyl oder an den ein Ring der 3 bis 5 Methylengruppen enthält, in dem ein C-Atom auch noch durch Sauerstoff ersetzt sein kann, ankondensiert ist.

8. 7 - [2 - (2 - Aminothiazol - 4 - yl) - 2 - syn-methoxyiminoacetamido] - 3 - [(2,3 - cyclopenteno - 1 - pyridinium)methyl]-ceph - 3 - em - 4 - carboxylat und seine physiologisch verträglichen Salze.

9. 7 - [2 - (2 - Aminothiazol - 4 - yl) - 2 - syn-methoxyiminoacetamido] - 3 - [(3,4 - cyclopenteno - 1 - pyridinium)methyl]-ceph - 3 - em - 4 - carboxylat und seine physiologisch verträglichen Salze.

10. 7 - [2 - (2 - Aminothiazol - 4 - yl) - 2 - syn-methoxyimino acetamido] - 3 - [(5,6,7,8 -

tetrahydro - 1 - chinolinium)methyl]-ceph - 3 - em - 4 - carboxylat und seine physiologisch verträglichen Salze.

11. 7 - [2 - (2 - Aminothiazol - 4 - yl) - 2 - syn-methoxyiminoacetamido] - 3 - [(3,4 - cyclohexeno - 1 - pyridinium)methyl]-ceph - 3 - em - 4 - carboxylat und seine physiologisch verträglichen Salze.

12. 7 - [2 - (2 - Aminothiazol - 4 - yl) - 2 - syn-methoxyiminoacetamido] - 3 - [(3,4 - Dihydro - 2H - pyrano[3,2 - c]pyridinium)methyl]-ceph - 3 - em - 4 - carboxylat und seine physiologisch verträglichen Salze.

13. 7 - [2 - (2 - Aminothiazol - 4 - yl) - 2 - syn-methoxyiminoacetamido] - 3 - [(4 - cyclopropyl - 1 - pyridinium)methyl]-ceph - 3 - em - 4 - carboxylat und seine physiologisch verträglichen Salze.

14. 7 - [2 - (2 - Aminothiazol - 4 - yl) - 2 - syn-methoxyiminoacetamido] - 3 - [(4 - methoxy - 1 - pyridinium)methyl]-ceph - 3 - em - 4 - carboxylat und seine physiologisch verträglichen Salze.

15. 7 - [2 - (2 - Aminothiazol - 4 - yl) - 2 - syn-methoxyiminoacetamido] - 3 - [(3 - methoxy - 1 - pyridinium)methyl]-ceph - 3 - em - 4 - carboxylat und seine physiologisch verträglichen Salze.

16. 7 - [2 - (2 - Aminothiazol - 4 - yl) - 2 - syn-methoxyiminoacetamido] - 3 - [(4 - äthoxy - 1 - pyridinium)methyl]-ceph - 3 - em - 4 - carboxylat und seine physiologisch verträglichen Salze.

17. 7 - [2 - (2 - Aminothiazol - 4 - yl) - 2 - syn-methoxyiminoacetamido] - 3 - [(4 - (1 - hydroxyäthyl) - 1 - pyridinium)methyl]-ceph - 3 - em - 4 - carboxylat und seine physiologisch verträglichen Salze.

18. 7 - [2 - (2 - Aminothiazol - 4 - yl) - 2 - syn-äthoxyimino-acetamido] - 3 - [(4 - cyclopropyl - 1 - pyridinium)methyl]-ceph - 3 - em - 4 - carboxylat und seine physiologisch verträglichen Salze.

**Revendications**

1. Dérivés de céphème répondant à la formule générale I

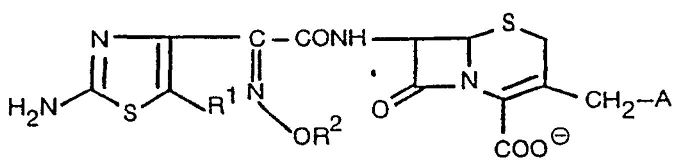

(I)

dans laquelle

$R^1$ représente l'hydrogène ou un halogène,

$R^2$ représente l'hydrogène ou un groupe alcoyle en $C_1$—$C_6$,

A représente un reste pyridinium

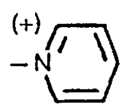

qui peut être substitué une ou plusieurs fois, de manière identique ou différente

par un groupe alcoyle en $C_1$—$C_6$, qui peut être lui-même substitué une ou plusieurs fois par un groupe hydroxy, carboxy, alcoxy($C_1$—$C_6$)-carbonyle, formyle, alcoyl($C_1$—$C_6$)-carbonyle, carbamoyle, N-hydroxy-carbamoyle, sulfo, alcoxy en $C_1$—$C_6$, hydroxy-alcoxy en $C_1$—$C_6$, alcoyl($C_1$—$C_6$)-thio, alcoyl($C_1$—$C_6$)-sulfinyle, alcoyl($C_1$—$C_6$)-sulfonyle, alcényl($C_2$—$C_6$)-oxy, alcényl($C_2$—$C_6$)-thio, alcényl($C_2$—$C_6$)-sulfinyle ou alcényl($C_2$—$C_6$)-sulfonyle, 2 groupes alcoyle pouvant également être fermés en un noyau di- à décaméthylène éventuellement substitué par un groupe alcoyle en $C_1$—$C_6$, alcoxy en $C_1$—$C_4$, hydroxyméthyle, halogéno, hydroxy, oxo, hydroximino, exométhylène, carboxy, alcoxy($C_1$—$C_6$)-carbonyle, cyano ou carbamoyle, dans lequel un atome de carbone peut être remplacé par un atome d'oxygène ou de soufre, et pouvant également contenir encore également une ou deux doubles liaisons;

par un groupe cyano-alcoyle en $C_1$—$C_3$, époxy-alcoyle en $C_2$—$C_6$, trifluorométhyle ou pentafluoroéthyle;

par un groupe hydroxyiminométhyle ou alcoxy($C_1$—$C_4$)iminométhyle;

par un groupe alcényle en $C_2$—$C_6$ qui peut être substitué par un groupe hydroxy;

par un groupe alcynyle en $C_2$—$C_6$;

par un groupe cycloalcoyle en $C_3$—$C_7$ ou cycloalcoyl($C_3$—$C_7$)-méthyle, le nombre d'atomes de carbone se rapportant à la partie cycloalcoyle et dans le cas de ces deux substituants le noyau pouvant également être substitué par un groupe hydroxy, halogéno, carboxy, alcoxy($C_1$—$C_6$)carbonyle ou cyano;

par un groupe cycloalcényle en $C_4$—$C_7$;

par un groupe alcoxy en $C_1$—$C_6$ qui peut être substitué par un groupe hydroxy, carboxy ou alcoxy($C_1$—$C_6$)carbonyle;

par un groupe époxy-alcoxy en $C_2$—$C_6$;

par un groupe alcényl($C_2$—$C_6$)-oxy ou alcynyl($C_2$—$C_6$)-oxy;

par un halogène, un groupe cyano, hydroxy ou mercapto;

par un groupe alcoyl($C_1$—$C_6$)-thio, alcoyl($C_1$—$C_6$)-sulfinyle ou alcoyl($C_1$—$C_6$)-sulfonyle qui peuvent être substitués dans la partie alcoyle par un groupe hydroxy;

par un groupe méthylthio, méthylsulfinyle ou méthylsulfonyle, qui peuvent être substitués dans la partie méthyle par un groupe carboxy ou alcoyl($C_1$—$C_6$)-oxycarbonyle;

par un groupe alcényl($C_2$—$C_6$)-thio, alcényl($C_2$—$C_6$)-sulfinyle ou alcényl($C_2$—$C_6$)-sulfonyle;

par un groupe phényle ou benzyle, qui peuvent également être substitués par un halogène;

par un groupe 2'-thiényle ou 3'-thiényle;

par un groupe formyle;

par un groupe alcoyl($C_1$—$C_6$)carbonyle qui peut également être substitué par un groupe hydroxy;

par un groupe benzoyle, par un groupe alcoyl($C_1$—$C_6$)-carbonylamino;

par un groupe carboxy ou alcoxy($C_1$—$C_6$)-carbonyle;

par un groupe carbamoyle qui peut être substitué une fois sur l'azote par un groupe alcoyle en $C_1$—$C_6$, hydroxy-alcoyle en $C_1$—$C_6$, alcoxy($C_1$—$C_6$)-carbonyle, alcoyl($C_1$—$C_6$)-carbonyle, carboxyméthyle, alcoxy($C_1$—$C_6$)-carbonylméthyle, aminocarbonylméthyle, alcoyl($C_1$—$C_6$)-aminocarbonyle, carbamoyle, hydroxy ou pyridyle, ou qui peut être substitué deux fois sur l'azote par un groupe alcoyle en $C_1$—$C_6$;

par un groupe carbazoyle qui peut être substitué par un groupe alcoyle en $C_1$—$C_4$; ou par un groupe N-carbamoylcarbazoyle;

par un groupe sulfamoyle qui peut être substitué une fois sur l'azote par un groupe alcoyl($C_1$—$C_6$)-aminocarbonyle;

par un groupe pyridyle ou 4-pyridon-1-yle;

et dans laquelle formule le groupe $R^2O$ est en position syn, et dans le cas où:

A) $R^1$ est l'hydrogène et

$R^2$ est un groupe méthyle,

A ne peut pas être un groupe pyridinium, quinoléinium ou pyridinium substitué une fois par un groupe 3-méthyle, 4-méthyle, 4-hydroxyméthyle, 4-carboxyméthyle, 4-trifluorométhyle, 3-chloro, 3-bromo, 3-iodo, 4-carbamoyle, 4-N-hydroxyméthylcarbamoyle, 4-méthoxycarbonyl-carbamoyle, hydroxycarbamoyle, N-hydroxy-N-méthyl-carbamoyle, N-hydroxycarbamoyl-méthyle;

B) $R^1$ est le chlore et

$R^2$ est un groupe méthyle ou éthyle,

A ne peut pas être un groupe pyridinium;

et leurs sels d'addition avec des acides physiologiquement acceptables.

2. Procédé pour la préparation de composés de céphème de formule générale I et de leurs sels d'addition avec des acides physiologiquement acceptables, caractérisé en ce qu'on fait réagir un composé de formule générale II

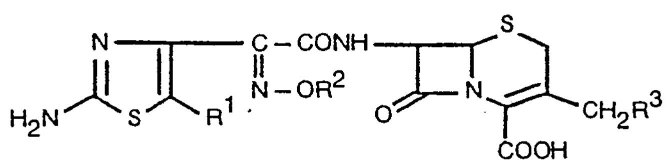

(II)

ou ses sels, où $R^1$ et $R^2$ ont les significations indiquées ci-dessus et $R^3$ représente un groupe échangeable par une pyridine ou une pyridine substituée qui correspondent aux groupes pyridinium A de formule I, avec la pyridine ou un tel dérivé de pyridine, ou en ce qu'on fait réagir un composé de 7-amino-céphème de formule générale III

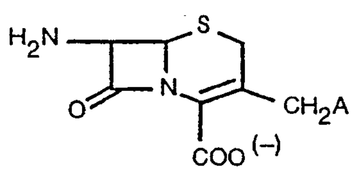

(III)

ou ses sels d'addition avec des acides, où le groupe amino peut également être présent sous la forme d'un dérivé réactif, avec un acide 2-(2-aminothiazol-4-yl)-2-syn-oximino-acétique de formule générale IV

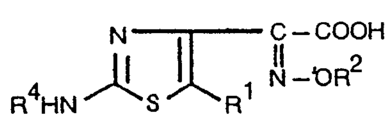

(IV)

où $R^1$ et $R^2$ ont les significations ci-dessus et $R^4$ représente l'hydrogène ou un groupe amino-protecteur, ou avec un dérivé activé de ce composé et α) on élimine un groupe protecteur éventuellement présent et β) si nécessaire, on convertit le produit obtenu en un sel d'addition avec un acide physiologiquement acceptable.

3. Procédé selon la revendication 2, caractérisé en ce que l'échange nucléophile du substituant $R^3$ a lieu en présence d'ions de sels neutres, en particulier d'ions iodure ou thiocyanate.

4. Compositions pharmaceutiques actives contre des infections bactériennes, caractérisées par une teneur en dérivés de céphème de formule générale I.

5. Procédé pour la préparation de compositions pharmaceutiques actives contre des infections bactériennes, caractérisé en ce qu'on met un dérivé de céphème de formule générale I sous une forme d'administration pharmaceutiquement appropriée, éventuellement avec des véhicules ou des diluants pharmaceutiques usuels.

6. Composés de 7-amino-céphème de formule générale III

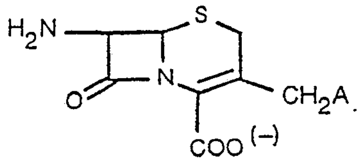

(III)

ou leurs sels d'addition, dans laquelle formule:

A représente un reste pyridinium

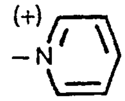

qui est substitué une ou plusieurs fois, de manière identique ou différente

par un groupe alcoyle en $C_1$—$C_6$, qui peut être lui-même substitué par un groupe hydroxy, carboxy, alcoxy($C_1$—$C_6$)-carbonyle, formyle, alcoyl($C_1$—$C_6$)-carbonyle, carbamoyle, N-hydroxy-carbamoyle, sulfo, alcoxy en $C_1$—$C_6$, hydroxy-alcoxy en $C_1$—$C_6$, alcoyl($C_1$—$C_6$)-thio, alcoyl($C_1$—$C_6$)-sulfinyle, alcoyl($C_1$—$C_6$)-sulfonyle, alcényl($C_2$—$C_6$)-oxy, alcényl($C_2$—$C_6$)-thio, alcényl($C_2$—$C_6$)-sulfinyle ou alcényl($C_2$—$C_6$)-sulfonyle, 2 groupes alcoyle pouvant également être fermés en un noyau di- à décaméthylène éventuellement substitué par un groupe alcoyle en $C_1$—$C_6$, alcoxy en $C_1$—$C_6$, hydroxyméthyle, halogéno, hydroxy, oxo, hydroxyimino, exométhylène, carboxy, alcoxy($C_1$—$C_6$)-carbonyle, cyano ou carbamoyle, dans lequel un atome de carbone peut être remplacé par un atome d'oxygène ou de soufre, et pouvant en outre contenir encore également une ou deux doubles liaisons;

par un groupe cyano-alcoyle en $C_1$—$C_3$, époxy-alcoyle en $C_2$—$C_6$, trifluorométhyle ou pentafluoroéthyle;

par un groupe hydroxyiminométhyle ou alcoxy($C_1$—$C_4$)iminométhyle;

par un groupe alcényle en $C_2$—$C_6$ qui peut être substitué par un groupe hydroxy;

par un groupe alcynyle en $C_2$—$C_6$;

par un groupe cycloalcoyle en $C_3$—$C_7$ ou cycloalcoyl($C_3$—$C_7$)-méthyle, le nombre d'atomes de carbone se rapportant au groupe cycloalcoyle et le noyau dans les deux substituants pouvant également être substitué par un groupe hydroxy, halogéno, carboxy, alcoxy($C_1$—$C_6$)-carbonyle ou cyano;

par un groupe cycloalcényle en $C_4$—$C_7$;

par un groupe alcoxy en $C_1$—$C_6$, qui peut être substitué par un groupe hydroxy, carboxy ou alcoxy($C_1$—$C_6$)carbonyle;

par un groupe époxy-alcoxy en $C_2$—$C_6$;

par un groupe alcényl($C_2$—$C_6$)-oxy ou alcynyl($C_2$—$C_6$)-oxy;

par un halogène, un groupe cyano, hydroxy ou mercapto;

par un groupe alcoyl($C_1$—$C_6$)-thio, alcoyl($C_1$—$C_6$)-sulfinyle ou alcoyl($C_1$—$C_6$)-sulfonyle, qui peuvent être substitués dans la partie alcoyle par un groupe hydroxy;

par un groupe méthylthio, méthylsulfinyle ou méthylsulfonyle, qui sont substitués dans la partie méthyle par un groupe carboxy ou alcoyl($C_1$—$C_6$)-carbonyle;

par un groupe alcényl($C_2$—$C_6$)-thio, alcényl($C_2$—$C_6$)-sulfinyle ou alcényl($C_2$—$C_6$)-sulfonyle;

par un groupe phényle ou benzyle, qui peuvent être également substitués par un halogène;

par un groupe 2'-thiényle ou 3'-thiényle;

par un groupe formyle;

par un groupe alcoyl($C_1$—$C_6$)carbonyle qui peut être également substitué par un groupe hydroxy;

par un groupe benzoyle, par un groupe alcoyl($C_1$—$C_6$)-carbonylamino;

par un groupe carboxy ou alcoxy($C_1$—$C_6$)-carbonyle;

par un groupe carbamoyle qui peut être substitué une fois sur l'azote par un groupe alcoyle en $C_1$—$C_6$, hydroxy-alcoyle en $C_1$—$C_6$, alcoxy($C_1$—$C_6$)-carbonyle, alcoyl($C_1$—$C_6$)-carbonyle, carboxyméthyle, alcoxy($C_1$—$C_6$)-carbonylméthyle, aminocarbonylméthyle, alcoyl($C_1$—$C_6$)-aminocarbonyle, carbamoyle, hydroxy ou pyridyle ou qui peut être substitué deux fois sur l'azote par un groupe alcoyle en $C_1$—$C_6$;

par un groupe carbazoyle qui peut être substitué par un groupe alcoyle en $C_1$—$C_4$, ou N-carbamoylcarbazoyle,

par un groupe sulfamoyle qui peut être substitué une fois sur l'azote par un groupe alcoyl($C_1$—$C_6$)-aminocarbonyle;

par un groupe pyridyle ou 4-pyridon-1-yle;

et dans laquelle formule le groupe $R^2O$ est en position syn, et dans le cas où:

A) $R^1$ est l'hydrogène et

$R^2$ est un groupe méthyle,

A ne peut pas être un groupe pyridinium, quinoléinium ou pyridinium substitué une fois par un groupe 3-méthyle, 4-méthyle, 4-hydroxyméthyle, 4-carboxyméthyle, 4-trifluorométhyle, 3-chloro, 3-bromo, 3-iodo, 4-carbamoyle, 4-N-hydroxyméthylcarbamoyle, 4-méthoxycarbonyl-carbamoyle, hydroxycarbamoyle, N-hydroxy-N-méthyl-carbamoyle, N-hydroxycarbamoyl-méthyle,

B) $R^1$ est le chlore et
$R^2$ est un groupe méthyle ou éthyle,
A ne peut pas être un groupe pyridinium.

7. Dérivés de céphème selon la revendication 1, dans lesquels:
$R^1$ est l'hydrogène, $R^2$ est un groupe alcoyle en $C_1$—$C_4$ et
A est un reste pyridinium, substitué par un groupe alcoxy en $C_1$—$C_4$ ou cycloalcoyle en $C_3$—$C_6$ ou sur lequel peut être condensé un noyau qui contient de 3 à 5 groupes méthylène, dans lequel un atome de carbone peut également être encore remplacé par de l'oxygène.

8. 7 - [2 - (2 - aminothiazol - 4 - yl) - 2 - syn-méthoxyiminoacétamido] - 3 - [(2,3 - cyclopenténo - 1 - pyridinium)méthyl]-céph - 3 - ème - 4 - carboxylate et ses sels physiologiquement acceptables.

9. 7 - [2 - (2 - aminothiazol - 4 - yl) - 2 - syn-méthoxyiminoacétamido] - 3 - [(3,4 - cyclopenténo - 1 - pyridinium)méthyl]-céph - 3 - ème - 4 - carboxylate et ses sels physiologiquement acceptables.

10. 7 - [2 - (2 - aminothiazol - 4 - yl) - 2 - syn-méthoxyiminoacétamido] - 3 - [(5,6,7,8 - tétrahydro - 1 - quinoléinium)méthyl]-céph - 3 - ème - 4 - carboxylate et ses sels physiologiquement acceptables.

11. 7 - [2 - (2 - aminothiazol - 4 - yl) - 2 - syn-méthoxyiminoacétamido] - 3 - [(3,4 - cyclohexéno - 1 - pyridinium)méthyl]-céph - 3 - ème - 4 - carboxylate et ses sels physiologiquement acceptables.

12. 7 - [2 - (2 - aminothiazol - 4 - yl) - 2 - syn-méthoxyiminoacétamido] - 3 - [(3,4 - dihydro - 2H - pyrano[3,2-c]pyridinium)méthyl]-céph - 3 - ème - 4 - carboxylate et ses sels physiologiquement acceptables.

13. 7 - [2 - (2 - aminothiazol - 4 - yl) - 2 - syn-méthoxyiminoacétamido] - 3 - [(4 - cyclopropyl - 1 - pyridinium)méthyl]-céph - 3 - ème - 4 - carboxylate et ses sels physiologiquement acceptables.

14. 7 - [2 - (2 - aminothiazol - 4 - yl) - 2 - syn-méthoxyiminoacétamido] - 3 - [(4 - méthoxy - 1 - pyridinium)méthyl]-céph - 3 - ème-carboxylate et ses sels physiologiquement acceptables.

15. 7 - [2 - (2 - aminothiazol - 4 - yl) - 2 - syn-méthoxyiminoacétamido] - 3 - [(3 - méthoxy - 1 - pyridinium)méthyl]-céph - 3 - ème - 4 - carboxylate et ses sels physiologiquement acceptables.

16. 7 - [2 - (2 - aminothiazol - 4 - yl) - 2 - syn-méthoxyiminoacétamido] - 3 - [(4 - éthoxy - 1 - pyridinium)méthyl]-céph - 3 - ème - 4 - carboxylate et ses sels physiologiquement acceptables.

17. 7 - [2 - (2 - aminothiazol - 4 - yl) - 2 - syn-méthoxyiminoacétamido] - 3 - [4 - (1 - hydroxy-éthyl) - 1 - pyridinium)méthyl]-céph - 3 - ème - 4 - carboxylate et ses sels physiologiquement acceptables.

18. 7 - [2 - (2 - aminothiazol - 4 - yl) - 2 - syn-éthoxyiminoacétamido] - 3 - [(4 - cyclopropyl - 1 - pyridinium)méthyl]-céph - 3 - ème - 4 - carboxylate et ses sels physiologiquement acceptables.

**Claims**

1. Cephem derivatives of the general formula I

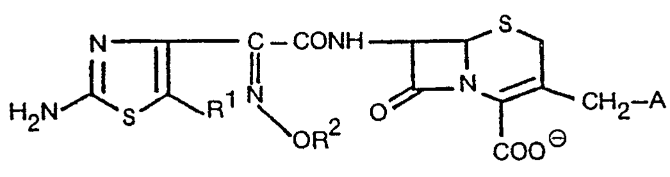

(I)

and their physiologically acceptable acid addition salts, wherein
$R^1$ denotes hydrogen or halogen,
$R^2$ denotes hydrogen or $C_1$—$C_6$-alkyl,
A denotes a pyridinium radical

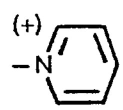

which can be monosubstituted or multisubstituted by identical or different substituents, namely by $C_1$—$C_6$-alkyl which can be monosubstituted or multisubstituted by hydroxyl, carboxyl, $C_1$—$C_6$-alkyloxycarbonyl, formyl, $C_1$—$C_6$-alkylcarbonyl, carbamoyl, N-hydroxycarbamoyl, sulfo, $C_1$—$C_6$-alkoxy, hydroxy-$C_1$—$C_6$-alkyloxy, $C_1$—$C_6$-alkylthio, $C_1$—$C_6$-alkylsulfinyl, $C_1$—$C_6$-alkylsulfonyl, $C_2$—$C_6$-alkenyloxy, $C_2$—$C_6$-alkenylthio, $C_2$—$C_6$-alkenylsulfinyl or $C_2$—$C_6$-alkenylsulfonyl, and of which 2 alkyl groups can also be linked to form a di- to deca-methylene ring which can be substituted by $C_1$—$C_6$-alkyl, $C_1$—$C_4$-alkoxy, hydroxymethyl, halogen, hydroxy, oxo, hydroximino, exomethylene, carboxy, $C_1$—$C_6$-alkoxycarbonyl, cyano or carbamoyl and in which ring one C atom can be replaced an oxygen or sulphur atom and which can additionally contain one or two double bonds, by cyano-$C_1$—$C_3$-alkyl, epoxy-$C_2$—$C_6$-alkyl, trifluoromethyl, or pentafluoroethyl, by hydroyiminomethyl or $C_1$—$C_4$-alkoxyiminomethyl, by $C_2$—$C_6$-alkenyl which can be substituted by hydroxyl,

by $C_2$—$C_6$-alkynyl, by $C_3$—$C_7$-cycloalkyl or $C_3$—$C_7$-cycloalkylmethyl, the number of carbons referring to the cycloalkyl radical and in which two substituents the ring can also be substituted by hydroxyl, halogen, carboxyl, $C_1$—$C_6$-alkyloxycarbonyl or cyano, by $C_4$—$C_7$-cycloalkenyl, by $C_1$—$C_6$-alkoxy, which can be substituted by hydroxyl, carboxyl or $C_1$—$C_6$-alkyloxycarbonyl, by epoxy-$C_2$—$C_6$-alkoxy, by $C_2$—$C_6$-alkenyloxy or $C_2$—$C_6$-alkynyloxy, by halogen, cyano, hydroxyl or mercapto, by $C_1$—$C_6$-alkylthio, $C_1$—$C_6$-alkylsulfinyl or $C_1$—$C_6$-alkylsulfonyl, all of which can be substituted by hydroxyl in the alkyl part, by methylthio, methylsulfinyl or methylsulfonyl, all of which are substituted in the methyl part by carboxyl or $C_1$—$C_6$-alkyloxycarbonyl, by $C_2$—$C_6$-alkenylthio, $C_2$—$C_6$-alkenylsulfinyl or $C_2$—$C_6$-alkenylsulfonyl, by phenyl or benzyl, all of which can also be substituted by halogen, by 2'-thienyl or 3'-thienyl, by formyl, by $C_1$—$C_6$-alkylcarbonyl which can also be substituted by hydroxyl, by benzoyl, by $C_1$—$C_6$-alkylcarbonylamino, by carboxyl or $C_1$—$C_6$-alkoxycarbonyl, by carbamoyl which can be monosubstituted on the nitrogen by $C_1$—$C_6$-alkyl, hydroxy-$C_1$—$C_6$-alkyl, $C_1$—$C_6$-alkyloxycarbonyl, $C_1$—$C_6$-alkylcarbonyl, carboxymethyl, $C_1$—$C_6$-alkyloxycarbonylmethyl, aminocarbonylmethyl, $C_1$—$C_6$-alkylaminocarbonyl, carbamoyl, hydroxyl or pyridyl, or which can be disubstituted on the nitrogen by $C_1$—$C_6$-alkyl, by carbazoyl which can be substituted by $C_1$—$C_4$-alkyl, or N-carbamoylcarbazoyl, by sulfamoyl which can be monosubstituted on the nitrogen by $C_1$—$C_6$-alkylaminocarbonyl, or by pyridyl or 4-pyridon-1-yl, and in which the $R^2O$ group is in the syn position but when

A. $R^1$ is hydrogen and
$R^2$ is methyl, then
A is other than pyridinium, chinolinium or pyridinium monosubstituted by 3-methyl, 4-methyl, 4-hydroxymethyl, 4-carboxymethyl, 4-trifluoromethyl, 3-chloro-, 3-bromo-, 3-iodo, 4-carbamoyl, 4-N-hydroxymethyl-carbamoyl, 4-methoxycarbonyl-carbamoyl, hydroxy-carbamoyl, N-hydroxy-N-methyl-carbamoyl, N-hydroxy-carbamoyl-methyl

B. $R^1$ is chlorine and
$R^2$ is methyl or ethyl,
A is other than pyridinium.

2. A process for preparing cephem compounds of the formula I and their physiologically acceptable acid addition salts, which process comprises reacting a compound of the general formula II

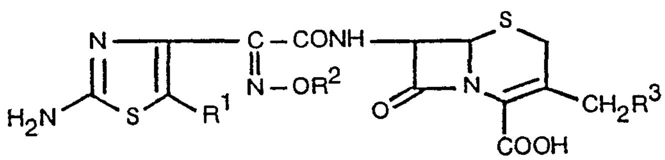

(II)

or its salts, wherein $R^1$ and $R^2$ have the abovementioned meaning and $R^3$ denotes a group which can be replaced by pyridine or substituted pyridines which correspond to the pyridinium radicals A of the formula I, with pyridine or such a pyridine derivative or reacting a 7-aminocephem compound of the general formula III

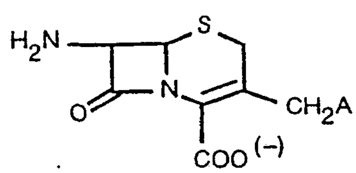

(III)

or its acid addition salts, in which the amino group can also be present in the form of a reactive derivative, with a 2-(2-aminothiazol-4-yl)-2-syn-oximinoacetic acid of the formula IV

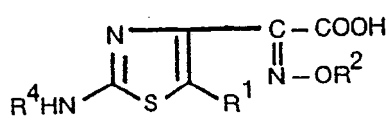

(IV)

wherein $R^1$ and $R^2$ have the above meaning and $R^4$ represents hydrogen or an amino protective group or with an activated derivative of this compound ad α) eliminating a protective group, if present, and β) if necessary, converting the product obtained into a physiologically acceptable acid addition salt.

3. The process as claimed in claim 2, wherein the nucleophilic replacement of the substituent $R^3$ is carried out in the presence of neutral salt ions, in particular of iodide or thiocyanate ions.

4. Pharmaceutical formulations which are active against bacterial infections, containing cephem derivatives of the formula I.

5. A process for preparing pharmaceutical formulations which are active against bacterial infections, which process comprises bringing a cephem derivative of the general formula I, if desired with pharmaceutically customary excipients or diluents, into a pharmaceutically suitable administration form.

6. 7-Aminopcephem compounds of the general formula III

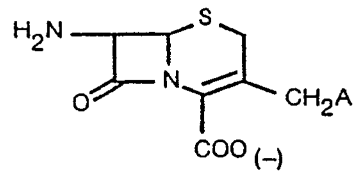

(III)

or their acid addition salts, wherein A represents a pyridinium radical

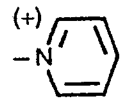

which is monosubstituted or multisubstituted by identical or different substituents, namely by identical or different substituents, namely by $C_1$—$C_6$-alkyl which can be monosubstituted or multisubstituted by hydroxyl, carboxyl, $C_1$—$C_6$-alkyloxycarbonyl, formyl, $C_1$—$C_6$-alkylcarbonyl, carbamoyl, N-hydroxy-carbamoyl, sulfo, $C_1$—$C_6$-alkoxy, hydroxy-$C_1$—$C_6$-alkyloxy, $C_1$—$C_6$-alkylthio, $C_1$—$C_6$-alkylsulfinyl, $C_1$—$C_6$-alkylsulfonyl, $C_2$—$C_6$-alkenyloxy, $C_2$—$C_6$-alkenylthio, $C_2$—$C_6$-alkenylsulfinyl or $C_2$—$C_6$-alkenylsulfonyl, and of which 2 alkyl groups can also be linked to form a di- to deca-methylene ring which can be substituted by $C_1$—$C_6$-alkyl, $C_1$—$C_4$-alkoxy, hydroxymethyl, halogen, hydroxy, oxo, hydroximino, exomethylene, carboxy, $C_1$—$C_6$-alkoxycarbonyl, cyano or carbamoyl and in which ring one C atom can be replaced an oxygen or sulphur atom and which can additionally contain one or two double bonds, by cyano-$C_1$—$C_3$-alkyl, epoxy-$C_2$—$C_6$-alkyl, trifluoromethyl, or pentafluoroethyl, by hydroyiminomethyl or $C_1$—$C_4$-alkoxyiminomethyl, by $C_2$—$C_6$-alkenyl which can be substituted by hydroxyl, by $C_2$—$C_6$-alkynyl, by $C_3$—$C_7$-cycloalkyl or $C_3$—$C_7$-cycloalkylmethyl, the number of carbons referring to the cycloalkyl radical and in which two substituents the ring can also be substituted by hydroxyl, halogen, carboxyl, $C_1$—$C_6$-alkyloxycarbonyl or cyano, by $C_4$—$C_7$-cycloalkenyl, by $C_1$—$C_6$-alkoxy, which can be substituted by hydroxyl, carboxyl or $C_1$—$C_6$-alkyloxy-carbonyl, by epoxy-$C_2$—$C_6$-alkoxy, by $C_2$—$C_6$-alkenyloxy or $C_2$—$C_6$-alkynyloxy, by halogen, cyano, hydroxyl or mercapto, by $C_1$—$C_6$-alkylthio, $C_1$—$C_6$-alkylsulfinyl or $C_1$—$C_6$-alkylsulfonyl, all of which can be substituted by hydroxyl in the alkyl part, by methylthio, methylsulfinyl or methylsulfonyl, all of which are substituted in the methyl part by carboxyl or $C_1$—$C_6$-alkyloxycarbonyl, by $C_2$—$C_6$-alkenylthio, $C_2$—$C_6$-alkenylsulfinyl or $C_2$—$C_6$-alkenylsulfonyl, by phenyl or benzyl, all of which can also be substituted by halogen, by 2'-thienyl or 3'-thienyl, by formyl, by $C_1$—$C_6$-alkylcarbonyl which can also be substituted by hydroxyl, by benzoyl, by $C_1$—$C_6$-alkylcarbonylamino, by carboxyl or $C_1$—$C_6$-alkoxycarbonyl, by carbamoyl which can be monosubstituted on the nitrogen by $C_1$—$C_6$-alkyl, hydroxy-$C_1$—$C_6$-alkyl, $C_1$—$C_6$-alkyloxy-carbonyl, $C_1$—$C_6$-alkylcarbonyl, carboxymethyl, $C_1$—$C_6$-alkyloxycarbonylmethyl, aminocarbonylmethyl, $C_1$—$C_6$-alkylaminocarbonyl, carbamoyl, hydroxyl or pyridyl, or which can be disubstituted on the nitrogen by $C_1$—$C_6$-alkyl, by carbazoyl which can be substituted by $C_1$—$C_4$-alkyl, or N-carbamoylcarbazoyl, by sulfamoyl which can be monosubstituted on the nitrogen by $C_1$—$C_6$-alkylaminocarbonyl, or by pyridyl or 4-pyridon-1-yl, and in which the $R^2$O group is in the syn position but when

A. $R^1$ is hydrogen and
$R^2$ is methyl, then
A is other than pyridinium, chinolinium or pyridinium monosubstituted by 3-methyl, 4-methyl, 4-hydroxymethyl, 4-carboxymethyl, 4-trifluoromethyl, 3-chloro-, 3-bromo-, 3-iodo, 4-carbamoyl, 4-N-hydroxymethyl-carbamoyl, 4-methoxycarbonyl-carbamoyl, hydroxy-carbamoyl, N-hydroxy-N-methyl-carbamoyl, N-hydroxy-carbamoyl-methyl

B. $R^1$ is chlorine and
$R^2$ is methyl or ethyl,
A is other than pyridinium.

7. Cephem derivatives claimed in claim 1, wherein $R_1$ denotes hydrogen, $R_2$ denotes $C_1$—$C_4$-alkyl and A denotes a pyridinium radical which is substituted by $C_1$—$C_4$-alkoxy or $C_3$—$C_6$-cycloalkyl or onto which a ring which contains 3 to 5 methylene groups in which ring one C atom can also be replaced by an oxygen, is fused.

8. 7 - [2 - (2 - Aminothiazol - 4 - yl) - 2-syn-methoxyiminoacetamido] - 3 - [(2,3 - cyclopenteno - 1 - pyridinium)-methyl]-ceph - 3 - em - 4 - carboxylate and its physiologically acceptable salts.

9. 7 - [2 - (2 - Aminothiazol - 4 - yl) - 2-syn-methoxyiminoacetamido] - 3 - [(3,4 - cyclopenteno - 1 - pyridinium)-methyl]-ceph - 3 - em - 4 - carboxylate and its physiologically acceptable salts.

10. 7 - [2 - (2 - Aminothiazol - 4 - yl) - 2-syn-methoxyiminoacetamido] - 3 - [(5,6,7,8 - tetrahydro - 1 - quinolinium)-methyl]-ceph - 3 - em - 4 - carboxylate and its physiologically acceptable salts.

11. 7 - [2 - (2 - Aminothiazol - 4 - yl) - 2-syn-methoxyiminoacetamido] - 3 - [(3,4 - cyclohexeno - 1 - pyridinium)-methyl]-ceph - 3 - em - 4 - carboxylate and its physiologically acceptable salts.

12. 7 - [2 - (2 - Aminothiazol - 4 - yl) - 2-syn-methoxyiminoacetamido] - 3 - [(3,4 - dihydro - 2H - pyrano[3,2 - c]pyridinium)-methyl]-ceph - 3 - em - 4 - carboxylate and its physiologically acceptable salts.

13. 7 - [2 - (2 - Aminothiazol - 4 - yl) - 2-syn-methoxyiminoacetamido] - 3 - [(4 - cyclopropyl-pyridinium)-methyl]-ceph - 3 - em - 4 - carboxylate and its physiologically acceptable salts.

14. 7 - [2 - (2 - Aminothiazol - 4 - yl) - 2-syn-methoxyiminoacetamido] - 3 - [(4 - methoxy - 1 - pyridinium)-methyl]-ceph - 3 - em - 4 - carboxylate and its physiologically acceptable salts.

15. 7 - [2 - (2 - Aminothiazol - 4 - yl) - 2-syn-methoxyiminoacetamido] - 3 - [(3 - methoxy - 1 - pyridinium)-methyl]-ceph - 3 - em - 4 - carboxylate and its physiologically acceptable salts.

16. 7 - [2 - (2 - Aminothiazol - 4 - yl) - 2-syn-methoxyiminoacetamido] - 3 - [(4 - ethoxy - 1 - pyridinium)-methyl]-ceph - 3 - em - 4 - carboxylate and its physiologically acceptable salts.

17. 7 - [2 - (2 - Aminothiazol - 4 - yl) - 2-syn-methoxyiminoacetamido] - 3 - [(4 - (1 - hydroxyethyl) - 1 - pyridinium)-methyl]-ceph - 3 - em - 4 - carboxylate and its physiologically acceptable salts.

18. 7 - [2 - (2 - Aminothiazol - 4 - yl) - 2-syn-ethoxyiminoacetamido] - 3 - [(4 - cyclopropyl - 1 - pyridinium)-methyl]-ceph - 3 - em - 4 - carboxylate and its physiologically acceptable salts.